# EUROPEAN PATENT SPECIFICATION

(11) **EP 4 558 509 B1**
(45) Date of publication and mention of the grant of the patent: **26.08.2026**
(21) Application number: 23748960.4
(22) Date of filing: 20.07.2023
(51) Int. Cl.: C07F 9/655, A61P 35/00

(54) **CATIONIC CHROMENONES AND THEIR USE IN ONCOLOGY**
KATIONISCHE CHROMENONE UND IHRE VERWENDUNG IN DER ONKOLOGIE
CHROMÉNONES CATIONIQUES ET LEUR UTILISATION EN ONCOLOGIE

(30) Priority: 21.07.2022 GB 202210689
(43) Date of publication of application: 28.05.2025
(73) Proprietor: Floratek Pharma SA, 1170 Aubonne (CH)
(72) Inventor: STOICESCU, Dan Florin, 1170 Aubonne (CH)
(74) Representative: Russell, Tim
(86) International application number: PCT/EP2023/070187
(87) International publication number: WO 2024/018017

(56) References cited:
- WO-A1-2010/052734
- WO-A1-2021/053205

## Description

### FIELD OF THE INVENTION

The present disclosure relates to phosphonium salt compounds, and to associated salts, multi-salts, solvates, and pharmaceutical compositions. The present disclosure also relates to the use of such compounds and compositions in the treatment and prevention of medical disorders and diseases, most especially in the prevention or treatment of cancer.

### BACKGROUND

There is a need to provide compounds with improved pharmacological and/or physiological and/or physiochemical properties and/or those that provide a useful alternative to known compounds. WO 2010/052734 A1 discloses chromene derivatives used for the treatment of cancer.

### SUMMARY OF THE INVENTION

The present disclosure addresses the above need(s).

The present disclosure is defined by the claims.

A first aspect of the disclosure provides a compound of formula (1):

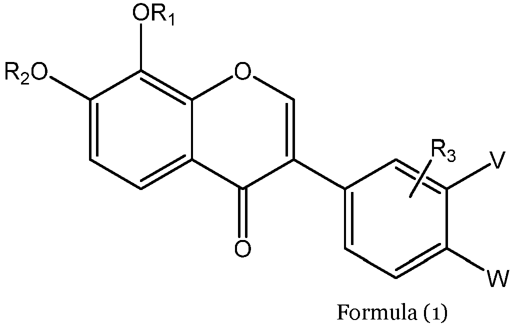

For example, the compound may be a compound of formula (2):

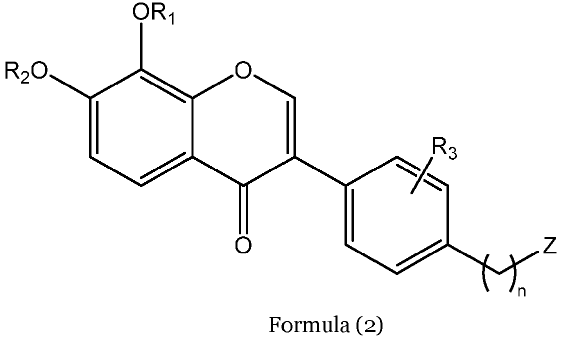

For example, the compound may be a compound of formula (2A):

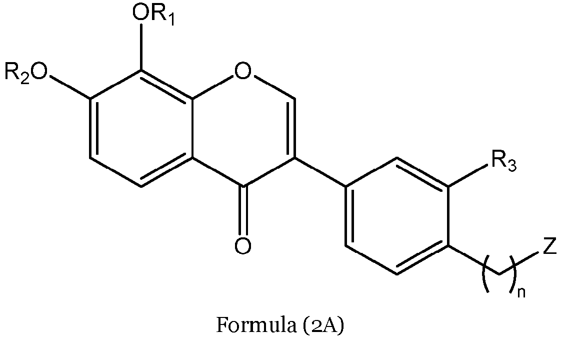

For example, the compound may be a compound of formula (2B):

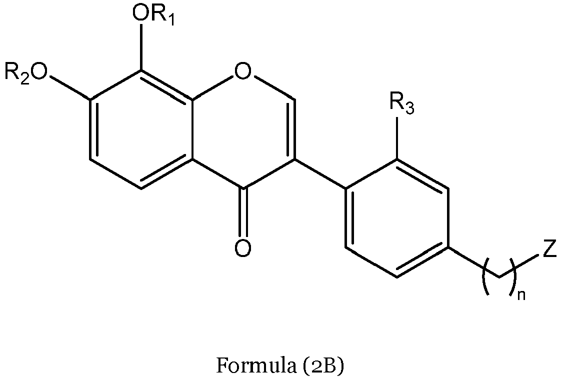

For example, the compound may be a compound of formula (2C):

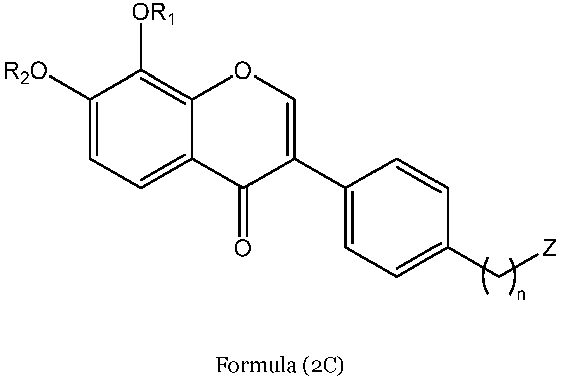

For example, the compound may be a compound of formula (3):

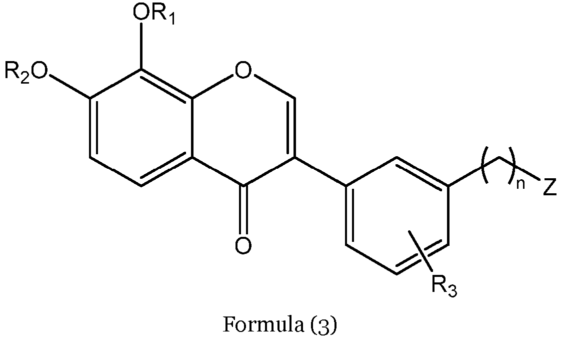

For example, the compound may be a compound of formula (3A):

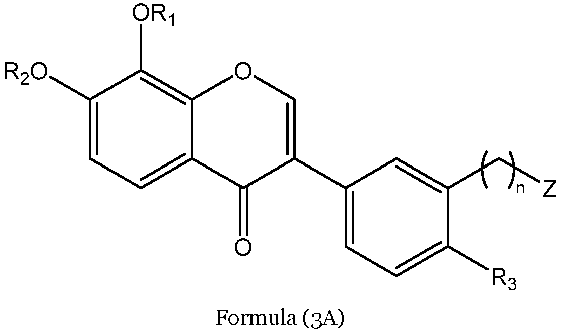

For example, the compound may be a compound of formula (3B):

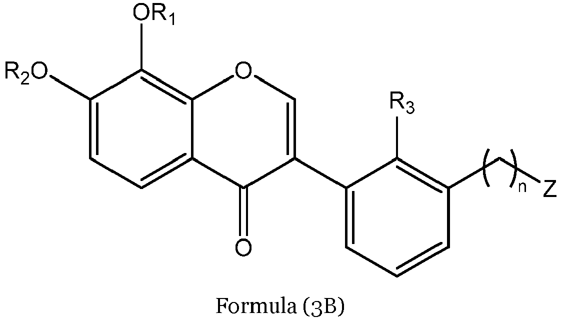

For example, the compound may be a compound of formula (3C):

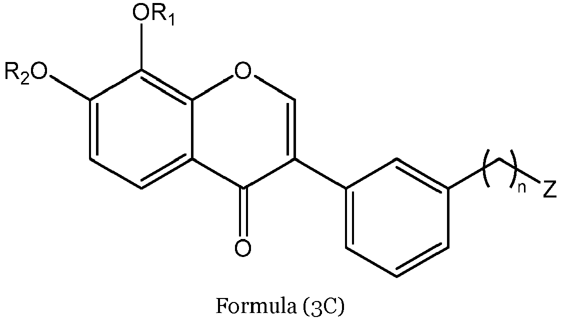

A second aspect provides a pharmaceutically acceptable salt, multi-salt, or solvate of the compound of the first aspect.

A third aspect provides a pharmaceutical composition comprising a compound of the first aspect of the disclosure, or a pharmaceutically acceptable salt, multi-salt, or solvate of the second aspect, and a pharmaceutically acceptable excipient.

A fourth aspect provides a compound of the first aspect, or a pharmaceutically acceptable salt, multi-salt, or solvate of the second aspect, or a pharmaceutical composition of the third aspect, for use in medicine, and/or for use in the treatment or prevention of a disease, disorder or condition. In one embodiment, the disease, disorder or condition is cancer.

A fifth aspect provides the use of a compound of the first aspect, a pharmaceutically effective salt, multi-salt, or solvate of the second aspect, or a pharmaceutical composition according to the third aspect, in the manufacture of a medicament for the treatment or prevention of a disease, disorder or condition. Typically the treatment or prevention comprises the administration of the compound, salt, multi-salt, or solvate, or pharmaceutical composition to a subject. In one embodiment, the disease, disorder or condition is cancer.

A sixth aspect of the invention provides a compound for use in a method of treatment or prevention of a disease, disorder or condition, the method comprising the step of administering an effective amount of a compound of the first aspect, or a pharmaceutically acceptable salt, multi-salt, or solvate of the second aspect, or a pharmaceutical composition of the third aspect, to thereby treat or prevent the disease, disorder or condition. Typically the administration is to a subject in need thereof. In one embodiment, the disease, disorder or condition is cancer.

A seventh aspect of the invention provides a compound for use in a method of treatment or prevention of a disease, disorder or condition, the method comprising the step of administering an effective amount of a compound according to formula (1) as defined herein, or a pharmaceutically acceptable salt, multi-salt, or solvate of the second aspect, or a pharmaceutical composition of the third aspect, to thereby treat or prevent the disease, disorder or condition. Typically the administration is to a subject in need thereof. In one embodiment, the disease, disorder or condition is cancer.

### Definitions

In the context of the present specification, a "hydrocarbyl" substituent group or a hydrocarbyl moiety in a substituent group only includes carbon and hydrogen atoms but, unless stated otherwise, does not include any heteroatoms, such as N, O or S, in its carbon skeleton. A hydrocarbyl group/moiety may be saturated or unsaturated (including aromatic), and may be straight-chained or branched, or be or include cyclic groups wherein, unless stated otherwise, the cyclic group does not include any heteroatoms, such as N, O or S, in its carbon skeleton. Examples of hydrocarbyl groups include alkyl, alkenyl, alkynyl, cycloalkyl, cycloalkenyl and aryl groups/moieties and combinations of all of these groups/moieties. Typically a hydrocarbyl group is a C₁-C₁₂ hydrocarbyl group. More typically a hydrocarbyl group is a C₁-C₁₀ hydrocarbyl group. A "hydrocarbylene" group is similarly defined as a divalent hydrocarbyl group.

An "alkyl" substituent group or an alkyl moiety in a substituent group may be linear or branched. Examples of alkyl groups/moieties include methyl, ethyl, n-propyl, i-propyl, n-butyl, i-butyl, t-butyl and n-pentyl groups/moieties. Unless stated otherwise, the term "alkyl" does not include "cycloalkyl". Typically an alkyl group is a C₁-C₁₂ alkyl group. More typically an alkyl group is a C₁-C₆ alkyl group. An "alkylene" group is similarly defined as a divalent alkyl group.

An "alkenyl" substituent group or an alkenyl moiety in a substituent group refers to an unsaturated alkyl group or moiety having one or more carbon-carbon double bonds. Examples of alkenyl groups/moieties include ethenyl, propenyl, 1-butenyl, 2-butenyl, 1-pentenyl, 1-hexenyl, 1,3-butadienyl, 1,3-pentadienyl, 1,4-pentadienyl and 1,4-hexadienyl groups/moieties. Unless stated otherwise, the term "alkenyl" does not include "cycloalkenyl". Typically an alkenyl group is a C₂-C₁₂ alkenyl group. More typically an alkenyl group is a C₂-C₆ alkenyl group. An "alkenylene" group is similarly defined as a divalent alkenyl group.

An "alkynyl" substituent group or an alkynyl moiety in a substituent group refers to an unsaturated alkyl group or moiety having one or more carbon-carbon triple bonds. Examples of alkynyl groups/moieties include ethynyl, propargyl, but-1-ynyl and but-2-ynyl. Typically an alkynyl group is a C₂-C₁₂ alkynyl group. More typically an alkynyl group is a C₂-C₆ alkynyl group. An "alkynylene" group is similarly defined as a divalent alkynyl group.

A "haloalkyl" substituent group or haloalkyl group in a substituent group refers to an alkyl, alkenyl, or alkynyl substituent group or moiety including one or more carbon atoms and one or more halo atoms, e.g. Cl, Br, I, or F. Each halo atom replaces a hydrogen of the alkyl, alkenyl, or alkynyl substituent group or moiety. Examples include -CH₂F -CHF₂, -CHI₂, -CHBr₂,-CHCl₂,-CF₃, -CH₂CF₃ and CF₂CH₃.

An "alkoxy" substituent group or alkoxy group in a substituent group refers to an alkyl, alkenyl, or alkynyl substituent group or moiety including one or more carbon atoms and one or more oxygen atoms. Each oxygen atom replaces a carbon atom (for example the terminal or bonding carbon) of the alkyl, alkenyl, or alkynyl substituent group or moiety. Examples include -OCH₃, -OCH₂CH₃, -OCH₂CH₂CH₃, and -OCH(CH₃)(CH₃).

An "alkylthio" substituent group or alkylthio group in a substituent group refers to an alkyl, alkenyl, or alkynyl substituent group or moiety including one or more carbon atoms and one or more sulphur atoms. Each sulphur atom replaces a carbon atom (for example the terminal or bonding carbon) of the alkyl, alkenyl, or alkynyl substituent group or moiety. Examples include -SCH₃, -SCH₂CH₃, -SCH₂CH₂CH₃, and - SCH(CH₃)(CH₃).

An "alkylsulfinyl" substituent group or alkylsulfinyl group in a substituent group refers to an alkyl, alkenyl, or alkynyl substituent group or moiety including one or more carbon atoms and one or more sulfinyl groups (-S(=O)-). Each sulfinyl group replaces a carbon atom (for example the terminal or bonding carbon) of the alkyl, alkenyl, or alkynyl substituent group or moiety. Examples include - S(=O)CH₃, - S(=O)CH₂CH₃, - S(=O)CH₂CH₂CH₃, and - S(=O)CH(CH₃)(CH₃).

An "alkylsulfonyl" substituent group or alkylsulfonyl group in a substituent group refers to an alkyl, alkenyl, or alkynyl substituent group or moiety including one or more carbon atoms and one or more sulfonyl groups (-SO₂-). Each sulfonyl group replaces a carbon atom (for example the terminal or bonding carbon) of the alkyl, alkenyl, or alkynyl substituent group or moiety. Examples include - SO₂(CH₃), - SO₂(CH₂CH₃), - SO₂(CH₂CH₂CH₃), and - SO₂(CH(CH₃)(CH₃)).

An "arylsulfonyl" substituent group or arylsulfonyl group in a substituent group refers to an aryl substituent group or moiety including one or more carbon atoms and one or more sulfonyl groups (-SO₂-). Each sulfonyl group replaces a carbon atom (for example the terminal or bonding carbon) of the alkyl, alkenyl, or alkynyl substituent group or moiety. Examples include - SO₂(CH₃), - SO₂(CH₂CH₃), - SO₂(CH₂CH₂CH₃), and - SO₂(CH(CH₃)(CH₃)).

A "cyclic" substituent group or a cyclic moiety in a substituent group refers to any hydrocarbyl ring, wherein the hydrocarbyl ring may be saturated or unsaturated and may include one or more heteroatoms, e.g. N, O or S, in its carbon skeleton. Examples of cyclic groups include aliphatic cyclic, cycloalkyl, cycloalkenyl, heterocyclic, aryl and heteroaryl groups as discussed below. A cyclic group may be monocyclic, bicyclic (e.g. bridged, fused or spiro), or polycyclic. Typically, a cyclic group is a 3- to 12-membered cyclic group, which means it contains from 3 to 12 ring atoms. More typically, a cyclic group is a 3- to 7-membered monocyclic group, which means it contains from 3 to 7 ring atoms.

A "heterocyclic" substituent group or a heterocyclic moiety in a substituent group refers to a cyclic group or moiety including one or more carbon atoms and one or more heteroatoms, e.g. N, O or S, in the ring structure. Examples of heterocyclic groups include heteroaryl groups as discussed below and non-aromatic heterocyclic groups such as azetidinyl, azetinyl, tetrahydrofuranyl, pyrrolidinyl, tetrahydrothiophenyl, tetrahydropyranyl, piperidinyl, piperazinyl, morpholinyl and thiomorpholinyl groups.

An "aliphatic cyclic" substituent group or aliphatic cyclic moiety in a substituent group refers to a hydrocarbyl cyclic group or moiety that is not aromatic. The aliphatic cyclic group may be saturated or unsaturated and may include one or more heteroatoms, e.g. N, O or S, in its carbon skeleton. Examples include cyclopropyl, cyclohexyl and morpholinyl. Unless stated otherwise, an aliphatic cyclic substituent group or moiety may include monocyclic, bicyclic or polycyclic hydrocarbyl rings.

A "cycloalkyl" substituent group or a cycloalkyl moiety in a substituent group refers to a saturated hydrocarbyl ring containing, for example, from 3 to 7 carbon atoms, examples of which include cyclopropyl, cyclobutyl, cyclopentyl and cyclohexyl. Unless stated otherwise, a cycloalkyl substituent group or moiety may include monocyclic, bicyclic or polycyclic hydrocarbyl rings.

A "cycloalkenyl" substituent group or a cycloalkenyl moiety in a substituent group refers to a non-aromatic unsaturated hydrocarbyl ring having one or more carbon-carbon double bonds and containing, for example, from 3 to 7 carbon atoms, examples of which include cyclopent-1-en-1-yl, cyclohex-1-en-1-yl and cyclohex-1,3-dien-1-yl. Unless stated otherwise, a cycloalkenyl substituent group or moiety may include monocyclic, bicyclic or polycyclic hydrocarbyl rings.

An "aryl" substituent group or an aryl moiety in a substituent group refers to an aromatic hydrocarbyl ring. The term "aryl" includes monocyclic aromatic hydrocarbons and polycyclic fused ring aromatic hydrocarbons wherein all of the fused ring systems (excluding any ring systems which are part of or formed by optional substituents) are aromatic. Examples of aryl groups/moieties include phenyl, naphthyl, anthracenyl and phenanthrenyl. Unless stated otherwise, the term "aryl" does not include "heteroaryl".

A "heteroaryl" substituent group or a heteroaryl moiety in a substituent group refers to an aromatic heterocyclic group or moiety. The term "heteroaryl" includes monocyclic aromatic heterocycles and polycyclic fused ring aromatic heterocycles wherein all of the fused ring systems (excluding any ring systems which are part of or formed by optional substituents) are aromatic. Examples of heteroaryl groups/moieties include the following: 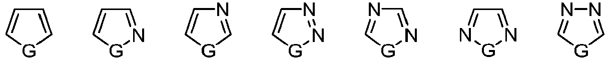 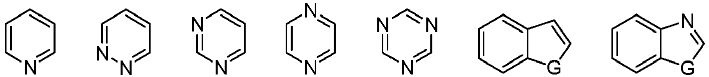 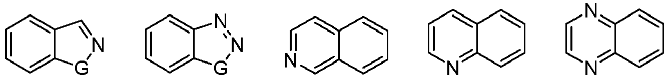 wherein G = O, S or NH.

For the purposes of the present specification, where a combination of moieties is referred to as one group, for example, arylalkyl, arylalkenyl, arylalkynyl, alkylaryl, alkenylaryl or alkynylaryl, the last mentioned moiety contains the atom by which the group is attached to the rest of the molecule. An example of an arylalkyl group is benzyl.

Typically a substituted group comprises 1, 2, 3 or 4 substituents, more typically 1, 2 or 3 substituents, more typically 1 or 2 substituents, and even more typically 1 substituent.

Unless stated otherwise, any divalent bridging substituent (e.g. -O-, -S-, -NH-, -N(R^{β})-or -R^{α}-) of an optionally substituted group or moiety must only be attached to the specified group or moiety and may not be attached to a second group or moiety, even if the second group or moiety can itself be optionally substituted.

The term "halo" includes fluoro, chloro, bromo and iodo.

Where reference is made to a carbon atom of a group being replaced by an N, O or S atom, what is intended is that: 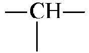 is replaced by 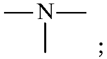
-CH₂- is replaced by -NH-, -O- or -S-;
-CH₃ is replaced by -NH₂, -OH, or -SH;
-CH= is replaced by -N=;
CH₂= is replaced by NH=, O= or S=; or
CH≡ is replaced by N≡.

In the context of the present specification, unless otherwise stated, a Cₓ-C_{y} group is defined as a group containing from x to y carbon atoms. For example, a C₁-C₄ alkyl group is defined as an alkyl group containing from 1 to 4 carbon atoms. Optional substituents and moieties are not taken into account when calculating the total number of carbon atoms in the parent group substituted with the optional substituents and/or containing the optional moieties. For the avoidance of doubt, replacement heteroatoms, e.g. N, O or S, are counted as carbon atoms when calculating the number of carbon atoms in a Cₓ-C_{y} group. For example, a morpholinyl group is to be considered a C₆ heterocyclic group, not a C₄ heterocyclic group.

A "protecting group" refers to a grouping of atoms that when attached to a reactive functional group (e.g. OH) in a compound masks, reduces or prevents reactivity of the functional group.

In the context of the present specification, = is a double bond; ≡ is a triple bond.

The protection and deprotection of functional groups is described in 'Protective Groups in Organic Synthesis', 2nd edition, T.W. Greene and P.G.M Wuts, Wiley-Interscience.

### DETAILED DESCRIPTION OF THE INVENTION

A first aspect of the invention provides a compound of formula (1): 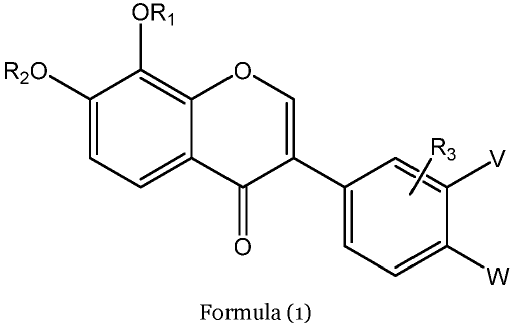 wherein:
either V is -R₃, and W is: 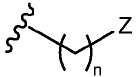
or W is -R₃, and V is: 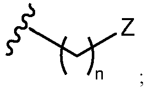
   Z is -[P(R⁵)₃]X;
   X is a counter anion;
   R¹and R², independently, are selected from -H, -C₁₋₄ alkyl, -C(O)R⁴, -C(O)NHR⁴, and -C(O)N(R⁴)₂; or R¹ and R² together form a C₁₋₄ alkylene group;
   R³ is selected from H; halo; -CN; -NO₂; -R^{β}; -OH; -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; -COOR^{β}; and -OCOR^{β};
   each -R^{β} is independently selected from a C₁-C₆ alkyl, C₂-C₆ alkenyl, C₂-C₆ alkynyl or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C≡CH, -CHO, - CON(CH₃)₂ or oxo (=O) groups;
   each -R⁴ is independently selected from C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl;
   R⁵ is independently selected from H, C₁-C₆ alkyl, C₂-C₆ alkenyl, C₃-C₁₄ aryl group, or C₃-C₁₄ aliphatic cyclic group; and wherein any -R⁵ may optionally be substituted with one or more C₁-C₄ alkyl, halo, -CF₃, -OH, -NH₂, -CN, -C≡CH or oxo (=O) groups; and
   n is an integer from 1 to 10.

In one embodiment, is provided a compound of formula (1) wherein:
V is -R₃, and W is: 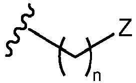
or W is -R₃, and V is: 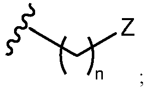
Z is -[P(R⁵)₃]X;
X is a counter anion;
R¹and R², independently, are selected from H and -C₁₋₄ alkyl;
R³ is selected from -H, -Cl, -F, -CH₃, -OCH₃;
R⁵ is phenyl, each R⁵ being optionally substituted with -CF₃; and
n is an integer from 1 to 10.

In one embodiment of formula (1),
V is -H, and W is: 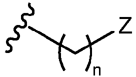
or W is -H, and V is:

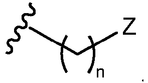

In one embodiment of formula (1), R³ is -H.

In one embodiment, the invention provides a compound of formula (2): 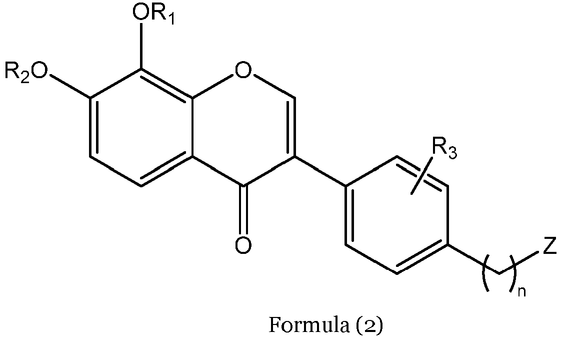 wherein:
Z is -[P(R⁵)₃]X;
X is a counter anion;
R¹and R², independently, are selected from -H, -C₁₋₄ alkyl, -C(O)R⁴, -C(O)NHR⁴, and -C(O)N(R⁴)₂; or R¹and R² together form a C₁₋₄ alkylene group;
R³ is selected from H; halo; -CN; -NO₂; -R^{β}; -OH; -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; -COOR^{β}; and -OCOR^{β};
each -R^{β} is independently selected from a C₁-C₆ alkyl, C₂-C₆ alkenyl, C₂-C₆ alkynyl or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C≡CH, -CHO, - CON(CH₃)₂ or oxo (=O) groups;
each -R⁴ is independently selected from C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl;
R⁵ is independently selected from H, C₁-C₆ alkyl, C₂-C₆ alkenyl, C₃-C₁₄ aryl group, or C₃-C₁₄ aliphatic cyclic group; and wherein any -R⁵ may optionally be substituted with one or more C₁-C₄ alkyl, halo, -CF₃, -OH, -NH₂, -CN, -C≡CH or oxo (=O) groups; and
n is an integer from 1 to 10.

In one embodiment, is provided a compound of formula (2) wherein:
Z is -[P(R⁵)₃]X;
X is a counter anion;
R¹and R², independently, are selected from H and -C₁₋₄ alkyl;
R³ is selected from -H, -Cl, -F, -CH₃, and -OCH₃;
R⁵ is phenyl, each R⁵ being optionally substituted with -CF₃; and
n is an integer from 1 to 10.

In one embodiment of formula (2), n is an integer from 1 to 5. In one embodiment of formula (2), n is an integer from 6 to 10.

In one embodiment of formula (2), R³ is -H.

In one embodiment, the compound may be a compound of formula (2A): 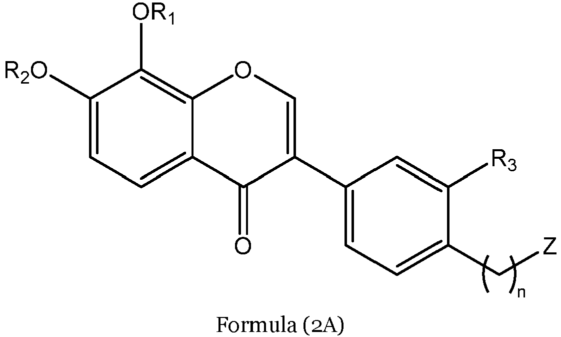 wherein R¹, R², R³, Z and n are as defined herein.

In one embodiment, is provided a compound of formula (2A) wherein:
Z is -[P(R⁵)₃]X;
X is a counter anion;
R¹and R², independently, are selected from H and -C₁₋₄ alkyl;
R³ is selected from -H, -Cl, -F, -CH₃, and -OCH₃;
R⁵ is phenyl, each R⁵ being optionally substituted with -CF₃; and
n is an integer from 1 to 10.

In one embodiment of formula (2A), n is an integer from 1 to 5. In one embodiment of formula (2A), n is an integer from 6 to 10.

In one embodiment of formula (2A), R³ is -H.

In one embodiment, the compound may be a compound of formula (2B): 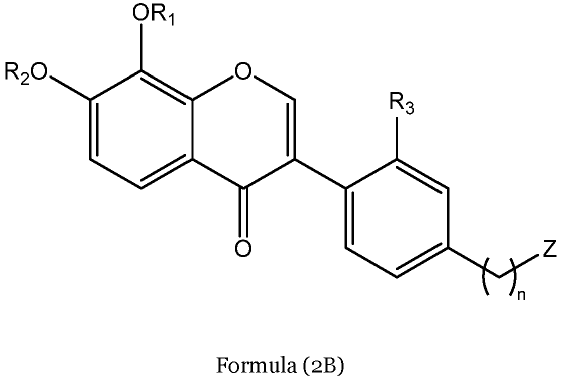 wherein R¹, R², R³, Z and n are as defined herein.

In one embodiment, is provided a compound of formula (2B) wherein:
Z is -[P(R⁵)₃]X;
X is a counter anion;
R¹and R², independently, are selected from H and -C₁₋₄ alkyl;
R³ is selected from -H, -Cl, -F, -CH₃, and -OCH₃;
R⁵ is phenyl, each R⁵ being optionally substituted with -CF₃; and
n is an integer from 1 to 10.

In one embodiment of formula (2B), n is an integer from 1 to 5. In one embodiment of formula (2B), n is an integer from 6 to 10.

In one embodiment of formula (2B), R³ is -H.

In one embodiment, the compound may be a compound of formula (2C): 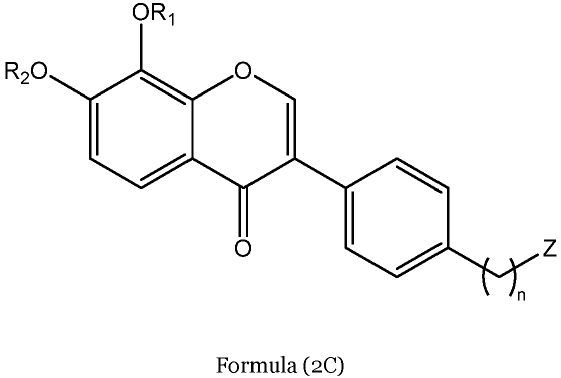 wherein R¹, R², Z and n are as defined herein.

In one embodiment, is provided a compound of formula (2C) wherein:
Z is -[P(R⁵)₃]X;
X is a counter anion;
R¹and R², independently, are selected from H and -C₁₋₄ alkyl;
R⁵ is phenyl, each R⁵ being optionally substituted with -CF₃; and
n is an integer from 1 to 10.

In one embodiment of formula (2C), n is an integer from 1 to 5. In one embodiment of formula (2C), n is an integer from 6 to 10.

In one embodiment, the compound may be a compound of formula (3): 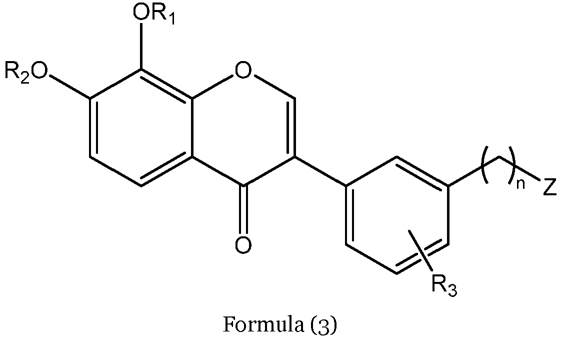 wherein:
Z is -[P(R⁵)₃]X;
X is a counter anion;
R¹and R², independently, are selected from -H, -C₁₋₄ alkyl, -C(O)R⁴, -C(O)NHR⁴, and -C(O)N(R⁴)₂; or R¹and R² together form a C₁₋₄ alkylene group;
R³ is selected from H; halo; -CN; -NO₂; -R^{β}; -OH; -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; -COOR^{β}; and -OCOR^{β};
each -R^{β} is independently selected from a C₁-C₆ alkyl, C₂-C₆ alkenyl, C₂-C₆ alkynyl or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C≡CH, -CHO, - CON(CH₃)₂ or oxo (=O) groups;
each -R⁴ is independently selected from C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl;
R⁵ is independently selected from H, C₁-C₆ alkyl, C₂-C₆ alkenyl, C₃-C₁₄ aryl group, or C₃-C₁₄ aliphatic cyclic group; and wherein any -R⁵ may optionally be substituted with one or more C₁-C₄ alkyl, halo, -CF₃, -OH, -NH₂, -CN, -C≡CH or oxo (=O) groups; and
n is an integer from 1 to 10.

In one embodiment, is provided a compound of formula (3) wherein:
Z is -[P(R⁵)₃]X;
X is a counter anion;
R¹and R², independently, are selected from H and -C₁₋₄ alkyl;
R³ is selected from -H, -Cl, -F, -CH₃, and -OCH₃;
R⁵ is phenyl, each R⁵ being optionally substituted with -CF₃; and
n is an integer from 1 to 10.

In one embodiment of formula (3), n is an integer from 1 to 5. In one embodiment of formula (3), n is an integer from 6 to 10.

In one embodiment of formula (3), R³ is -H.

In one embodiment, the compound may be a compound of formula (3A): 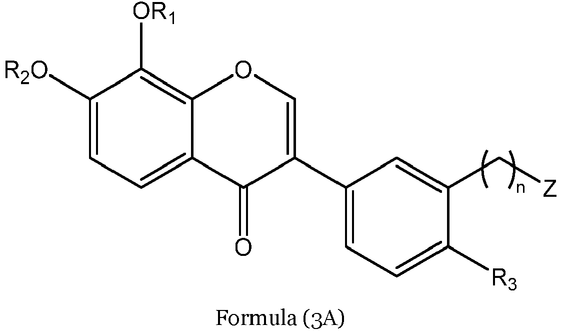 wherein R¹, R², R³, Z and n are as defined herein.

In one embodiment, is provided a compound of formula (3A) wherein:
Z is -[P(R⁵)₃]X;
X is a counter anion;
R¹and R², independently, are selected from H and -C₁₋₄ alkyl;
R³ is selected from -H, -Cl, -F, -CH₃, and -OCH₃;
R⁵ is phenyl, each R⁵ being optionally substituted with -CF₃; and
n is an integer from 1 to 10.

In one embodiment of formula (3A), n is an integer from 1 to 5. In one embodiment of formula (3A), n is an integer from 6 to 10.

In one embodiment of formula (3A), R³ is -H.

In one embodiment, the compound may be a compound of formula (3B): 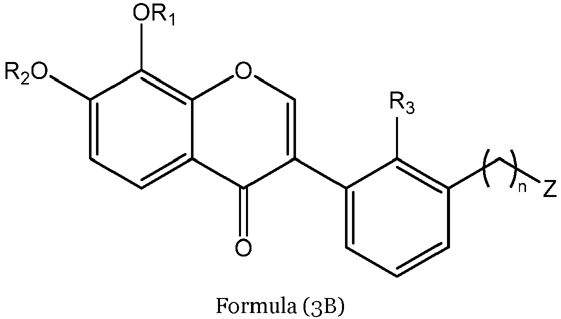 wherein R¹, R², R³, Z and n are as defined herein.

In one embodiment, is provided a compound of formula (3B) wherein:
Z is -[P(R⁵)₃]X;
X is a counter anion;
R¹and R², independently, are selected from H and -C₁₋₄ alkyl;
R³ is selected from -H, -Cl, -F, -CH₃, -OCH₃;
R⁵ is phenyl, each R⁵ being optionally substituted with -CF₃; and
n is an integer from 1 to 10.

In one embodiment of formula (3B), n is an integer from 1 to 5. In one embodiment of formula (3B), n is an integer from 6 to 10.

In one embodiment of formula (3B), R³ is -H.

In one embodiment, the compound may be a compound of formula (3C): 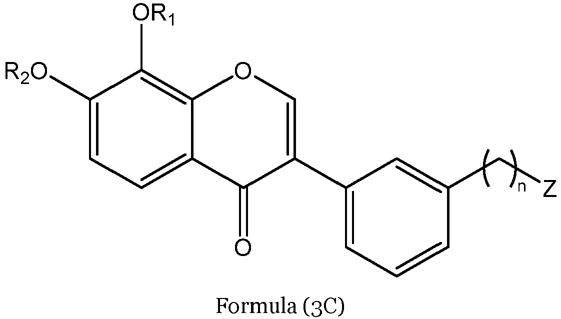 wherein R¹, R², Z and n are as defined herein.

In one embodiment, is provided a compound of formula (3C) wherein:
Z is -[P(R⁵)₃]X;
X is a counter anion;
R¹and R², independently, are selected from H and -C₁₋₄ alkyl;
R⁵ is phenyl, each R⁵ being optionally substituted with -CF₃; and
n is an integer from 1 to 10.

In one embodiment of formula (3C), n is an integer from 1 to 5. In one embodiment of formula (3C), n is an integer from 6 to 10.

In the context of formulae (1), (2), (2A), (2B), (2C), (3), (3A), (3B) and (3C) described herein, the following embodiments are provided by the present disclosure.

In one embodiment, R¹and R², independently, are selected from H and -C₁₋₄ alkyl. For example, R¹and R², independently, may be selected from H, -CH₃, and -CH₂CH₃. For example, R¹and R², independently, may be selected from H and -CH₃.

In one embodiment, R¹and R² are both -H.

In one embodiment, R¹and R² are both -CH₃.

In one embodiment, R¹ is -H, and R² is -CH₃.

In one embodiment, R¹ is -CH₃, and R² is -H.

In one embodiment, R¹and R² together form a C₁₋₄ alkylene group.

In one embodiment, R³ is selected from H; halo; -CN; -NO₂; -R^{β}; -OH; -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; -COOR^{β}; and -OCOR^{β}. In one embodiment, R³ is selected from H; halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; and -COOR^{β}. In one embodiment, R³ is selected from H; halo; -CN; -NO₂; -R^{β}; -OH; -OR^{β}; -NH₂; -NHR^{β}; -N(R^{β})₂; -CHO; -COR^{β}; -COOH; -COOR^{β}; and -OCOR^{β}. In one embodiment, R³ is selected from H; halo; -CN; -NO₂; -R^{β}; -NH₂; -NHR^{β}; -N(R^{β})₂; -CHO; -COR^{β}; -COOH; and -COOR^{β}. In one embodiment, R³ is selected from H; halo; -CN; -NO₂; -R^{β}; -OH; -OR^{β}; -NH₂; -NHR^{β}; and -N(R^{β})₂. In one embodiment, R³ is selected from H; halo; -CN; -NO₂; -R^{β}; -NH₂; -NHR^{β}; and -N(R^{β})₂. In one embodiment, R³ is selected from H; halo; -CN; -NO₂; -R^{β}; -OH; -OR^{β}; and -NH₂. In one embodiment, R³ is selected from H; halo; -CN; -NO₂; -R^{β}; and -NH₂. In one embodiment, R³ is selected from H; -Cl, -F, - CH₃ and -OCH₃. In one embodiment, R³ is selected from H; halo; -CN; -NO₂; and -NH₂. In one embodiment, R³ is H.

In one embodiment, each -R^{β} is independently selected from a C₁-C₆ alkyl, C₂-C₆ alkenyl, C₂-C₆ alkynyl or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C≡CH, - CHO, -CON(CH₃)₂ or oxo (=O) groups.

In one embodiment, each -R^{β} is independently selected from a C₁-C₆ alkyl, C₂-C₆ alkenyl, C₂-C₆ alkynyl or C₃-C₁₄ cyclic group, and wherein any -R^{β} may optionally be substituted with one or more halo, -OH, -NH₂, -CN, -NO₂, -C≡CH, -CHO, -CON(CH₃)₂ or oxo (=O) groups.

In one embodiment, each -R^{β} is independently selected from a C₁-C₆ alkyl, C₂-C₆ alkenyl, C₂-C₆ alkynyl or C₃-C₁₄ cyclic group.

In one embodiment, each -R^{β} is independently selected from -CF₃ and -CHF₂.

In one embodiment, each -R^{β} is independently selected from a methyl, ethyl, n-propyl, i-propyl, n-butyl, i-butyl, t-butyl, n-pentyl, ethenyl, propenyl, 1-butenyl, 2-butenyl, 1-pentenyl, 1-hexenyl, 1,3-butadienyl, 1,3-pentadienyl, 1,4-pentadienyl, 1,4-hexadienyl, ethynyl, propargyl, but-1-ynyl or but-2-ynyl group.

In one embodiment, each -R^{β} is independently selected from a methyl, ethyl, n-propyl, i-propyl, n-butyl, i-butyl, t-butyl, or n-pentyl group.

In one embodiment, X is selected from but not limited to halides (for example fluoride, chloride, bromide or iodide) or other inorganic anions (for example nitrate, perchlorate, sulfate, bisulfate, or phosphate) or organic anions (for example propianoate, butyrate, glycolate, lactate, mandelate, citrate, acetate, benzoate, salicylate, succinate, malate, tartrate, fumarate, maleate, hydroxymaleate, galactarate, gluconate, pantothenate, pamoate, methanesulfonate, trifluoromethanesulfonare, ethanesulfonare, 2-hydroxyethanesulfonate, benzenesulfonate, toluene-p-sulfonate, naphthalene-2-sulfonate, camphorsulfonate, ornithinate, glutamate or aspartate).

In one embodiment, X may be a fluoride, chloride, bromide or iodide. In one embodiment, X is bromide or chloride. In one embodiment, X is bromide. In one embodiment, X is chloride.

In one embodiment, each -R⁵ is independently selected from H, or C₁-C₆ alkyl, or C₃-C₁₄ aryl group; wherein any - R⁵ may optionally be substituted with one or more C₁-C₄ alkyl, halo, -OH, -NH₂, -CN, -C≡CH or oxo (=O) groups. Alternatively, any - R⁵ may optionally be substituted with one or more -CF₃.

In one embodiment, each - R⁵ is independently a C₃-C₁₄ aryl group; wherein any - R⁵ may optionally be substituted with one or more C₁-C₄ alkyl, halo, -OH, -NH₂, -CN, -C≡CH or oxo (=O) groups. Alternatively, each R⁵ may be substituted with one or more -CF₃.

In one embodiment, two of the R⁵ groups are the same. In one embodiment, each R⁵ group is the same.

In one embodiment, each - R⁵ is a phenyl group; each phenyl group may optionally be substituted with one or more C₁-C₄ alkyl, halo, -OH, -NH₂, -CN, -C≡CH or oxo (=O) groups.

In one embodiment, each R⁵ is a phenyl group.

In one embodiment, each R⁵ is a phenyl group optionally substituted with -CF₃. For example, each R⁵ may be a phenyl group substituted with -CF₃. For example, each R⁵ may be a phenyl group substituted with -CF₃ at the para-position.

In one embodiment, Z is -[P(Ph)_{3]}X, wherein X is a counter anion. For example, X may be bromide or chloride, or X may be bromide. In one embodiment, X is chloride.

In one embodiment, Z is -[P(p-CF₃-Ph)₃]X, wherein X is a counter anion. For example, X may be bromide or chloride, or X may be bromide. In one embodiment, X is chloride.

In one embodiment, each -R⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl.

In one embodiment, each - R⁴ is independently selected from C₁₋₄ alkyl.

In one embodiment, each - R⁴ is independently selected from H, methyl, ethyl, n-propyl, i-propyl, n-butyl, i-butyl, t-butyl, n-pentyl, ethenyl, propenyl, 1-butenyl, 2-butenyl, 1-pentenyl, 1-hexenyl, 1,3-butadienyl, 1,3-pentadienyl, 1,4-pentadienyl, 1,4-hexadienyl, ethynyl, propargyl, but-1-ynyl or but-2-ynyl group.

In one embodiment, each - R⁴ is independently selected from H, methyl, ethyl, n-propyl, i-propyl, n-butyl, i-butyl, t-butyl, or n-pentyl group.

In one embodiment, each - R⁴ is independently selected from H, methyl, ethyl, propyl, and butyl.

In one embodiment, n is an integer from 1 to 10, for example from 2 to 10. In one embodiment, n is 1, 2, 3, 4, 5, 6, 7, 8, 9 or 10. In one embodiment, n is an integer from 1 to 6 or an integer from 2 to 6. In one embodiment, n is an integer from 2 to 5. In one embodiment, n is 3, 4 or 5.

In one embodiment, n is 1. In one embodiment, n is 2. In one embodiment, n is 3. In *5* one embodiment, n is 4. In one embodiment, n is 6. In one embodiment, n is 8. In one embodiment, n is 10.

In one embodiment, the compound of formula (1) is selected from:

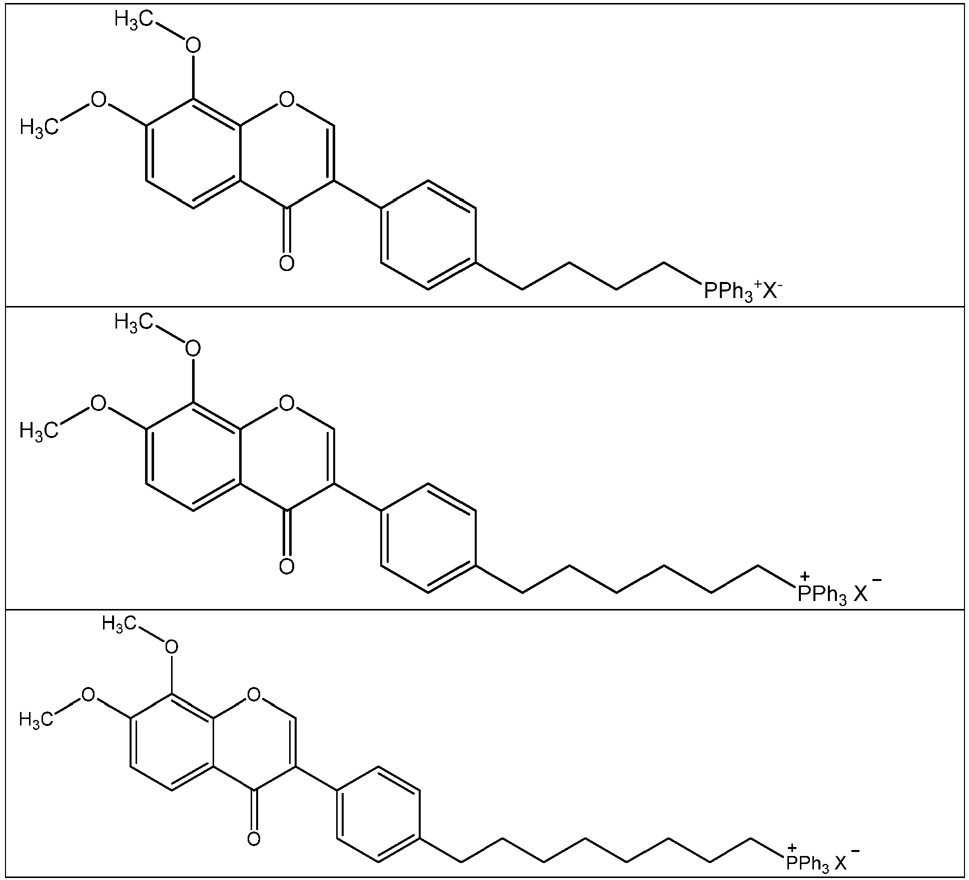

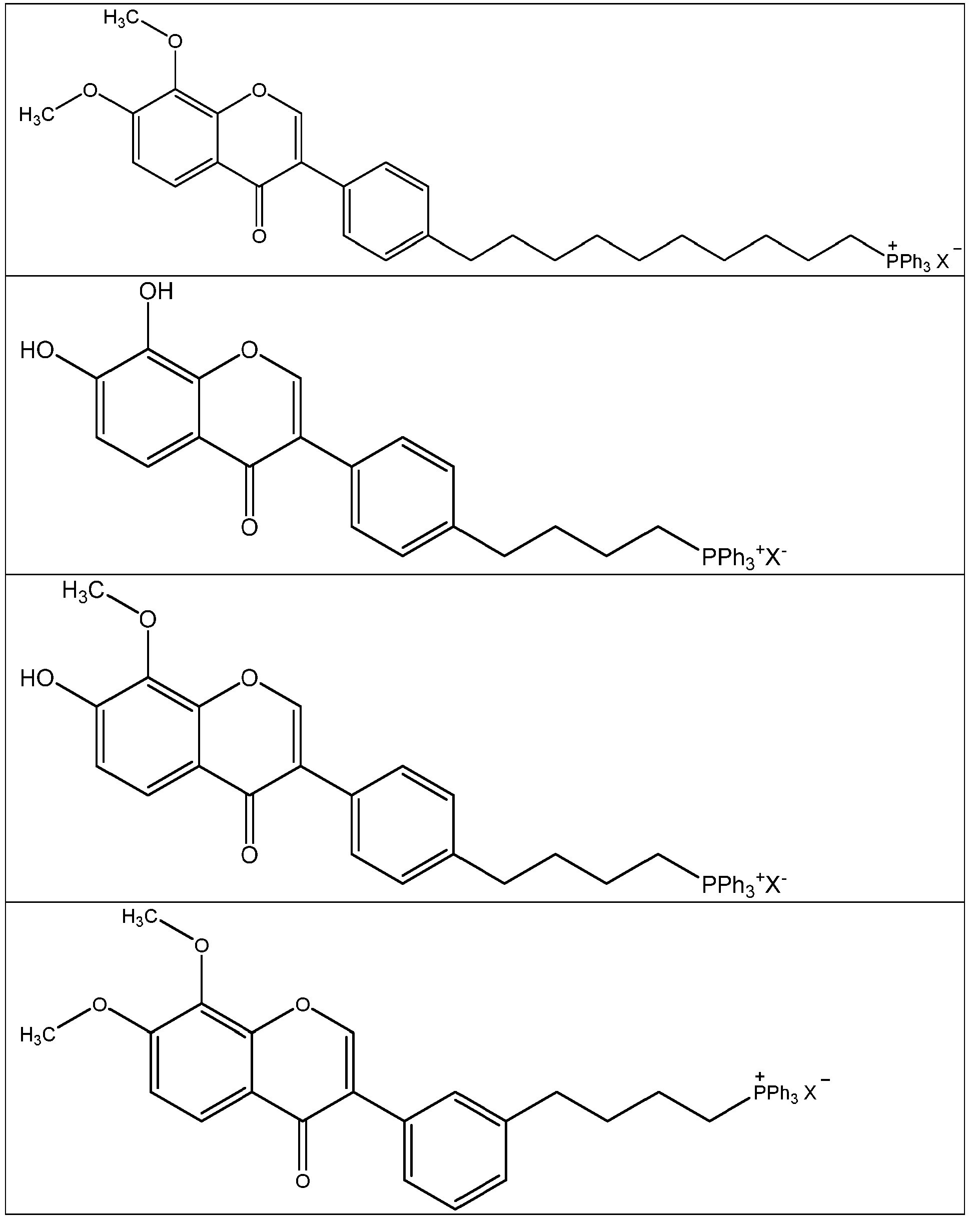

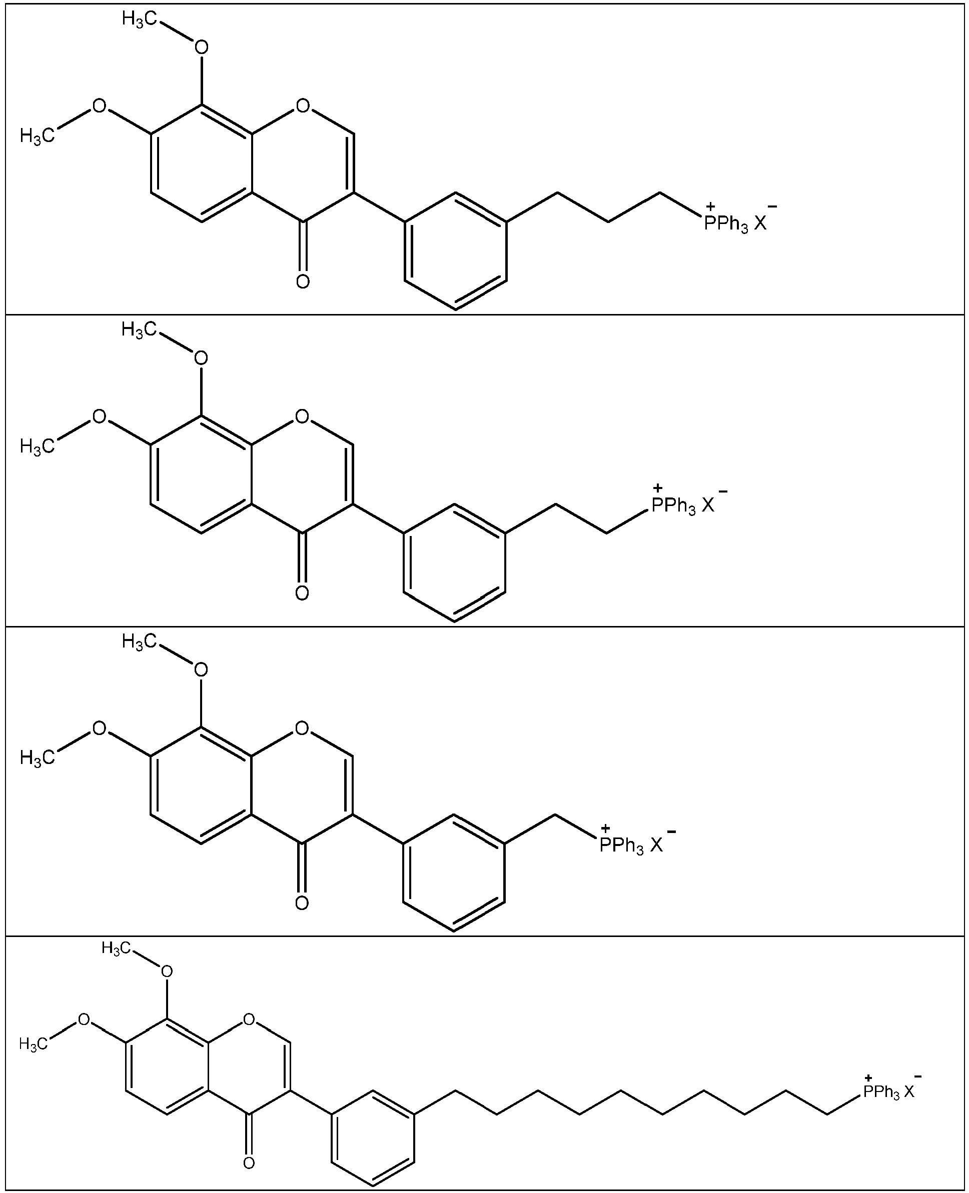

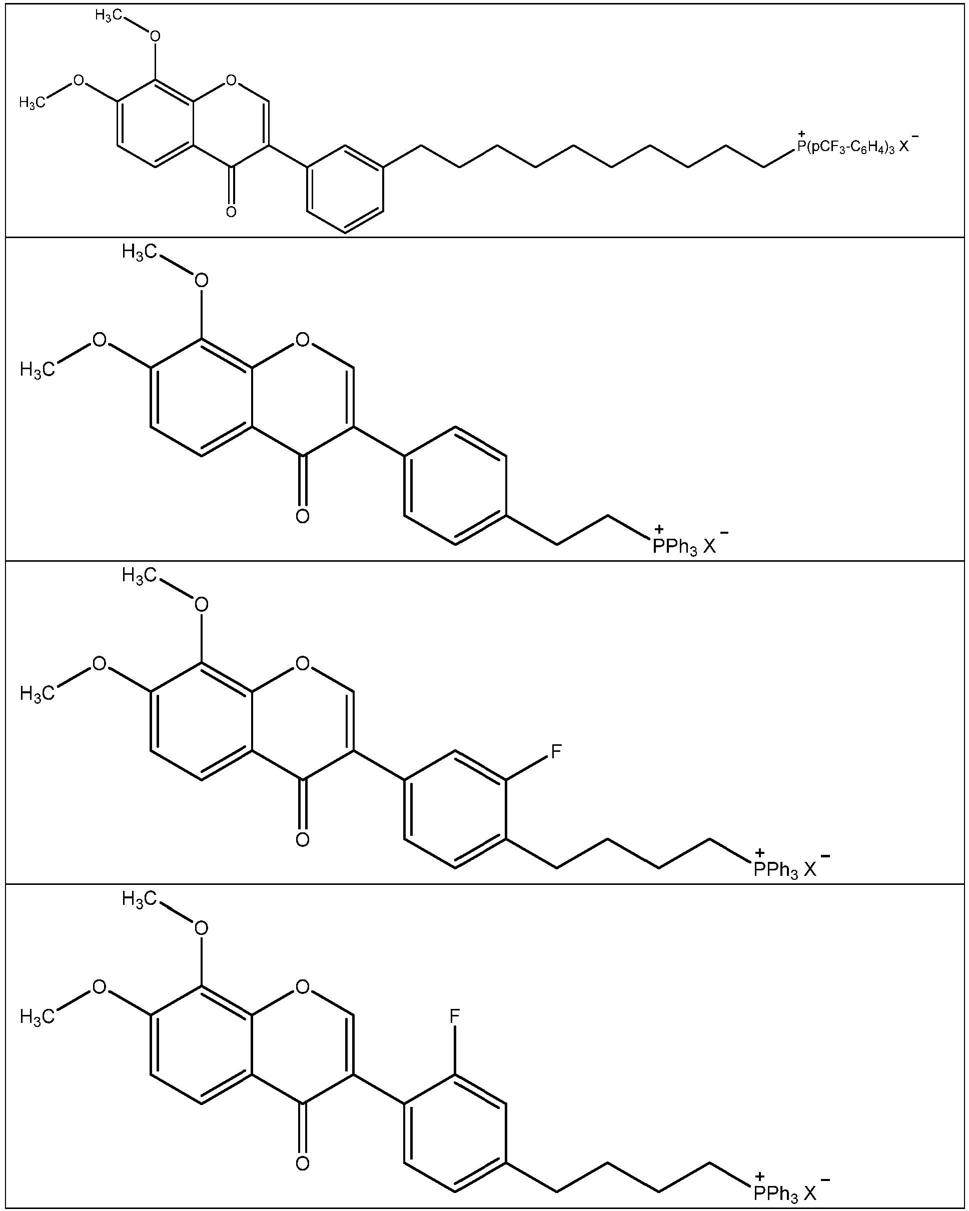

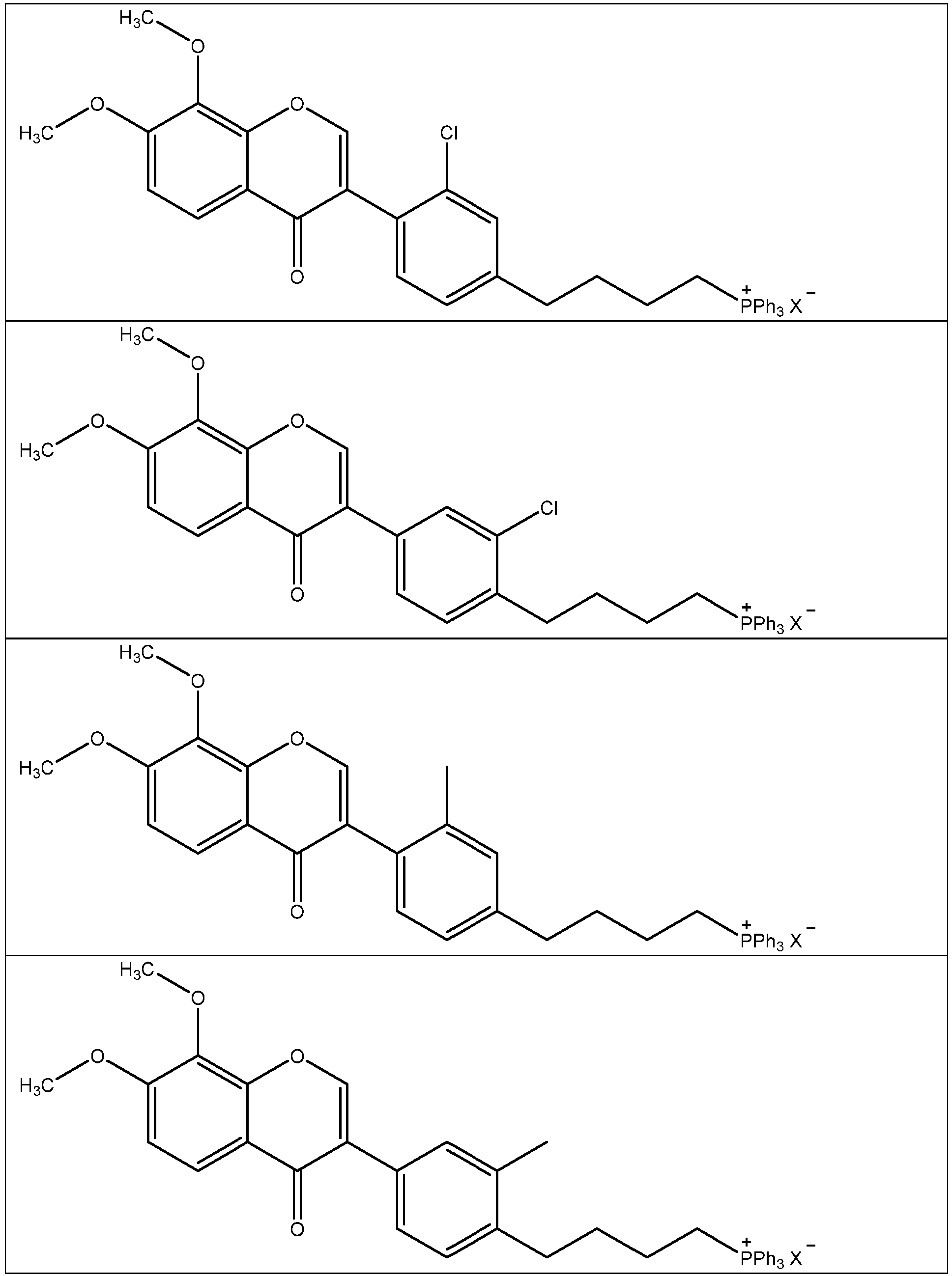

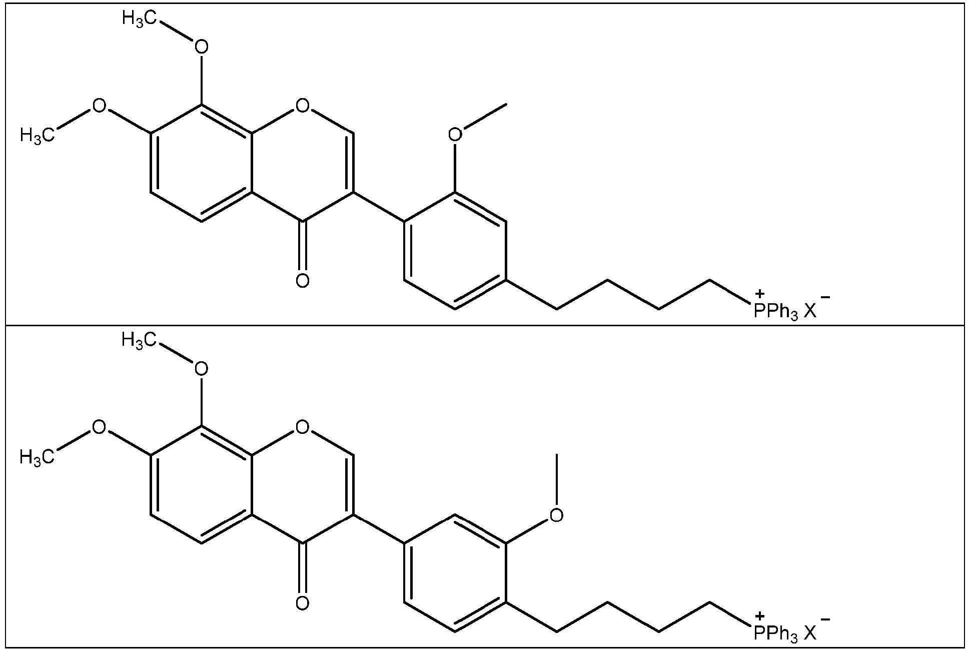

In one embodiment, the compound of formula (1) is selected from:

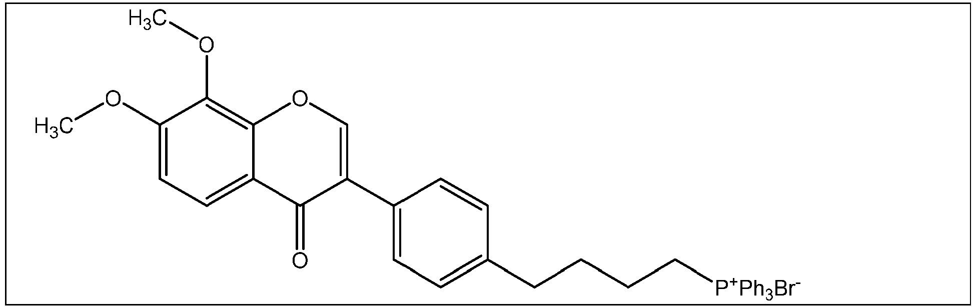

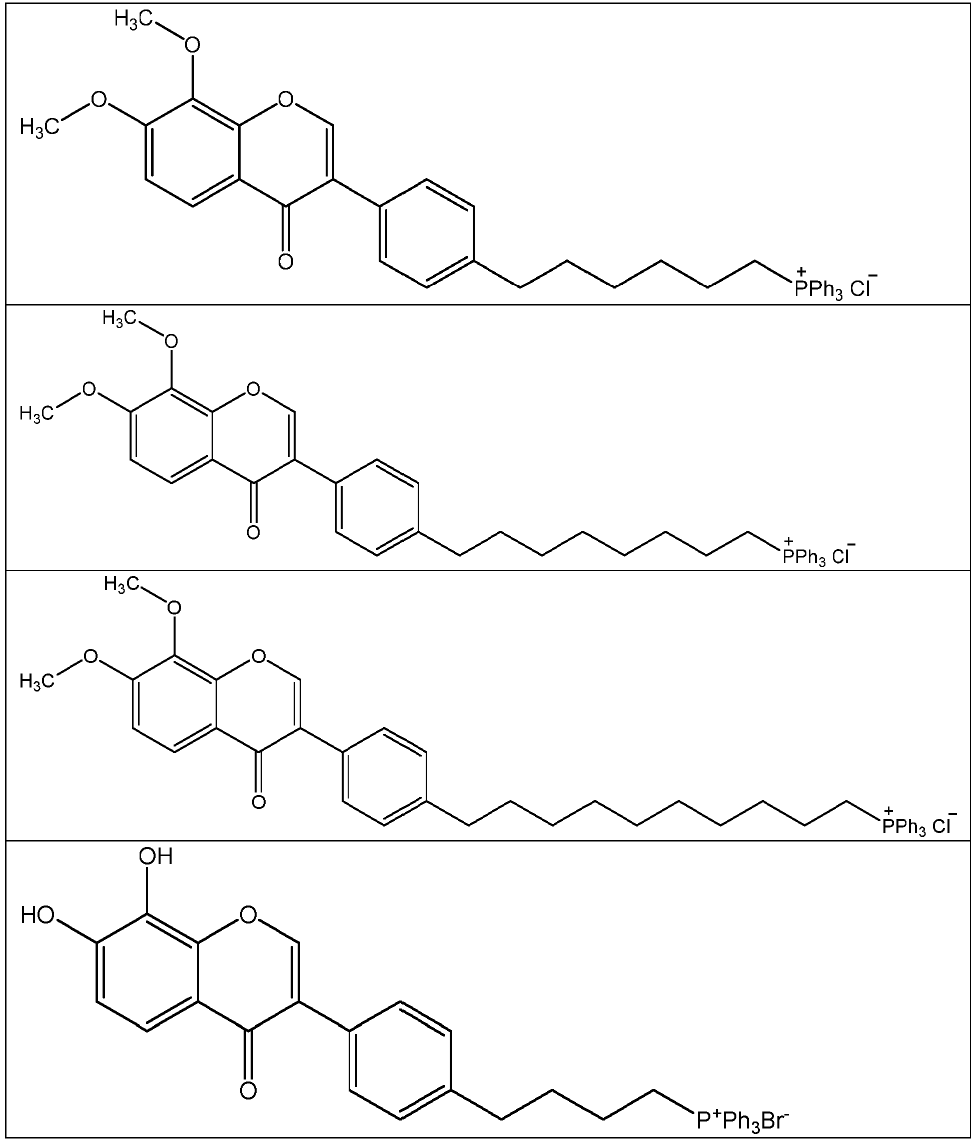

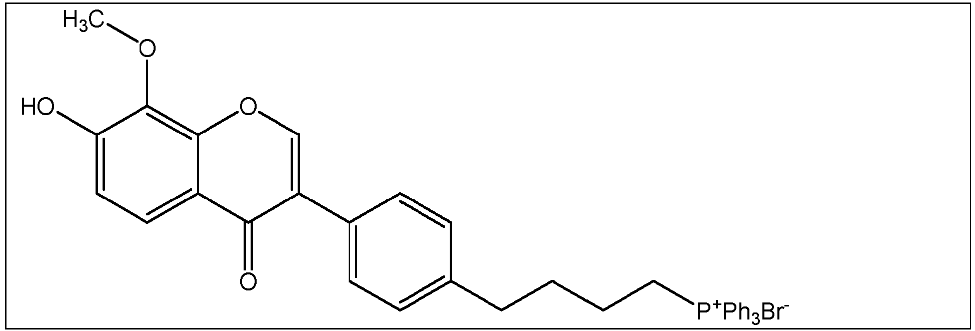

In one embodiment, the compound of formula (1) is selected from:

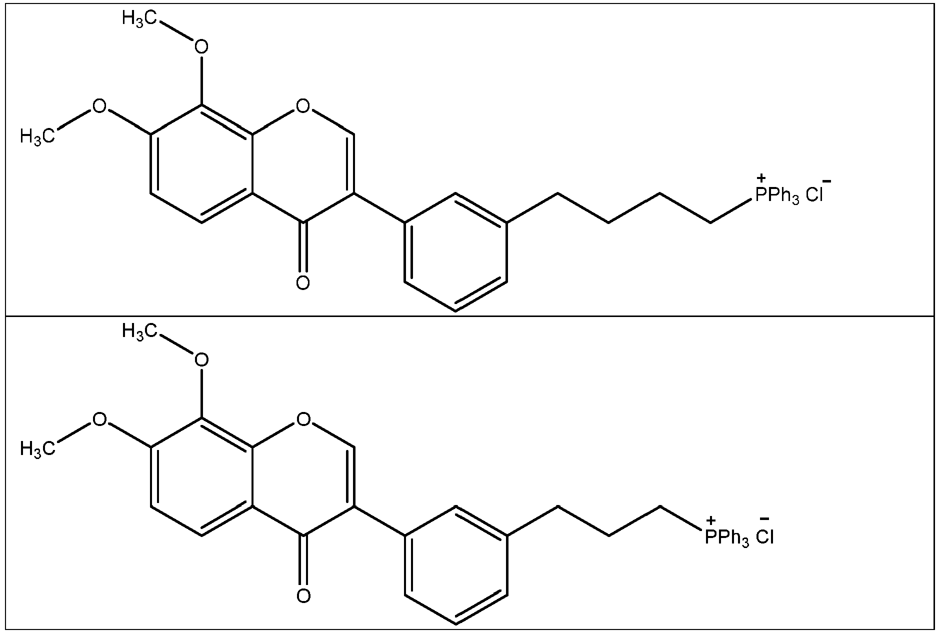

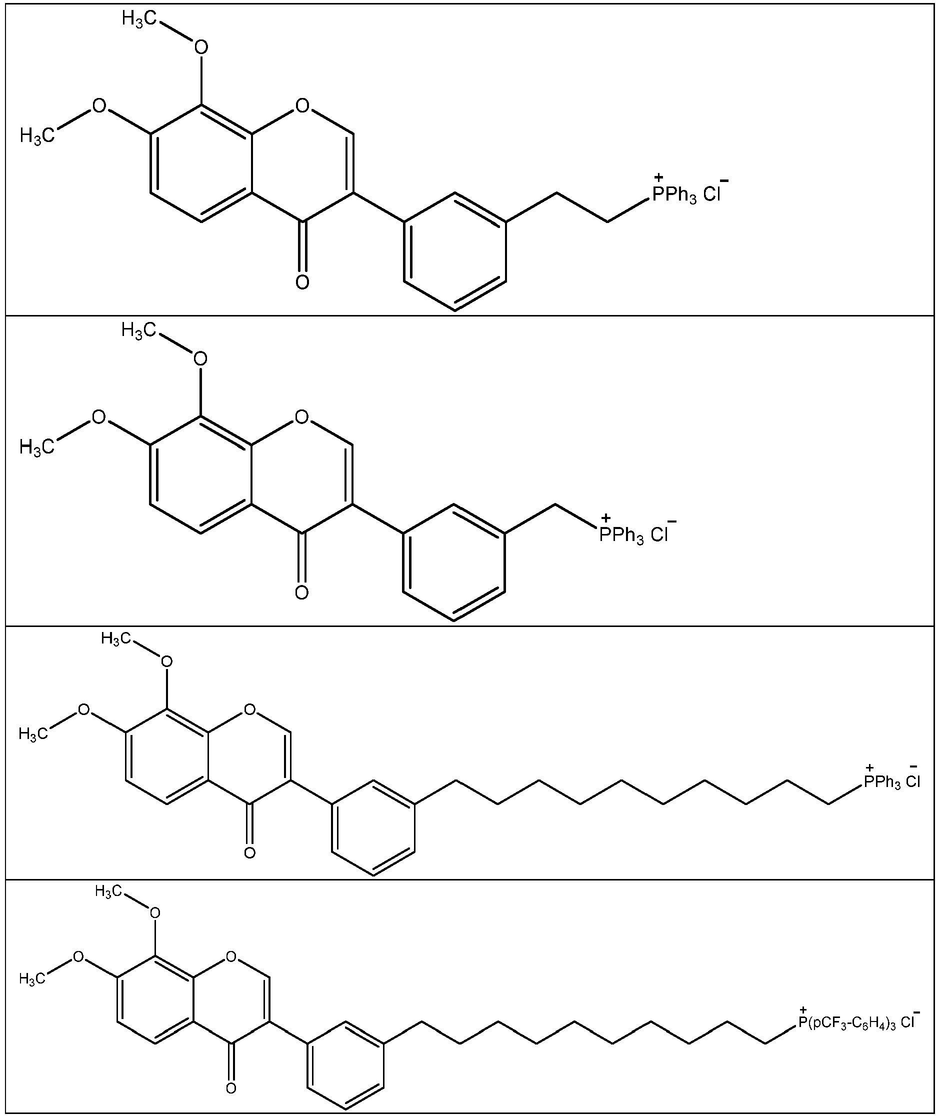

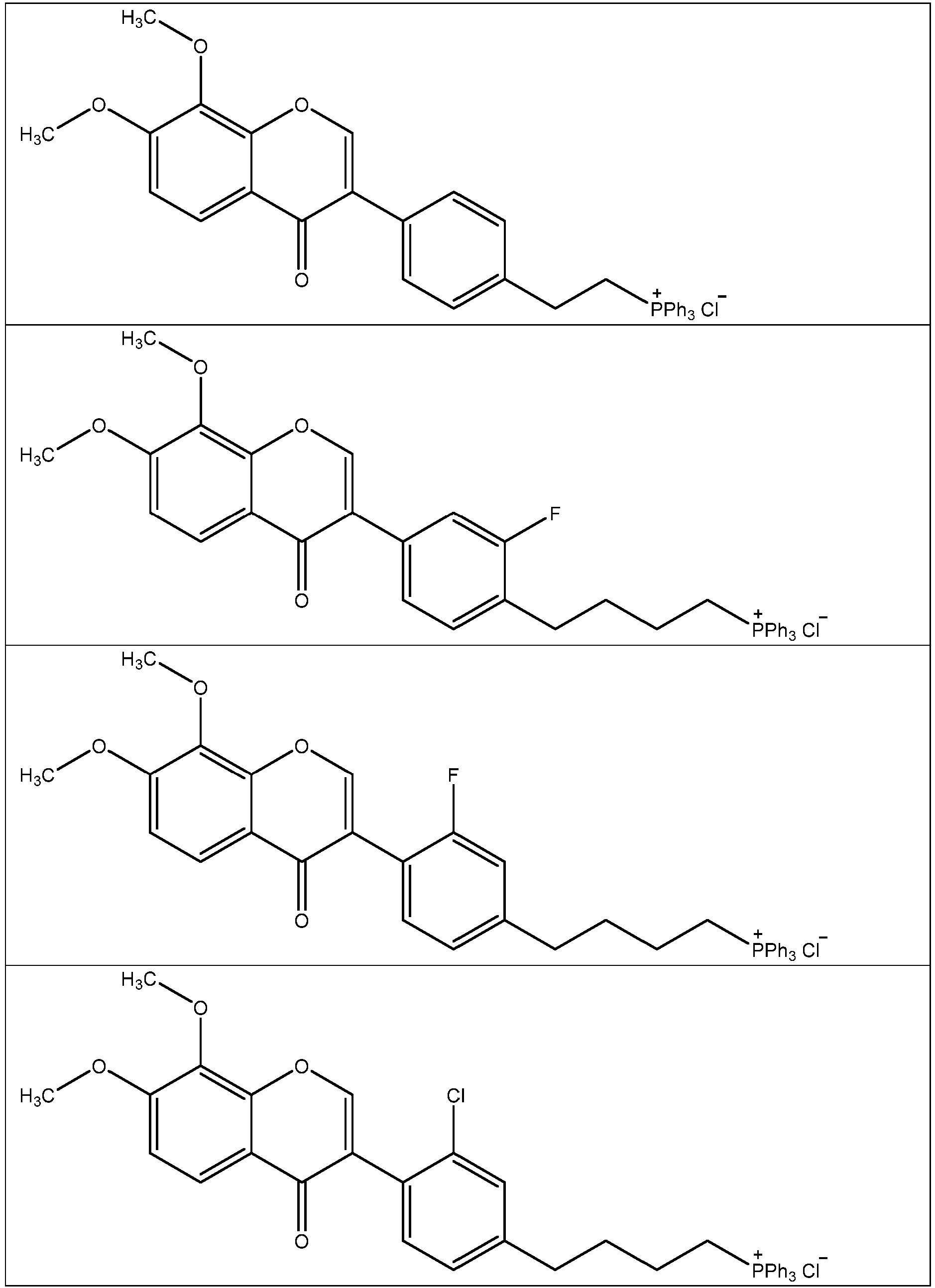

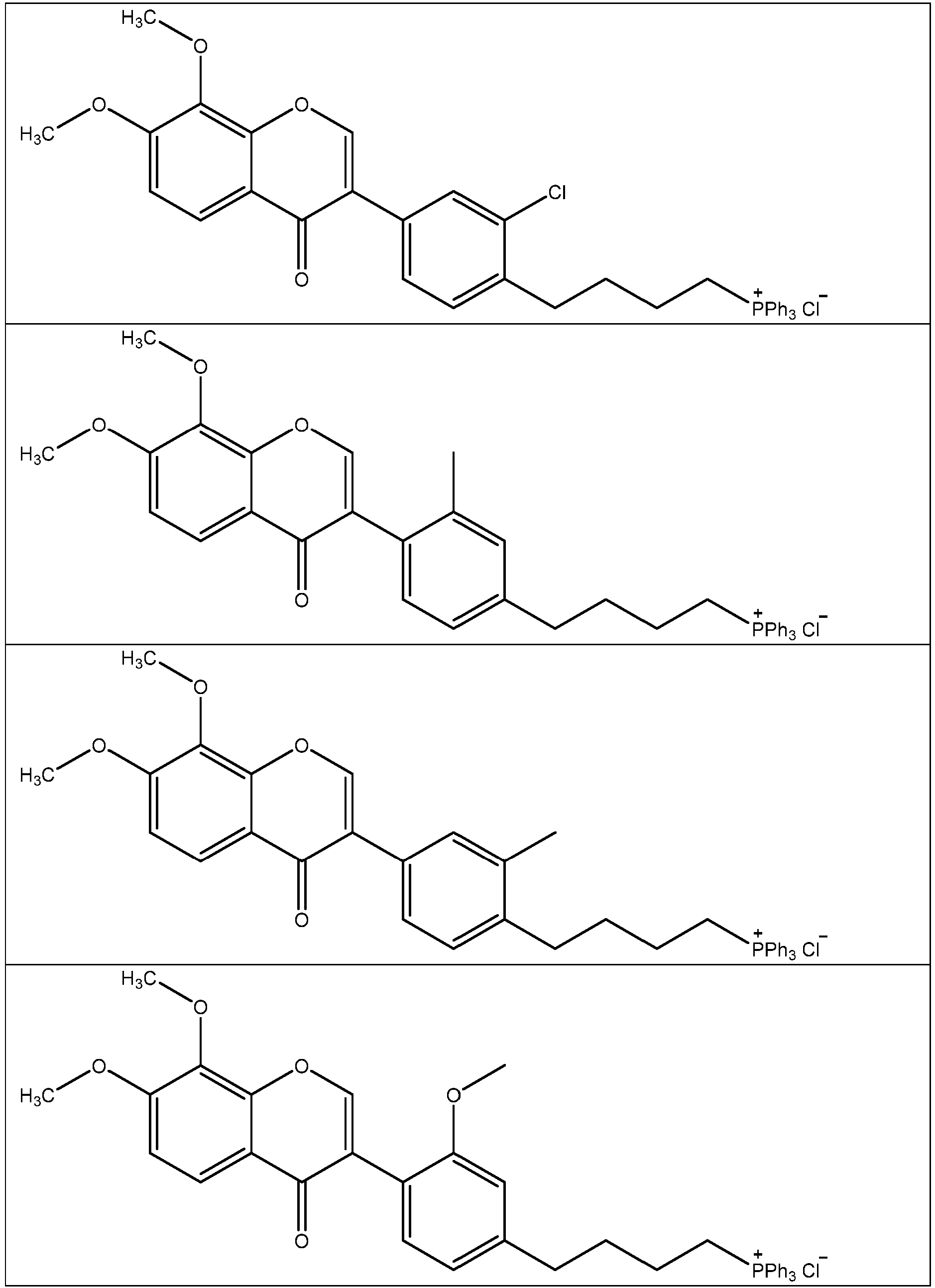

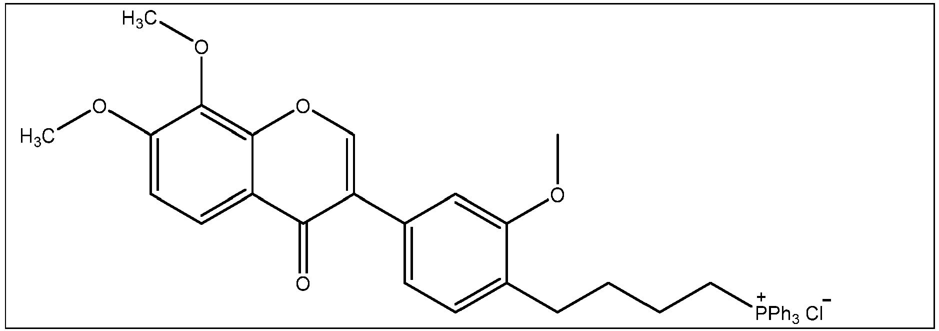

The compounds include a quaternary phosphonium group and X is a counter anion. Preferably, the counter anion X may be any pharmaceutically acceptable, non-toxic counter ion. For example, X may be bromide or chloride, or X may be bromide. For example, X may be chloride.

The counter anion may optionally be singly, doubly or triply charged. As the quaternary group is singly charged, if the counter anion is triply charged then the stoichiometric ratio of the quaternary group to counter anion will typically be 3:1 and if the counter anion is doubly charged then the stoichiometric ratio of the quaternary group to counter anion will typically be 2:1. If both the quaternary group and the counter anion are singly charged then the stoichiometric ratio of the quaternary group to counter anion will typically be 1:1.

In one embodiment, the counter anion will be a singly charged anion. Suitable anions X include but are not limited to halides (for example fluoride, chloride, bromide or iodide) or other inorganic anions (for example nitrate, perchlorate, sulfate, bisulfate, or phosphate) or organic anions (for example propianoate, butyrate, glycolate, lactate, mandelate, citrate, acetate, benzoate, salicylate, succinate, malate, tartrate, fumarate, maleate, hydroxymaleate, galactarate, gluconate, pantothenate, pamoate, methanesulfonate, trifluoromethanesulfonare, ethanesulfonare, 2-hydroxyethanesulfonate, benzenesulfonate, toluene-p-sulfonate, naphthalene-2-sulfonate, camphorsulfonate, ornithinate, glutamate or aspartate). The counter anion may be fluoride, chloride, bromide or iodide. For example, X may be bromide or chloride, or X may be bromide.

A second aspect of the invention provides a pharmaceutically acceptable salt, multi-salt, or solvate of any compound of the first aspect of the invention.

The compounds of the present invention can be used both in their quaternary salt form (as a single salt). Additionally, the compounds of the present invention may contain one or more (e.g. one or two) acid addition or alkali addition salts to form a multi-salt. A multi-salt includes a quaternary salt group as well as a salt of a different group of the compound of the invention.

For the purposes of this invention, a "multi-salt" of a compound of the present invention includes an acid addition salt. Acid addition salts are preferably pharmaceutically acceptable, non-toxic addition salts with suitable acids, including but not limited to inorganic acids such as hydrohalogenic acids (for example, hydrofluoric, hydrochloric, hydrobromic or hydroiodic acid) or other inorganic acids (for example, nitric, perchloric, sulfuric or phosphoric acid); or organic acids such as organic carboxylic acids (for example, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic or pamoic acid), organic sulfonic acids (for example, methanesulfonic, trifluoromethanesulfonic, ethanesulfonic, 2-hydroxyethanesulfonic, benzenesulfonic, toluene-p-sulfonic, naphthalene-2-sulfonic or camphorsulfonic acid) or amino acids (for example, ornithinic, glutamic or aspartic acid). The acid addition salt may be a mono-, di-, tri- or multi-acid addition salt. A preferred salt is a hydrohalogenic, sulfuric, phosphoric or organic acid addition salt. A preferred salt is a hydrochloric acid addition salt.

The compounds of the present invention can be used both, in quaternary salt form and their multi-salt form. For the purposes of this invention, a "multi-salt" of a compound of the present invention includes one formed between a protic acid functionality (such as a carboxylic acid group) of a compound of the present invention and a suitable cation. Suitable cations include, but are not limited to lithium, sodium, potassium, magnesium, calcium and ammonium. The salt may be a mono-, di-, tri- or multi-salt. Preferably the salt is a mono- or di-lithium, sodium, potassium, magnesium, calcium or ammonium salt. More preferably the salt is a mono- or di-sodium salt or a mono- or dipotassium salt.

Preferably any salt or multi-salt is a pharmaceutically acceptable non-toxic salt. However, in addition to pharmaceutically acceptable salts and multi-salts, other salts are included in the present invention, since they have potential to serve as intermediates in the purification or preparation of other, for example, pharmaceutically acceptable salts, or are useful for identification, characterisation or purification of the free acid or base.

The compounds, salts and/or multi-salts of the present invention may be anhydrous or in the form of a hydrate (e.g. a hemihydrate, monohydrate, dihydrate or trihydrate) or other solvate. Such solvates may be formed with common organic solvents, including but not limited to, alcoholic solvents e.g. methanol, ethanol or isopropanol.

The compounds, salts, multi-salts, and solvates of the present invention may contain at least one chiral centre. The compounds, salts, multi-salts, and solvates may therefore exist in at least two isomeric forms. The present invention encompasses racemic mixtures of the compounds, salts, multi-salts, and solvates of the present invention as well as enantiomerically enriched and substantially enantiomerically pure isomers. For the purposes of this invention, a "substantially enantiomerically pure" isomer of a compound comprises less than 5% of other isomers of the same compound, more typically less than 2%, and most typically less than 0.5% by weight.

The compounds, salts, multi-salts, and solvates of the present invention may contain any stable isotope including, but not limited to ¹²C, ¹³C, ¹H, ²H (D), ¹⁴N, ¹⁵N, ¹⁶O, ¹⁷O, ¹⁸O, ¹⁹F and ¹²⁷I, and any radioisotope including, but not limited to ¹¹C, ¹⁴C, ³H (T), ¹³N, ¹⁵O, ¹⁸F, ¹²³I, ¹²⁴I, ¹²⁵I and ¹³¹I.

The compounds, salts, multi-salts, and solvates of the present invention may be in any polymorphic or amorphous form.

A third aspect of the invention provides a pharmaceutical composition comprising a compound of the first aspect of the invention, or a pharmaceutically acceptable salt, multi-salt, or solvate of the second aspect of the invention, and a pharmaceutically acceptable excipient.

Conventional procedures for the selection and preparation of suitable pharmaceutical formulations are described in, for example, "Aulton's Pharmaceutics - The Design and Manufacture of Medicines", M. E. Aulton and K. M. G. Taylor, Churchill Livingstone Elsevier, 4th Ed., 2013.

Pharmaceutically acceptable excipients including adjuvants, diluents or carriers that may be used in the pharmaceutical compositions of the invention are those conventionally employed in the field of pharmaceutical formulation, and include, but are not limited to, sugars, sugar alcohols, starches, ion exchangers, alumina, aluminium stearate, lecithin, serum proteins such as human serum albumin, buffer substances such as phosphates, glycerine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose-based substances, polyethylene glycol, sodium carboxymethylcellulose, polyacrylates, waxes, polyethylene-polyoxypropylene-block polymers, polyethylene glycol and wool fat.

A fourth aspect of the invention provides a compound of the first aspect of the invention, or a pharmaceutically acceptable salt, multi-salt, or solvate of the second aspect of the invention, or a pharmaceutical composition of the third aspect of the invention, for use in medicine, and/or for use in the treatment or prevention of a disease, disorder or condition. In one embodiment, the disease, disorder or condition is cancer.

A fifth aspect of the invention provides the use of a compound of the first aspect, a pharmaceutically effective salt, multi-salt, or solvate of the second aspect, or a pharmaceutical composition according to the third aspect, in the manufacture of a medicament for the treatment or prevention of a disease, disorder or condition. Typically the treatment or prevention comprises the administration of the compound, salt, multi-salt, solvate, or pharmaceutical composition to a subject. In one embodiment, the disease, disorder or condition is cancer.

A sixth aspect of the invention provides a method of treatment or prevention of a disease, disorder or condition, the method comprising the step of administering an effective amount of a compound of the first aspect, or a pharmaceutically acceptable salt, multi-salt, or solvate of the second aspect, or a pharmaceutical composition of the third aspect, to thereby treat or prevent the disease, disorder or condition. Typically the administration is to a subject in need thereof. In one embodiment, the disease, disorder or condition is cancer.

A seventh aspect of the invention provides a method of treatment or prevention of a disease, disorder or condition, the method comprising the step of administering an effective amount of a compound according to formula (1) as defined herein, or a pharmaceutically acceptable salt, multi-salt, or solvate of the second aspect, or a pharmaceutical composition of the third aspect, to thereby treat or prevent the disease, disorder or condition. Typically the administration is to a subject in need thereof. In one embodiment, the disease, disorder or condition is cancer.

In general embodiments, the disease, disorder or condition is cancer.

In one embodiment, the cancer is brain cancer, breast cancer, colon cancer, leukaemia, lung cancer, lymphoma, pancreatic cancer, sarcoma or skin cancer (melanoma).

In one embodiment the cancer is brain cancer.

In one embodiment the cancer is breast cancer.

In one embodiment the cancer is colon cancer.

In one embodiment the cancer is leukaemia.

In one embodiment the cancer is lung cancer.

In one embodiment the cancer is lymphoma.

In one embodiment the cancer is pancreatic cancer.

In one embodiment the cancer is sarcoma.

In one embodiment the cancer is skin cancer (melanoma).

The term "treatment" as used herein refers equally to curative therapy, and ameliorating or palliative therapy. The term includes obtaining beneficial or desired physiological results, which may or may not be established clinically. Beneficial or desired clinical results include, but are not limited to, the alleviation of symptoms, the prevention of symptoms, the diminishment of extent of disease, the stabilisation (i.e., not worsening) of a condition, the delay or slowing of progression/worsening of a condition/symptoms, the amelioration or palliation of the condition/symptoms, and remission (whether partial or total), whether detectable or undetectable. The term "palliation", and variations thereof, as used herein, means that the extent and/or undesirable manifestations of a physiological condition or symptom are lessened and/or time course of the progression is slowed or lengthened, as compared to not administering a compound, multi-salt, solvate, or pharmaceutical composition of the present invention. The term "prevention" as used herein in relation to a disease, disorder or condition, relates to prophylactic or preventative therapy, as well as therapy to reduce the risk of developing the disease, disorder or condition. The term "prevention" includes both the avoidance of occurrence of the disease, disorder or condition, and the delay in onset of the disease, disorder or condition. Any statistically significant avoidance of occurrence, delay in onset or reduction in risk as measured by a controlled clinical trial may be deemed a prevention of the disease, disorder or condition. Subjects amenable to prevention include those at heightened risk of a disease, disorder or condition as identified by genetic or biochemical markers. Typically, the genetic or biochemical markers are appropriate to the disease, disorder or condition under consideration and may include for example, beta-amyloid 42, tau and phosphor-tau.

Unless stated otherwise, in any aspect of the invention, the subject may be any human or other animal. Typically, the subject is a mammal, more typically a human or a domesticated mammal such as a cow, pig, lamb, goat, horse, cat, dog, etc. Most typically, the subject is a human.

Any of the medicaments employed in the present invention can be administered by oral, parental (including intravenous, subcutaneous, intramuscular, intradermal, intratracheal, intraperitoneal, intraarticular, intracranial and epidural), airway (aerosol), rectal, vaginal or topical (including transdermal, buccal, mucosal and sublingual) administration.

Typically, the mode of administration selected is that most appropriate to the disorder or disease to be treated or prevented.

For oral administration, the compounds, multi-salts, or solvates of the present invention will generally be provided in the form of tablets, capsules, hard or soft gelatine capsules, caplets, troches or lozenges, as a powder or granules, or as an aqueous solution, suspension or dispersion.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose. Corn starch and alginic acid are suitable disintegrating agents. Binding agents may include starch and gelatine. The lubricating agent, if present, may be magnesium stearate, stearic acid or talc. If desired, the tablets may be coated with a material, such as glyceryl monostearate or glyceryl distearate, to delay absorption in the gastrointestinal tract. Tablets may also be effervescent and/or dissolving tablets.

Capsules for oral use include hard gelatine capsules in which the active ingredient is mixed with a solid diluent, and soft gelatine capsules wherein the active ingredient is mixed with water or an oil such as peanut oil, liquid paraffin or olive oil.

Powders or granules for oral use may be provided in sachets or tubs. Aqueous solutions, suspensions or dispersions may be prepared by the addition of water to powders, granules or tablets.

Any form suitable for oral administration may optionally include sweetening agents such as sugar, flavouring agents, colouring agents and/or preservatives.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

For parenteral use, the compounds, multi-salts, or solvates of the present invention will generally be provided in a sterile aqueous solution or suspension, buffered to an appropriate pH and isotonicity. Suitable aqueous vehicles include Ringer's solution and isotonic sodium chloride or glucose. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate. The compounds of the invention may also be presented as liposome formulations.

For transdermal and other topical administration, the compounds, multi-salts, or solvates of the invention will generally be provided in the form of ointments, cataplasms (poultices), pastes, powders, dressings, creams, plasters or patches.

Suitable suspensions and solutions can be used in inhalers for airway (aerosol) administration.

The dose of the compounds, multi-salts, or solvates of the present invention will, of course, vary with the disorder or disease to be treated or prevented. In general, a suitable dose will be in the range of 0.01 to 500 mg per kilogram body weight of the recipient per day. The desired dose may be presented at an appropriate interval such as once every other day, once a day, twice a day, three times a day or four times a day. The desired dose may be administered in unit dosage form, for example, containing 1 mg to 50 g of active ingredient per unit dosage form.

For the avoidance of doubt, insofar as is practicable any embodiment of a given aspect of the present invention may occur in combination with any other embodiment of the same aspect of the present invention. In addition, insofar as is practicable it is to be understood that any preferred, typical or optional embodiment of any aspect of the present invention should also be considered as a preferred, typical or optional embodiment of any other aspect of the present invention.

### EXAMPLES

The following nomenclature is used to refer to the following compounds.

| | |
|---|---|
| SND 470 | 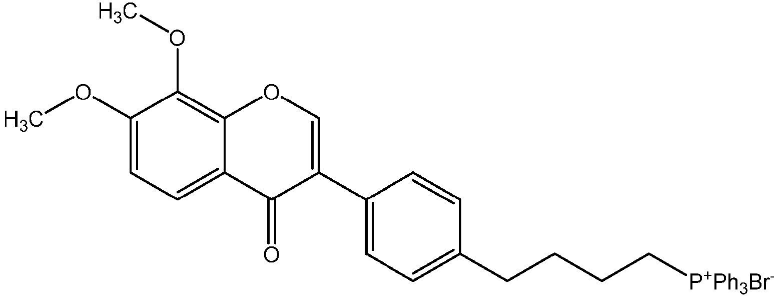 |
| SND 477 | 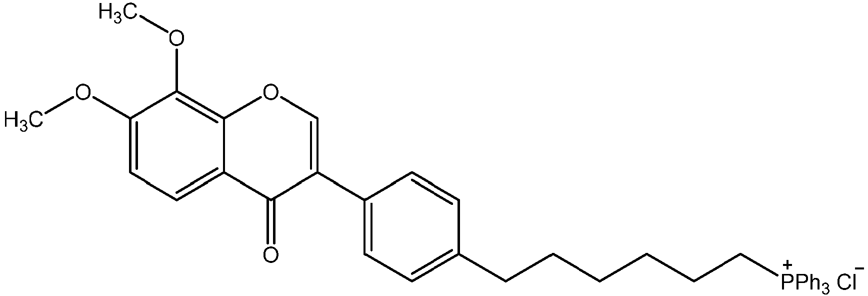 |
| SND 478 | 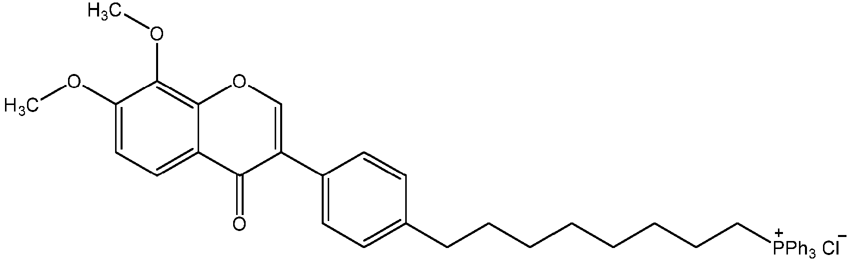 |
| SND 479 | 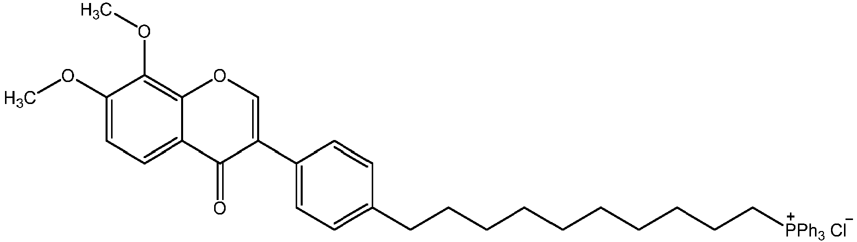 |
| SND 490 | 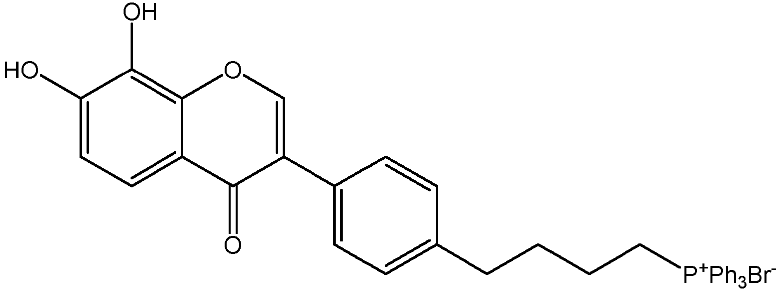 |
| SND 530 | 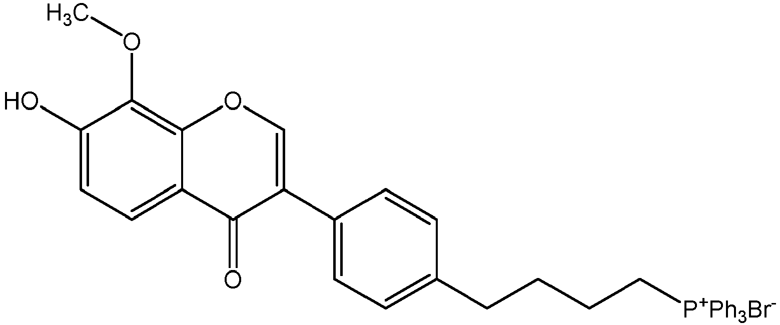 |
| SND 540 | 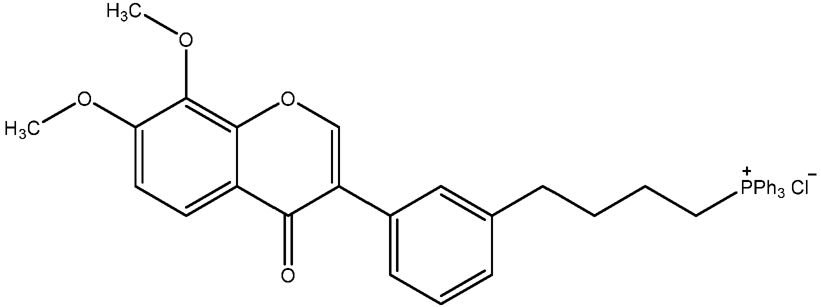 |
| SND 541 | 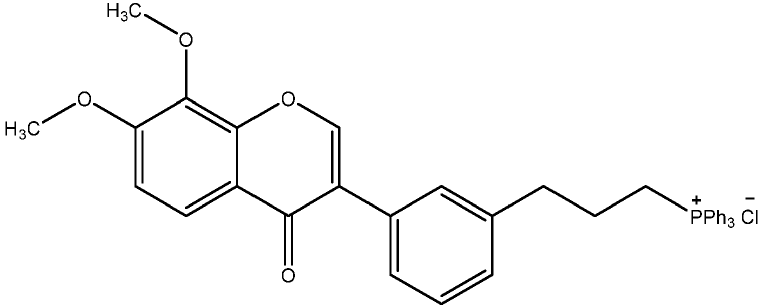 |
| SND 542 | 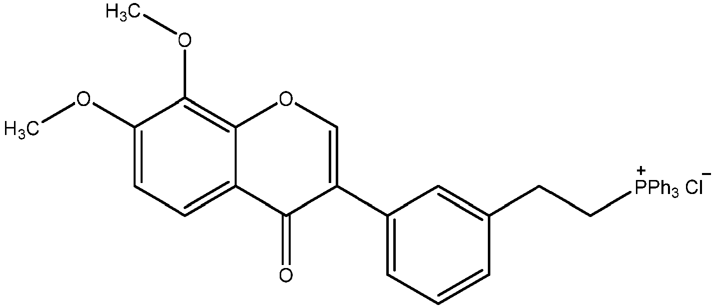 |
| SND 543 | 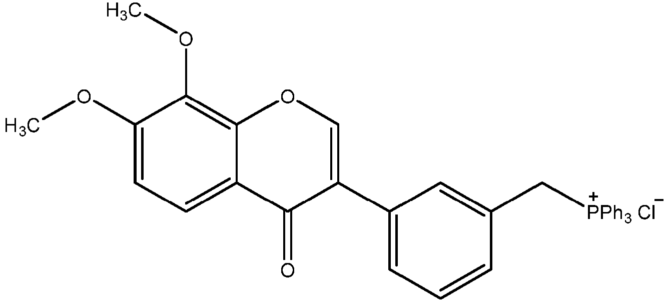 |
| SND 544 | 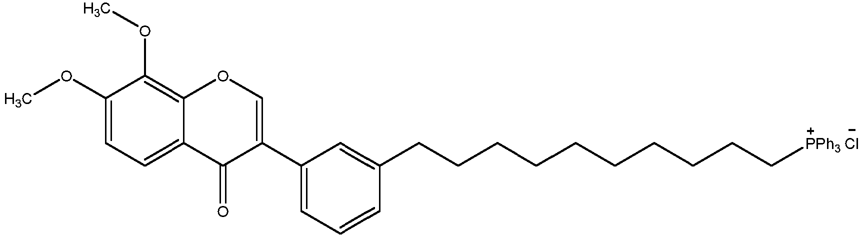 |
| SND 544B | 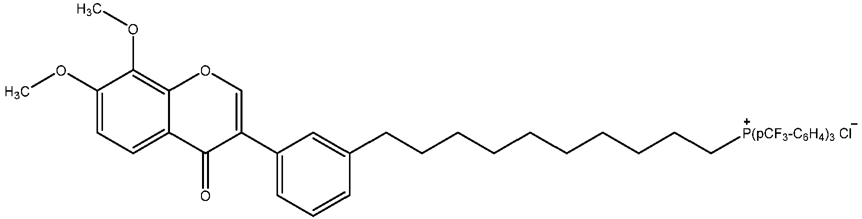 |
| SND 550 | 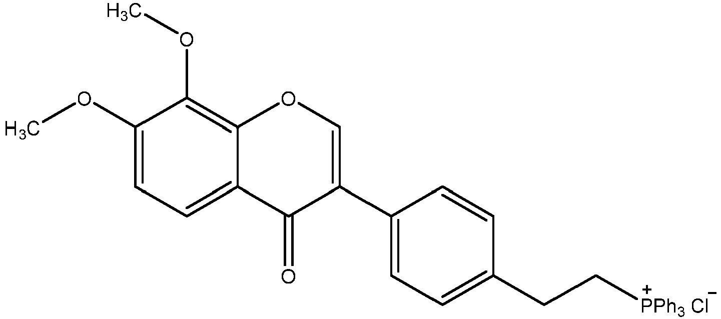 |
| SND 551 | 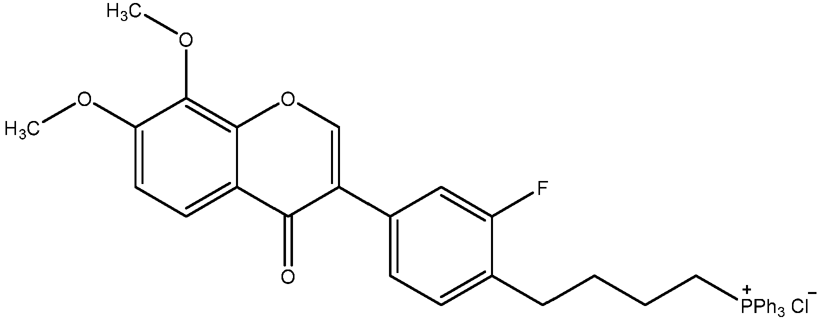 |
| SND 552 | 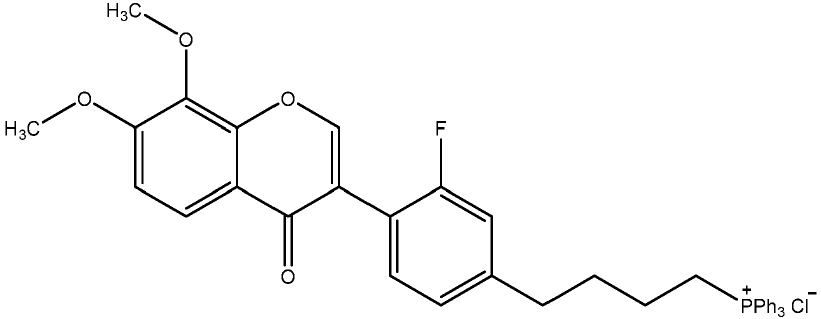 |
| SND 553 | 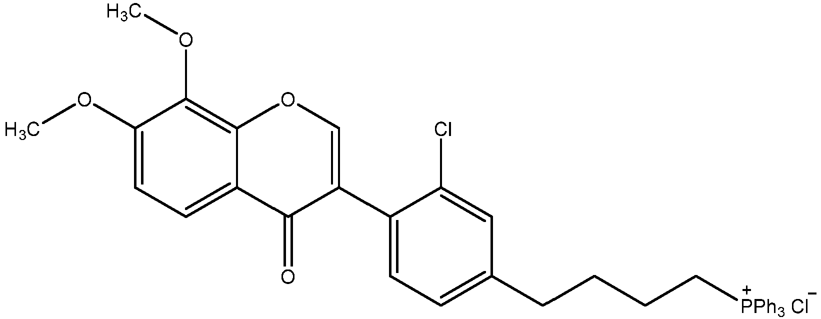 |
| SND 554 | 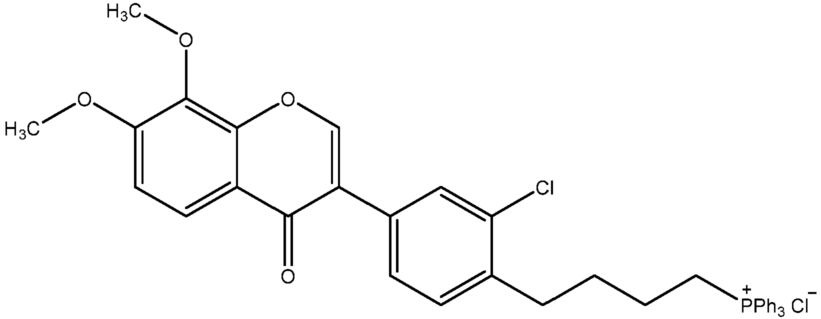 |
| SND 555 | 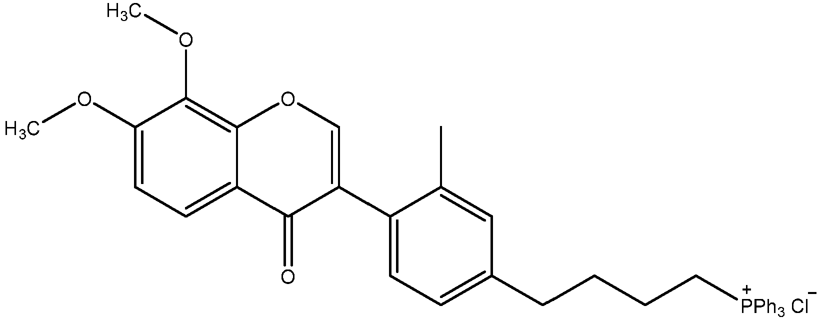 |
| SND 556 | 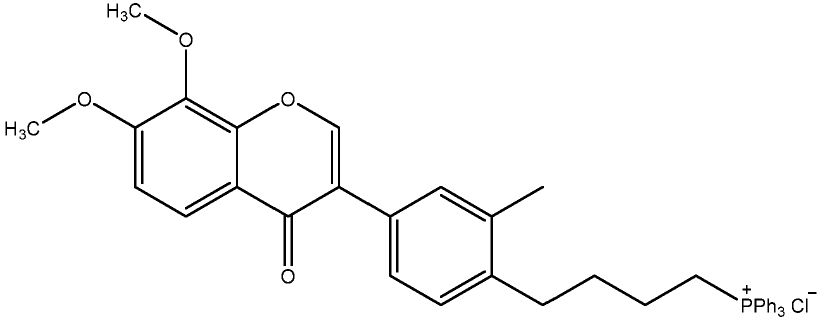 |
| SND 557 | 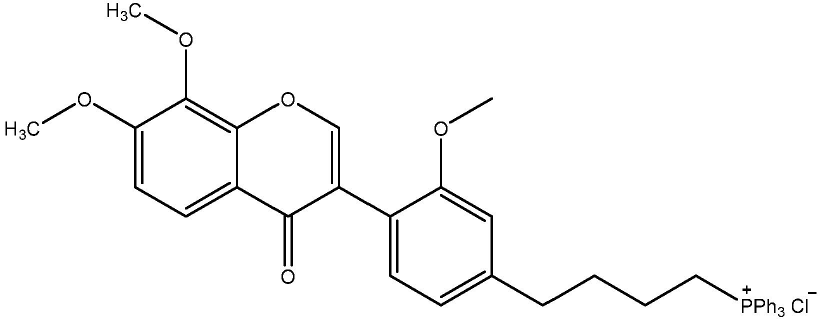 |
| SND 558 | 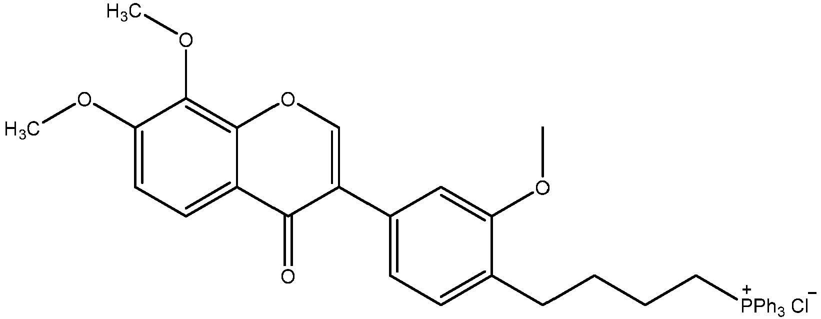 |

### EXAMPLES - COMPOUND SYNTHESIS

Compounds of the invention are synthesised employing a route of synthesis shown below.

### Synthesis of SND470 (Compound 43)

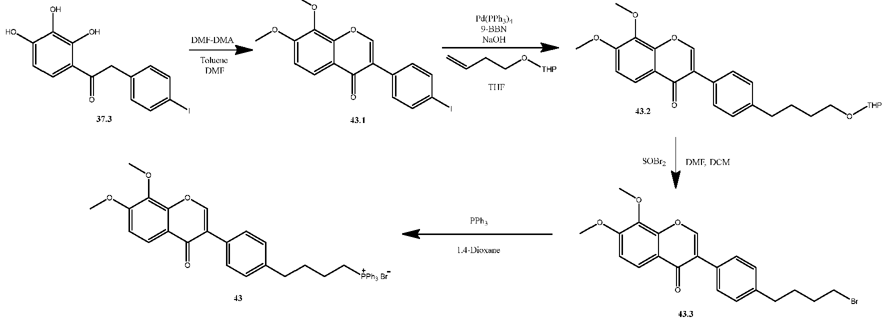

### 3-(4-Iodophenyl)-7,8-dimethoxy-4H-chromen-4-one (43.1).

A suspension of 2-(4-iodophenyl)-1-(2,3,4-trihydroxy-phenyl)ethan-1-one (37.3) (8.90 g, 1 Eq, 24 mmol) in dry toluene (150 mL) and dry DMF (20 mL) was treated with DMF-DMA (11.5 g, 12.8 mL, 4 Eq, 96 mmol) under nitrogen atmosphere and heated to reflux. The mixture was refluxed for 5 h, before additional DMF-DMA (2.87 g, 3.2 mL, 1 Eq, 24 mmol) were added and the mixture was refluxed for additional 5 h. The reaction mixture was allowed to cool to room temperature and concentrated. The residue was diluted with 250 mL of EtOAc and washed with 250 mL of sat. NaHCO₃. The aqueous layer was extracted with 3 x 250 mL of EtOAc. Organic layers were combined, dried with sodium sulfate, filtered and evaporated to dryness. Crude product was treated with 100 mL of hot MeOH, refluxed briefly and allowed to cool overnight. The resultant solid was filtered and washed with 4 x 10 mL of MeOH. The resultant solid was recrystallized in EtOAc/heptane to yield 3-(4-iodophenyl)-7,8-dimethoxy-4*H*-chromen-4-one (43.1) (5.39 g, 12 mmol, 52% yield, 94% purity) as a beige powder.

### 2,2-Diphenyl-7-(4-(4-((tetrahydro-2H-pyran-2-yl)oxy)butyl)phenyl)-6H-[1,3]dioxolo[4,5-h]chromen-6-one (43.2).

2-(But-3-en-1-yloxy)tetrahydro-2*H*-pyran (2.21 g, 1.5 Eq, 14 mmol) was added under nitrogen flow to a solution of 9-BBN (3.458 g, 28.3 mL, 0.5 molar, 1.5 Eq, 14 mmol) in THF. The mixture was stirred at room temperature for 22 h, before NaOH (1.3 mL, 30% Wt, 1.4 Eq, 13 mmol) and a suspension of 3-(4-iodophenyl)-7,8-dimethoxy-4*H-*chromen-4-one (43.1) (3.856 g, 1 Eq, 9.4 mmol) in THF (70 mL) were added. The reaction mixture was then degassed by bubbling nitrogen through the solution, followed by the addition of Pd(PPh3)4 (551 mg, 0.05 Eq, 0.48 mmol). The reaction mixture was degassed again and the mixture was heated at 60 °C for 2.5 h. The reaction mixture was allowed to cool to room temperature, diluted with 250 mL of EtOAc and washed with 250 mL of sat. NaHCO3. The aqueous layer was extracted with 250 mL of EtOAc, organic layers were combined, dried with sodium sulfate, filtered and evaporated to dryness. Crude product was purified twice by normal phase flash chromatography using EtOAc:heptane as the eluent to yield 7,8-dimethoxy-3-(4-(4-((tetrahydro-2*H*-pyran-2-yl)oxy)butyl)phenyl)-4*H*chromen-4-one (43.2) (3.063 g, 6.8 mmol, 72% yield, 97% purity) as an orange oil.

### 3-(4-(4-Bromobutyl)phenyl)-7,8-dimethoxy-4H-chromen-4-one (43.3).

Thionyl bromide (2.20 g, 820 µL, 2.4 Eq, 10.6 mmol) was added under nitrogen flow to a solution of 7,8-dimethoxy-3-(4-(4-((tetrahydro-2*H*-pyran-2-yl)oxy)butyl)phenyl)-4*H*-chromen-4-one (43.2) (1.925 g, 1 Eq, 4.39 mmol) in dry DCM (34 mL) and dry DMF (3.2 g, 3.4 mL, 10 Eq, 44 mmol) at 5 °C. The reaction mixture was stirred for 1 min, before the solution was allowed to warm to room temperature and stirred under nitrogen until it was complete by LC-MS (1.5 h). The reaction mixture was cooled to + 5 °C, quenched with 50 mL of ice-cold water and basified to pH = 8-9 with sat. NaHCO₃. The mixture was extracted with 3 x 50 mL of DCM. Organic layers were combined and washed with 75 mL of brine, which in turn was extracted with 25 mL of DCM. Organic layers were combined, dried with sodium sulfate, filtered and evaporated to dryness. Purification by normal phase *flash*-chromatography gave 3-(4-(4-bromobutyl)phenyl)-7,8-dimethoxy-4*H*-chromen-4-one (43.3) (1.621 g, 3.8 mmol, 86% yield, 97% purity) as a white solid.

### (4-(4-(7,8-Dimethoxy-4-oxo-4H-chromen-3-yl)phenyl)butyl)triphenylphosphonium bromide (43).

Triphenyl-phosphine (809 mg, 5.0 Eq, 3.1 mmol) was added to a solution of 3-(4-(4-bromobutyl)phenyl)-7,8-dimethoxy-4H-chromen-4-one (43.3) (258 mg, 1 Eq, 0.62 mmol) in dry 1,4-dioxane (3 mL). The solution was refluxed for 44 h before it was allowed to cool to room temperature. 20 mL of toluene were added and the mixture was sonicated for 45 min. The supernatant was decanted and another 20 mL of toluene were added and the mixture was sonicated briefly again. The beige solid was filtered and washed with 2 x 20 mL of toluene. Crude product was purified by normal phase *flash*-chromatography using DCM:MeOH as the eluent and by preparative reversedphase LC using MeCN:0.05% HBr in water to yield (4-(4-(7,8-dimethoxy-4-oxo-4H-chromen-3-yl)phenyl)butyl)triphenylphosphonium bromide (43) (224 mg, 0.32 mmol, 52% yield, 97% purity) as a beige powder.

### Synthesis of SND490 (Compound 37)

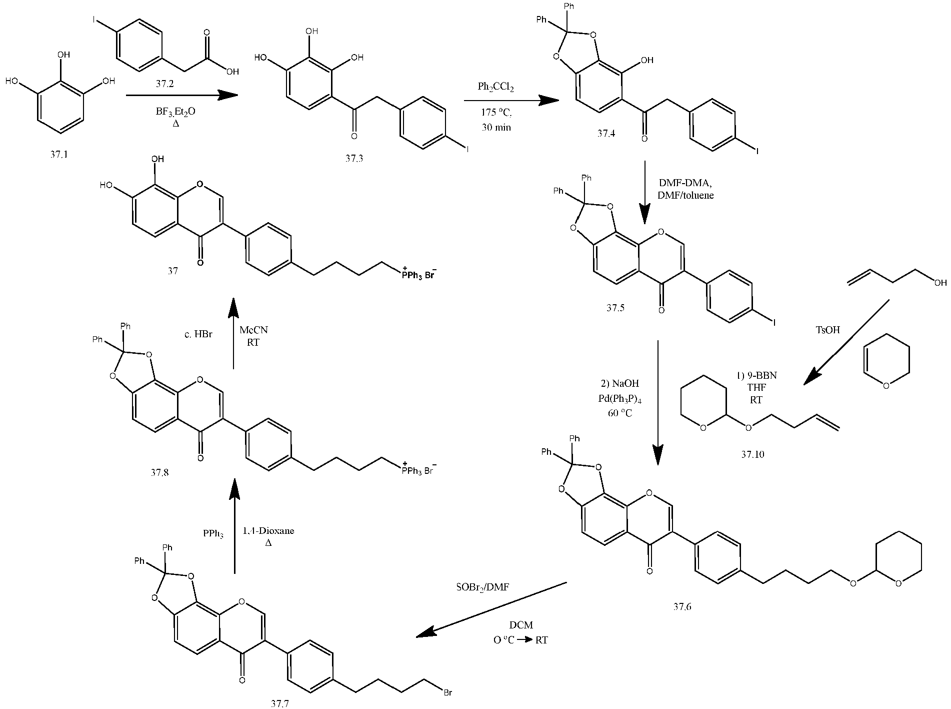

### 2-(4-Iodophenyl)-1-(2,3,4-trihydroxyphenyl)ethan-1-one (37.3).

Pyrogallol (5.51 g, 1 Eq, 43.7 mmol) and 2-(4-iodophenyl)acetic acid (11.42 g, 1 Eq, 43.6 mmol) were treated with boron trifluoride etherate (99.2 g, 88.6 mL, 16 Eq, 699 mmol) under nitrogen flow. The mixture was then heated to 80 °C. After 6 h, the reaction mixture was allowed to cool to room temperature and slowly poured to 250 mL of sat. NaHCO3, which is an exothermic process. The aqueous layer was extracted with 2 × 250 mL of EtOAc. The organic layers were combined and washed multiple times with 250 mL of sat. NaHCO3, until the aqueous layer remained basic. The organic layers were dried with sodium sulfate, filtered and evaporated to dryness. Crude product was dissolved in 30 mL of hot MeOH and diluted with 200 mL of water. The resultant precipitate was filtered, washed with water and dried under air flow. The solid was transferred to a flask and traces of water were co-evaporated off with toluene. The solid was then dried under vacuum before it was suspended in 25 mL of DCM and filtered. The purple solid on the filter was washed with 3 × 10 mL DCM. The filtrate was purified by normal phase *flash*chromatography, using DCM:EtOAc as the eluent, to yield additional material. In total, 2-(4-iodophenyl)-1-(2,3,4-trihydroxyphenyl)ethan-1-one (37.3) (10.45 g, 27 mmol, 61% yield, 95% purity) was obtained as a beige powder.

### 1-(4-Hydroxy-2,2-diphenylbenzo[d][1,3]dioxol-5-yl)-2-(4-iodophenyl)ethan-1-one (37.4).

2-(4-Iodophenyl)-1-(2,3,4-trihydroxyphenyl)ethan-1-one (37.3) (2.956 g, 1 Eq, 8.0 mmol), dichlorodiphenylmethane (2.00 g, 1.62 mL, 1.06 Eq, 8.43 mmol) and diphenyl ether (18 mL) were heated to 175 °C for 30 min before it was allowed to cool to room temperature. The reaction mixture was poured to 180 mL of heptane and allowed to precipitate for several hours. The resultant precipitate was filtered, washed with 4 × 25 mL of heptane and dried to yield 1-(4-hydroxy-2,2-diphenylbenzo[*d*][1,3]dioxol-5-yl)-2-(4-iodophenyl)ethan-1-one (37.4) (3.15 g, 5.8 mmol, 72% yield, 98% purity) as a brown solid.

### 7-(4-Iodophenyl)-2,2-diphenyl-6H-[1,3]dioxolo[4,5-h]chronien-6-one (37.5).

1-(4-Hydroxy-2,2-diphenylbenzo[d]-[1,3]dioxol-5-yl)-2-(4-iodophenyl)ethan-1-one (37.4) (3.15 g, 1 Eq, 5.9 mmol) was dissolved in a mixture of dry toluene (56 mL) and dry DMF (6 mL). DMF-DMA (1.76 g, 1.96 mL, 2.5 Eq, 15 mmol) was then added to the solution and the reaction mixture was refluxed under nitrogen for 1.5 h before it was stopped and allowed to cool to room temperature. The reaction mixture was evaporated to dryness, suspended in 250 mL of EtOAc and washed with 250 mL of sat. NaHCO₃. The aqueous layer was extracted with 3 × 250 mL of EtOAc. Organic layers were dried with sodium sulfate, filtered and concentrated. Crude product was purified by normal phase *flash*chromatography, using DCM:EtOAc as the eluent, to yield 7-(4-iodophenyl)-2,2-diphenyl-6*H*-[1,3]dioxolo[4,5-*h*]chromen-6-one (37.5) (2.80 g, 5.1 mmol, 86% yield, 99% purity) as a beige solid.

### 2-(But-3-en-1-yloxy)tetrahydro-2H-pyran (37.10).

*p*-Toluenesulfonic acid monohydrate (442 mg, 0.02 Eq, 2.32 mmol) and 3,4-dihydro-2*H*-pyran (19 g, 21 mL, 2 Eq, 0.23 mol) were added to a solution of but-3-en-1-ol (8.38 g, 10 mL, 1 Eq, 116 mmol) in DCM (50 mL) at 0 °C. After 2 h at 0 °C, the reaction mixture was quenched with 50 mL of sat. NaHCO3, diluted with 50 mL of DCM and the layers were separated. The organic layer was washed with 50 mL of water and 50 mL of brine, before it was dried with sodium sulfate, filtered and evaporated to dryness. Purification by normal phase *flash*-chromatography, using heptane:EtOAc as the eluent, gave 2-(but-3-en-1-yloxy)tetrahydro-2*H*-pyran (37.10) (13.75 g, 88 mmol, 76% yield) as a transparent oil.

### 2,2-Diphenyl-7-(4-(4-((tetrahydro-2H-pyran-2-yl)oxy)butyl)phenyl)-6H-[1,3]dioxolo[4,5-h]chromen-6-one (37.6).

2-(But-3-en-1-yloxy)tetrahydro-2*H*-pyran (37.10) (1.20 g, 1.5 Eq, 7.7 mmol) was added under nitrogen flow to a solution of 9-BBN (1.89 g, 15.5 mL, 0.5 molar, 1.5 Eq, 7.75 mmol) in THF. The solution was stirred at room temperature under nitrogen for 70 h before NaOH (0.96 g, 0.72 mL, 30% Wt, 1.4 Eq, 7.2 mmol) and a solution of 7-(4-iodophenyl)-2,2-diphenyl-6*H*-[1,3]dioxolo[4,5-*h*]chromen-6-one (37.5) (2.807 g, 1 Eq, 5.16 mmol) in THF (40 mL) were added. The reaction mixture was degassed, followed by the addition of Pd(PPh3)4 (469 mg, 0.08 Eq, 0.41 mmol). The reaction mixture was degassed again before heating the mixture at 60 °C for 2 h. The reaction mixture was allowed to cool to room temperature, diluted with 150 mL of EtOAc and washed with 150 mL of sat. NaHCO3. The aqueous layer was then extracted with another 150 mL of EtOAc. Organic fractions were combined, dried with sodium sulfate, filtered and evaporated to dryness. Purification by normal phase *flash-*chromatography, using DCM:EtOAc as the eluent, gave 2,2-diphenyl-7-(4-(4-((tetrahydro-2*H*-pyran-2-yl)oxy)butyl)phenyl)-6*H*-[1,3]dioxolo-[4,5-*h*]chromen-6-one (37.6) (1.276 g, 2.2 mmol, 43% yield, 99% purity) as a beige powder.

### 7-(4-(4-Bromobutyl)phenyl)-2,2-diphenyl-6H-[1,3]dioxolo[4,5-h]chromen-6-one (37.7).

Thionyl bromide (1.25 g, 465 µL, 2.4 Eq, 6.0 mmol) was added under nitrogen flow to a solution of 2,2-diphenyl-7-(4-(4-((tetrahydro-2*H*-pyran-2-yl)oxy)butyl)phenyl)-6*H-*[1,3]dioxolo[4,5-*h*]chromen-6-one (37.6) (1.44 g, 1 Eq, 2.51 mmol) in a dry DMF (1.8 g, 1.9 mL, 9.8 Eq, 25 mmol) and DCM (20 mL) mixture at 5 °C. The reaction mixture was then allowed to warm to room temperature and stirred for 2.5 h before additional thionyl bromide (0.10 g, 39 µL, 0.20 Eq, 0.50 mmol) was added and the mixture was stirred for another 1 h. The reaction mixture was cooled with an ice-bath, quenched with 40 mL of water and basified with NaHCO3 until pH = 8 - 9. The mixture was then extracted with 4 × 30 mL of DCM. Organic layers were combined and washed with 75 mL of brine, which in turn was extracted with 15 mL of DCM. Organic layers were combined, dried with sodium sulfate, filtered and concentrated. Purification by normal phase *flash*-chromatography, using DCM:EtOAc as the eluent, gave 7-(4-(4-bromobutyl)phenyl)-2,2-diphenyl-6*H*-[1,3]-dioxolo[4,5-*h*]chromen-6-one (37.7) (1.306 g, 2.3 mmol, 93% yield, 99% purity) as a beige foam.

### (4-(4-(6-Oxo-2,2-diphenyl-6H-[1,3]dioxolo[4,5-h]chromen-7-yl)phenyl)butyl)triphenylphosphonium bromide (37.8).

Triphenylphosphine (579 mg, 5.0 Eq, 2.2 mmol) was added to a solution of 7-(4-(4-bromobutyl)phenyl)-2,2-diphenyl-6*H*-[1,3]dioxolo[4,5-*h*]chromen-6-one (37.7) (245 mg, 1 Eq, 0.44 mmol) in dry 1,4-dioxane (3 mL) under nitrogen. The solution was refluxed for 38 h before it was allowed to cool to room temperature. The oily residue at the bottom of the flask was diluted with 25 mL of toluene and sonicated for 30 min. The resultant solid was filtered and washed 2 x 25 mL of toluene and 25 mL of Et2O. Crude product was purified by normal phase *flash*-chromatography, using DCM:MeOH as the eluent, to yield (4-(4-(6-oxo-2,2-diphenyl-6*H*-[1,3]dioxolo[4,5-*h*]chromen-7-yl)phenyl)butyl)-triphenylphosphonium bromide (37.8) (299 mg, 0.36 mmol, 80% yield, 97% purity) as a beige solid.

### (4-(4-(7,8-Dihydroxy-4-oxo-4H-chromen-3-yl)phenyl)butyl)triphenylphosphonium bromide (37).

A solution of (4-(4-(6-oxo-2,2-diphenyl-6*H*-[1,3]dioxolo[4,5-*h*]chromen-7-yl)phenyl)butyl)triphenylphosphonium bromide (37.8) (299 mg, 1 Eq, 0.37 mmol) in MeCN (3 mL) was treated with c. HBr (0.95 mL, 48% Wt, 23 Eq, 8.4 mmol) and stirred under nitrogen at room temperature for 2 h. The reaction mixture was concentrated and traces of water were removed azeotropically with 2 x 5 mL of MeCN. The oily residue was suspended in 8 mL of DCM and evaporated to dryness. The resultant solid was suspended in 2 mL of MeCN, filtered and dried in vacuum to yield (4-(4-(7,8-dihydroxy-4-oxo-4*H*-chromen-3-yl)phenyl)butyl)triphenylphosphonium bromide (37) (159 mg, 0.24 mmol, 66% yield, 99% purity) as a white powder.

### Synthesis of SND530 (Compound 38)

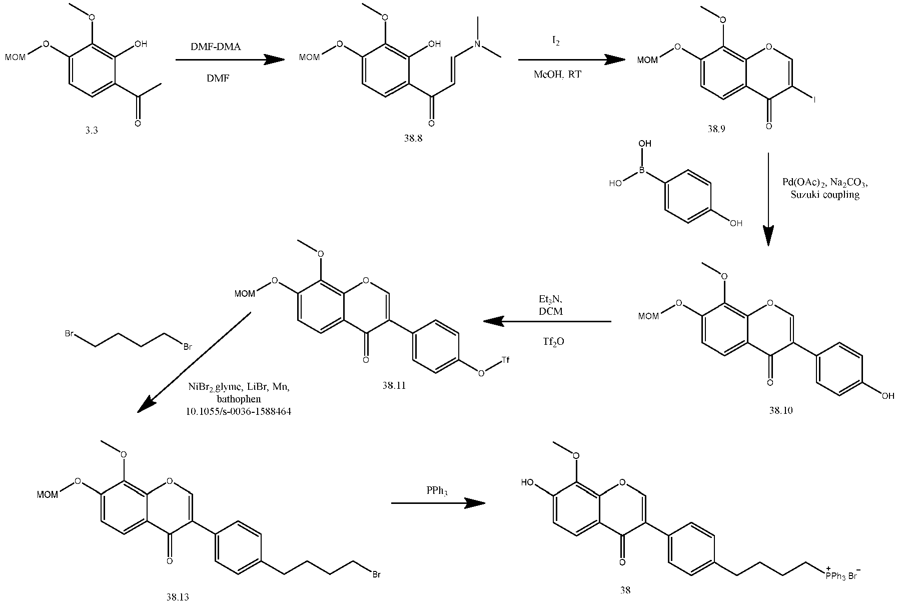

### (E)-3-(Dimethylamino)-1-(2-hydroxy-3-methoxy-4-(methoxymethoxy)phenyl)prop-2-en-1-one (38.8).

To a solution of 1-(2-hydroxy-3-methoxy-4-(methoxymethoxy)phenyl)ethan-1-one (3.3) (5.00 g, 1.00 Eq, 22.1 mmol) in DMF (40 mL) was added DMF-DMA (13.2 g, 14.7 mL, 5.00 Eq, 111 mmol) dropwise. The resulting mixture was heated to 74 °C overnight. Upon reaction completion was confirmed by LCMS, the reaction mixture was cooled down, quenched with water, and extracted with EtOAc (5 × 100 mL). The combined extracts were washed with brine, dried over Na2SO4, filtered, and concentrated in *vacuo.* This yielded sufficiently pure (*E*)-3-(dimethylamino)-1-(2-hydroxy-3-methoxy-4-(methoxymethoxy)phenyl)prop-2-en-1-one (38.8) (6.20 g, 22 mmol, 99%) as a yellow crystalline solid.

### 3-Iodo-8-methoxy-7-(methoxymethoxy)-4H-chromen-4-one (38.9).

A solution (*E*)-3-(dimethylamino)-1-(2-hydroxy-3-methoxy-4-(methoxymethoxy)phenyl)prop-2-en-1-one (38.8) (6.20g, 1.00 Eq, 22 mmol) and iodine (8.11 g, 1.45 Eq, 32 mmol) in methanol (100 mL) was stirred for 16 hours at room temperature. Upon conversion was confirmed by LCMS, the reaction mixture was concentrated in *vacuo,* to give a dark-red residue. To remove residual iodine, the resulting material was treated with saturated sodium sulphite solution, until the mixture became clear. The mixture was then extracted with DCM (3 × 40 mL), and the extracts were dried over sodium sulfate, filtered, and concentrated under reduced pressure. This yielded sufficiently pure 3-iodo-8-methoxy-7-(methoxymethoxy)-4*H-*chromen-4-one (38.9) (6.99 g, 19 mmol, 88%) as a yellow solid.

### 4,4,5,5-Tetramethyl-2-(4-(4-((tetrahydro-2H-pyran-2-yl)oxy)butyl)phenyl)-1,3,2-dioxaborolane (38.16).

To a mixture of 2-(4-(4-bromophenyl)butoxy)tetrahydro-2*H*-pyran (8.5) (2.00 g, 1.00 Eq, 6.39mmol), bis(pinacolato)diboron (1.78 g, 1.10 Eq, 7.02 mmol), Pd(dppf)Cl2-CH2Cl2 adduct (1.04 g, 0.20 Eq, 1.28 mmol), and potassium acetate (1.88 g, 3.00 Eq, 19.2 mmol) was added degassed DMF. The resulting solution was further degassed, and then warmed up to 80 °C and stirred for 18 hours. Upon conversion was confirmed by TLC, the reaction mixture was cooled and poured into water, and then extracted with ethyl acetate (3 × 100 mL). The organic extracts were washed with water and brine (100 mL each), dried over sodium sulfate, filtered, and evaporated. The residual oil was purified by normal-phase column chromatography (silica, gradient of ethyl acetate in heptane), yielding the desired 4,4,5,5-tetramethyl-2-(4-(4-((tetrahydro-2*H*-pyran-2-yl)oxy)butyl)phenyl)-1,3,2-dioxaborolane (38.16) (1.86 g, 5.17 mmol, 81%) as a transparent yellowish oil.

### 8-Methoxy-7-(methoxymethoxy)-3-(4-(4-((tetrahydro-2H-pyran-2-yl)oxy)butyl)phenyl)-4H-chromen-4-one (38.15).

Upon continuous flow of nitrogen through 20 mL of 1,4-dioxane solution of 4,4,5,5-tetramethyl-2-(4-(4-((tetrahydro-2*H*-pyran-2-yl)oxy)butyl)phenyl)-1,3,2-dioxaborolane (38.16) (1.74 g, 2.50 Eq, 4.83 mmol), Na2CO3 (492 mg, 2.40 Eq, 4.64 mmol) was added, followed by degassed solution of 3-iodo-8-methoxy-7-(methoxymethoxy)-4*H*-chromen-4-one (38.9) (700 mg, 1.00 Eq, 1.93 mmol) in dry 1,4-dioxane (3 mL), and degassed solution of fresh palladium(II) acetate (22 mg, 0.05 Eq, 97 µmol) in dry 1,4-dioxane (1 mL). The resulting mixture was degassed for additional 10 minutes, before heating it to 60 °C. Heating was continued for 18 hours. Then, 10 mL of methanol were added*, and the resulting mixture was left stirring under inert atmosphere, while gradually cooling down to room temperature. The mixture was diluted with EtOAc (200 mL), and washed with saturated NaHCO3 (100 mL). Aqueous layer was extracted with additional volume of EtOAc (200 mL), and combined organic layers were washed with brine (50 mL), dried over Na2SO4, filtered, and concentrated under reduced pressure. The obtained crude product was purified by normal-phase column chromatography (silica, gradient of dichloromethane in ethyl acetate), appropriate fractions were combined and concentrated, yielding 8-methoxy-7-(methoxymethoxy)-3-(4-(4-((tetrahydro-2*H*pyran-2-yl)oxy)butyl)phenyl)-4*H-*chromen-4-one (38.15) (636 mg, 1.36 mmol, 70%) as a yellow oil.

### 3-(4-(4-Bromobutyl)phenyl)-7-hydroxy-8-methoxy-4H-chromen-4-one (38.13).

A solution 8-methoxy-7-(methoxymethoxy)-3-(4-(4-((tetrahydro-2*H*-pyran-2-yl)oxy)butyl)phenyl)-4*H*-chromen-4-one (38.15) (753 mg, 1 Eq, 1.61 mmol) in dry DCM (15 mL) and DMF (1.1 g, 1.2 mL, 9.6 Eq, 15 mmol) was cooled to 0°C under nitrogen atmosphere. Then, thionyl bromide (1.1 g, 0.40 mL, 3.2 Eq, 5.2 mmol) was added. After 10 minutes, the cooling bath was removed, and the orange solution was stirred at room temperature. Reaction progress was controlled by LCMS. Upon completion (3 hours), the reaction mixture was cooled with ice-bath, quenched with 30 mL of water, and basified with NaHCO3 to pH = 8 - 9. The aqueous layer was extracted with 3 x 25 mL of DCM. Organic layers were combined and washed with 50 mL of brine, which in turn was extracted with 4 x 20 mL of DCM. Organic layers were combined, dried with sodium sulfate, filtered, and evaporated to dryness. The crude product was suspended in acetonitrile (3 mL) and treated with HCl (0.7 g, 0.5 mL, 37% Wt, 5 Eq, 8 mmol), to ensure full MOM deprotection. LCMS confirmed complete deprotection after 1 hour. The reaction mixture was evaporated to dryness, 10 mL of acetonitrile were added, and the mixture was evaporated to dryness again. Crude product was purified by normal-phase column chromatography (silica, gradient of dichloromethane in ethyl acetate). Appropriate fractions were combined and concentrated, yielding 3-(4-(4-bromobutyl)phenyl)-7-hydroxy-8-methoxy-4*H*-chromen-4-one (38.13) (518 mg, 1.3 mmol, 79%) as an orange oil, which solidifies upon storage, turning into yellow solid.

### (4-(4-(7-Hydroxy-8-methoxy-4-oxo-4H-chromen-3-yl)phenyl)butyl)triphenylphosphonium bromide (38).

To a suspension of 3-(4-(4-bromobutyl)phenyl)-7-hydroxy-8-methoxy-4*H*-chromen-4-one (38.13) (250 mg, 1.00 Eq, 620 µmol) in dry 1,4-dioxane (2 mL) in a sealed vessel was added triphenylphosphine (813 mg, 5.00 Eq, 3.10 mmol). The reaction mixture was heated to 115 °C for 18 hours. Full conversion was confirmed by LCMS and TLC (DCM:MeOH 9:1). Upon completion, the reaction mixture was diluted with toluene and diethyl ether, sonicated (until chunky material turned into pale powder), triturated, and filtered. Resulting solid was rinsed with toluene and diethyl ether, then dried *in vacuo.* Residual solvents were removed by dissolving material in minimal amount of methanol, sonicating, and co-evaporating with an excess of acetonitrile. This process was repeated 4 times, until beige powder remained. Upon drying it was confirmed to be the desired product. It was then purified using normal phase column chromatography (gradient of methanol in DCM) twice, yielding (4-(4-(7-hydroxy-8-methoxy-4-oxo-4*H-*chromen-3-yl)phenyl)butyl)triphenylphosphonium bromide (38) (193 mg, 290 µmol, 47 %) as a white solid, which dissolves well in methanol, and DMSO.

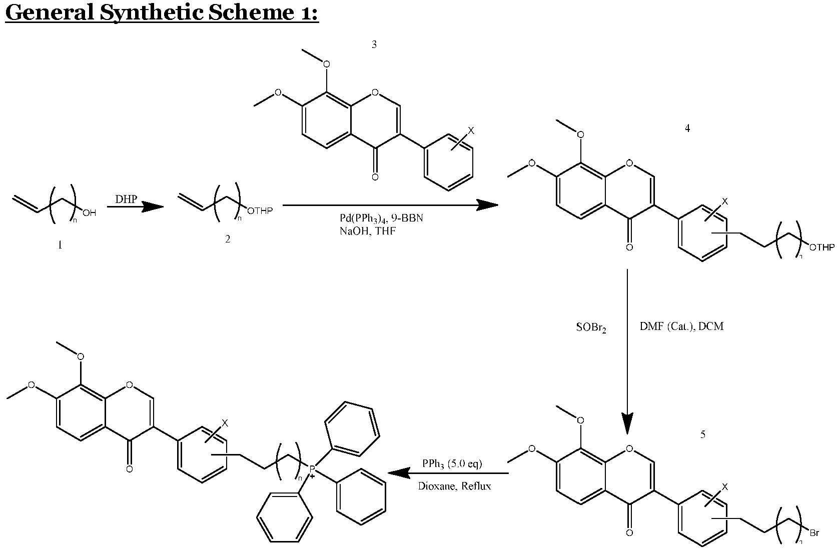

### Preparation of compound 2

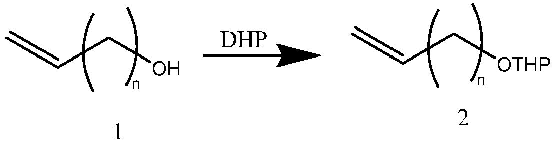

To a solution of Compound 1 (1.00 eq) and p-TSOH (0.05 eq) in dry DCM (5 mL/ 1 g Compound 1) was added DHP (1.20 eq) at 25°C over 30 min, the resulting mixture was stirred at 25 °C for 2 hours. TLC (SiO2. PE / EA = 10 /1, Rf =0.5) showed Compound 1 was consumed and a new spot was detected. The reaction mixture was concentrated under reduced pressure to give a residue. The residue was purified by column chromatography (SiO2, PE / EA = 50 /1 to 10 /1). Compound 2 was obtained as a white oil.

### Preparation of compound 4

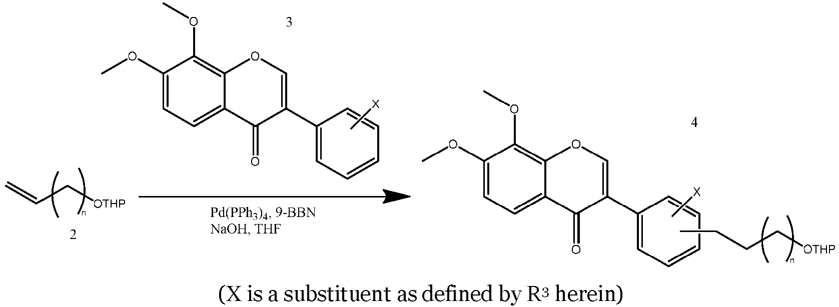

Compound 2 (1.50 eq) was added under nitrogen flow to a solution of 9-BBN (0.5 M, 51.45 mL, 1.50 eq) in THF (10 mL/1 g Compound 2). The mixture was stirred at 25 °C for 24 hrs under nitrogen, then K3PO4 (3 M, 2.50 eq) and a suspension of Compound 3 (1.00 eq) in DMF (5 mL/1 g Compound 2) were added. The reaction mixture was then degassed with N2, followed by the addition of Pd(dppf)Cl2 (0.10 eq). The reaction mixture was degassed again and the mixture was heated at 60 °C for 2.5 h. TLC (SiO2, PE / EA = 5/1,Rf = 0.3) showed Compound 3 was consumed and new spots was detected. The reaction mixture was filtered and extracted with ethyl acetate (40 ml × 3) and H2O (100 ml × 3), the organic phase was dried over Na2SO4, filtered and concentrated under reduced pressure to give a residue. The residue was purified by column chromatography (SiO2, PE / EA = 50 /1 to 87/13). Compound 4 was obtained as a yellow oil.

### Preparation of Compound 5

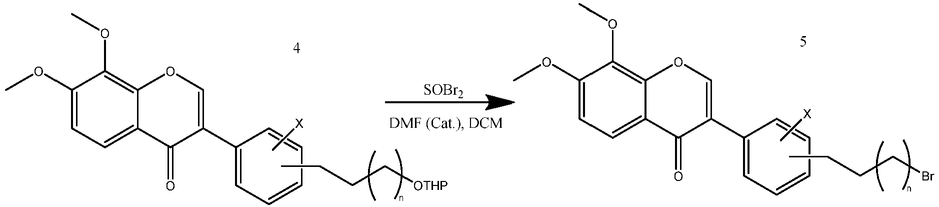

Thionyl bromide (5.00 eq) was added dropwise under nitrogen flow to a solution of Compound 4 (1.00 eq) in dry DCM (20 mL/1 g Compound 4) and DMF (10.00 eq) at 0 °C. The reaction mixture was stirred for 10 min, before the solution was allowed to warm to 25 °C and stirred under nitrogen until it was complete by LC-MS. TLC (petroleum ether: ethyl acetate = 5:1, twice) showed the starting material was consumed and a new spot was detected. The combined reaction mixture was poured onto ice, the mixture was basified with the saturated NaHCO3 to pH = 8. The mixture was separated between DCM and water, the combined organic layers was washed with brine and dried over Na2SO4. After filtration and concentration, the residue was purified by silica gel chromatography (petroleum ether : ethyl acetate, from 100:0 to 83:17). Compound 5 was obtained as a yellow solid.

### Preparation of final compound, e.g. SND540-SND558

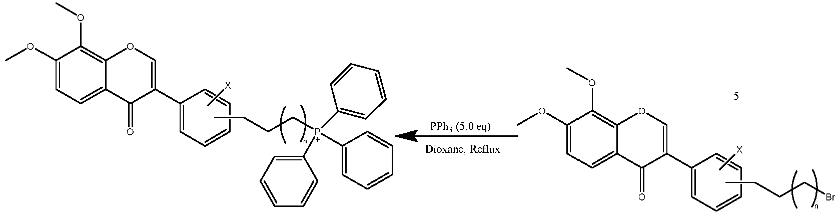

To a solution of Compound 5 (1.00 eq) in dry dioxane was added PPh3 (5.00 eq) and NaI (0.1eq), and the mixture was stirred at 105 °C for 16 h under N2. TLC (SiO2, DCM/ MeOH = 10/1, Rf = 0.4) showed Compound 5 was consumed and a new spot was detected. The reaction mixture was concentrated under reduced pressure to give a residue. The residue was purified by column chromatography (SiO2, PE / EA = 1/0 to 0/1 to DCM /MeOH = 10/1). The residue was purified by prep-HPLC (HCl). The product was obtained as a yellow solid.

### Synthesis of SND477

Compound SND477 was synthesised using the standard procedures of general synthetic scheme 1 above, in which compound SND477 has the following details.

| | | |
|---|---|---|
| SND477 | X = -H | n = 4 |

1H NMR (400 MHz, DMSO-d6) δ = 8.48 (s, 1H), 7.92 - 7.85 (m, 4H), 7.84 - 7.74 (m, 12H), 7.48-7.46 (d, J = 8.0 Hz, 2H), 7.32-7.20 (d, J = 9.2 Hz, 1H), 7.22-7.20 (d, J = 8.4 Hz, 2H), 3.97 (s, RH), 3.88 (s, 3H), 3.66 - 3.48 (m, 2H), 2.57-2.55 ( d, J = 7.2 Hz, 2H), 1.59 - 1.46 (m, 6H), 1.34-1.32 ( d, J = 5.6 Hz, 2H).

LCMS: MS (ESI) Retention time: 2.277 min, (M+1) + = 627.2

### Synthesis of SND479

Compound SND479 was synthesised using the standard procedures of general synthetic scheme 1 above, in which compound SND479 has the following details.

| | | |
|---|---|---|
| SND479 | X = -H | n = 8 |

1H NMR (400 MHz, DMSO-d6) δ = 8.48 (s, 1H), 7.92 - 7.84 (m, 4H), 7.83 - 7.73 (m, 12H), 7.49-7.47 (d, J = 8.0 Hz, 2H), 7.32-7.20 (d, J = 9.2 Hz, 1H), 7.25-7.23 (d, J = 8.0 Hz, 2H), 3.97 (s, 3H), 3.88 (s, RH), 3.61 - 3.50 (m, 2H), 2.60-2.58 ( t, J = 7.4 Hz, 2H), 1.60 - 1.39 (m, 6H), 1.31 - 1.16 (m, 10H).

LCMS: MS (ESI) Retention time: 2.458 min, (M+1) + = 683.2

### Synthesis of SND540

Compound SND540 was synthesised using the standard procedures of general synthetic scheme 1 above, in which compound SND540 has the following details.

| | | |
|---|---|---|
| SND540 | X = -H | n = 2 |

**1H NMR** (400 MHz, DMSO-d6) δ = 8.47 (s, 1H), 7.92 - 7.86 (m, 4H), 7.83 - 7.74 (m, 13H), 7.48-7.46 (d, J = 8.0 Hz, 2H), 7.33-7.31 (d, J = 9.2 Hz, 1H), 7.20-7.22 (d, J = 8.4 Hz, 2H), 3.97 (s, 3H), 3.89 (s, 3H), 3.68 - 3.59 (m, 2H), 2.67-2.65 ( J = 7.2 Hz, 2H), 1.80-1.78 ( J = 7.2 Hz, 2H), 1.65 - 1.52 (m, 2H).

**LCMS:** MS (ESI) Retention time: 2.062 min, (M+1) + = 599.2.

### Synthesis of SND541

Compound SND541 was synthesised using the standard procedures of general synthetic scheme 1 above, in which compound SND541 has the following details.

| | | |
|---|---|---|
| SND541 | X = -H | n = 1 |

**¹H NMR** (400 MHz, DMSO-d6) δ = 8.49 (s, 1H), 7.92 - 7.84 (m, 4H), 7.83 - 7.73 (m, 12H), 7.43 - 7.30 (m, 4H), 7.20-7.18 (d, J = 7.2 Hz, 1H), 3.97 (s, 3H), 3.89 (s, 3H), 3.65 (br s, 2H), 2.84-2.82 ( t, J = 7.2 Hz, 2H), 1.88-1.86 (br d, J = 8.0 Hz, 2H).

**LCMS: MS (ESI)** Retention time: 1.993 min, (M+1)⁺ =585.2.

### Synthesis of SND544

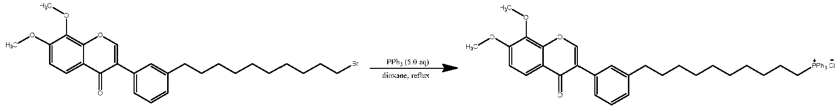

To a solution of 3-(3-(10-bromodecyl)phenyl)-7,8-dimethoxy-4H-chromen-4-one (1.4 g, 2.79 mmol, 1 eq) in dioxane (10 mL) was added PPh₃ (3.66 g, 13.96 mmol, 5 eq) and NaI (41.85 mg, 279.19 µmol, 0.1 eq), and the mixture was stirred at 105 °C for 16h. A main new spot was detected on TLC (SiO₂, DCM/ MeOH = 10/1, Rf =0.4). The desired mass was detected as the main peak on LCMS (EW30065-893-P1A). The reaction mixture was concentrated under reduced pressure to remove solvent. The residue was purified by column chromatography (SiO2, PE / EA = 1/0 to 0/1 to DCM / MeOH = 10/1). The residue was purified by prep-HPLC (HCl). The product (689.14 mg, 956.75 µmol, 34.27% yield, HCl) was obtained as a yellow solid.

**¹H NMR** (400 MHz, DMSO-d6) δ = 8.49 (s, 1H), 7.94 - 7.84 (m, 4H), 7.83 - 7.71 (m, 12H), 7.51 - 7.27 (m, 4H), 7.20-7.18 (d, J = 7.2 Hz, 1H), 3.97 (s, 3H), 3.88 (s, 3H), 3.58 - 3.46 (m, 2H), 2.60-2.58 (t, J = 7.6 Hz, 2H), 1.65 - 1.36 (m, 6H), 1.33 - 1.03 (m, 10H).

**LCMS: MS (ESI)** Retention time: 0.676 min, (M+1)⁺ = 683.5.

### Synthesis of SND544B

Compound SND544B was synthesised using the standard procedures of general synthetic scheme 1 above, in which compound SND544B has the following details.

| | | |
|---|---|---|
| SND544B | X = -H | n = 8 |

**¹H NMR** (400 MHz, DMSO-d6) δ = 8.49 (s, 1H), 8.19 - 8.10 (m, 13H), 7.86-784 (d, J = 9.2 Hz, 1H), 7.40 - 7.29 (m, 4H), 7.20-7.18 (br d,J = 6.8 Hz, 1H), 3.97 (s, 3H), 3.88 (s, 3H), 3.86 - 3.76 (m, 2H), 2.60-2.58 (br t, J = 7.6Hz, 2H), 1.60 - 1.50 (m, 4H), 1.47 - 1.40 (m,2H), 1.29 - 1.18 (m, 10H).

### Synthesis of SND550

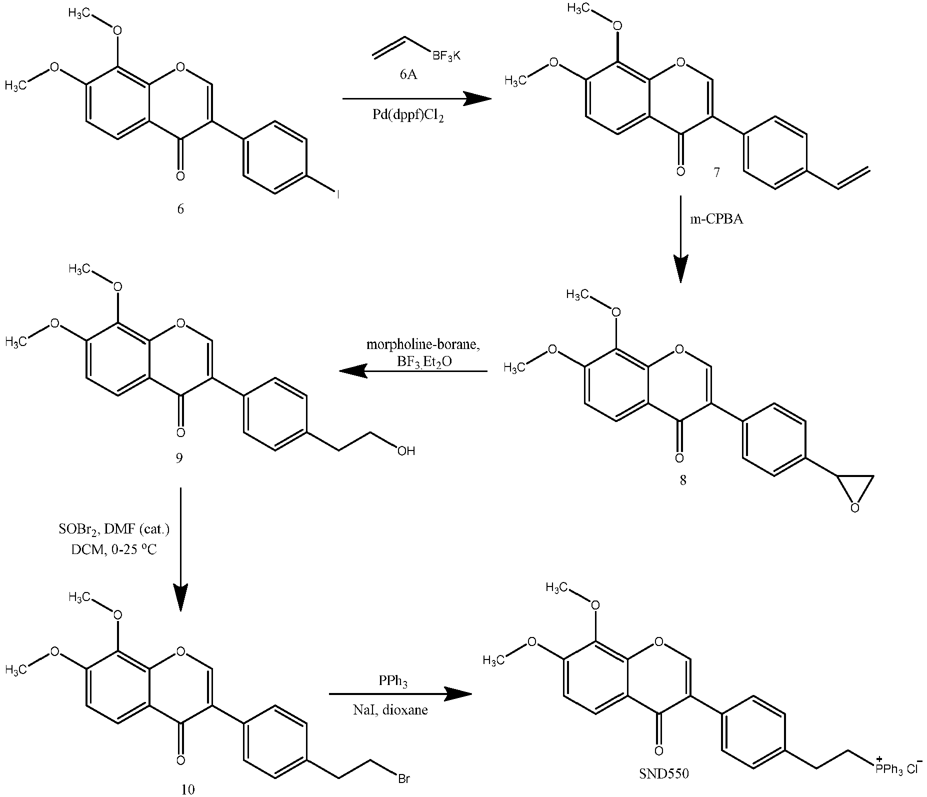

### Preparation of Compound 7

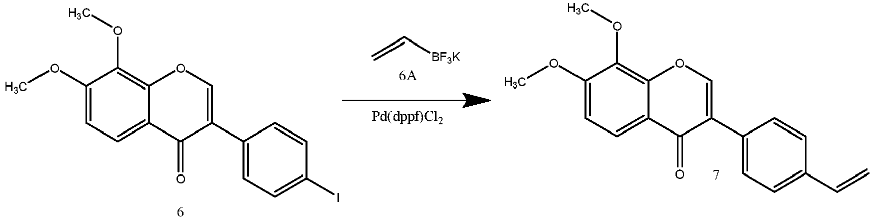

A stirred solution of Compound 6 (6 g, 14.70 mmol, 1.00 eq), Compound 6A (5.91 g, 44.10 mmol, 3.00 eq) and K2CO3 (6.09 g, 44.10 mmol, 3.00 eq) in DMSO (50 mL) was degassed with N2 for 0.5 h at 25 °C, and then Pd(dppf)Cl2 (1.08 g, 1.47 mmol, 0.10 eq) was added at 25 °C , and the resulting mixture was stirred at 100 °C for 16 h. LCMS (EW30065-746-P1A) showed Compound 6 was consumed and desired mass was detected. The reaction mixture was concentrated under reduced pressure to give a residue. The residue was purified by column chromatography (SiO2, PE/EA = 0/1 to 5/1). Compound 7 (2 g, 6.29 mmol, 42.81% yield, 97% purity) was obtained as a white solid.

**¹H NMR** (400 MHz, CHLOROFORM-d) δ = 8.16 - 8.00 (m, 2H), 7.59 - 7.52 (m, 2H), 7.52 -7.45 (m, 2H), 7.09-7.07 (d, J = 9.2 Hz, 1H), 6.77-6.75 (dd, J = 10.8, 17.5 Hz, 1H), 5.81-5.79 (d, J = 17.6 Hz, 1H), 5.30-5.28 (d, J = 10.8 Hz, 1H), 4.02 (s, 6H).

**LCMS: MS (ESI)** Retention time: 0.662 min, (M+1)⁺ = 309.0, EW30065-746-P1B.

### Preparation of Compound 8

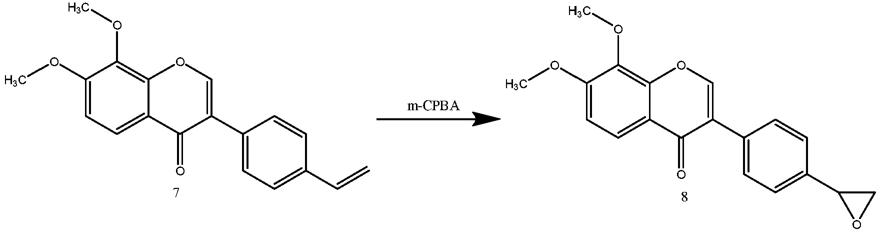

To a solution of Compound 7 (1.5 g, 4.86 mmol, 1.00 eq) in DCM (15 mL) was added m-CPBA (1.57 g, 7.30 mmol, 80% purity, 1.50 eq) at 0 °C , and the mixture was stirred at 0 °C for 2 h. TLC (SiO₂, PE / EA = 1/1, Rf = 0.5) showed Compound 7 was consumed and a new spot was detected. LCMS (EW30065-764-P1A) showed Compound 7 was consumed and desired mass was detected. The reaction mixture was quenched with the saturated Na₂SO₃ (30 ml), and extracted with DCM (10 ml × 3) and H₂O (10 ml × 3), the organic phase was dried over Na₂SO₄, filtered and concentrated under reduced pressure to give a residue. The residue was purified by column chromatography (SiO₂, PE/ EA = 0/1 to 2/1). Compound 8 (900 mg, 2.77 mmol, 57.04% yield) was obtained as a white solid, which was confirmed by HNMR.

**¹H NMR** (400 MHz, CHLOROFORM-d) δ = 8.08 - 8.02 (m, 2H), 7.57-7.55 (d, J = 8.0 Hz, 2H), 7.38-7.36 (d, J = 8.0 Hz, 2H), 7.09-7.07 (d, J = 9.2 Hz, 1H), 4.02 (s, 6H), 3.93-3.91 (t, J = 3.2 Hz, 1H), 3.19-3.17 (t, J = 4.8 Hz, 1H), 2.84-2.82 (dd, J = 2.4, 5.4 Hz, 1H). **LCMS: MS (ESI)** Retention time: 0.612 min, (M+1)⁺ = 325.0, EW30065-764-P1B.

### Preparation of Compound 9

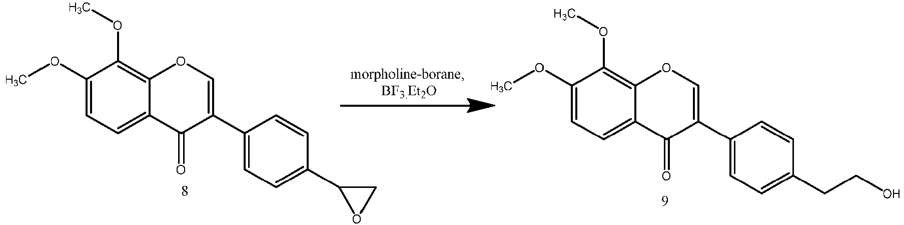

A solution of Compound 8 (900 mg, 2.77 mmol, 1.00 eq) and borane;morpholine (280.15 mg, 2.77 mmol, 1.00 eq) in THF (9 mL) was added BF3•Et2O (393.85 mg, 2.77 mmol, 342.48 µL, 1.00 eq) dropwised at 25 °C, and then the mixture was stirred at 25 °C for 2 h. LCMS showed Compound 8 was consumed and desired mass was detected. TLC (SiO₂, PE / EA = 1/1, Rf =0.3) showed Compound 8 was consumed and a new spot was detected. The reaction mixture was concentrated under reduced pressure to give a residue. The residue was purified by prep-TLC (SiO₂, PE / EA = 1/1). Compound 9 (780 mg, 2.39 mmol, 86.13% yield) was obtained as a white solid, which was confirmed by HNMR.

**¹H NMR** (400 MHz, CHLOROFORM-d) δ = 8.06 (d, J = 9.0 Hz, 1H), 8.02 (s, 1H), 7.55 -7.49 (m, 2H), 7.32 (d, J = 8.3 Hz, 2H), 7.08 (d, J = 9.0 Hz, 1H), 4.02 (d, J = 1.1 Hz, 6H), 3.94- 3.87 (m, 2H), 2.92 (t, J = 6.5 Hz, 2H).

**LCMS: MS (ESI)** Retention time: 0.574 min, (M+1)⁺ = 327.0, EW30065-767-P1A.

### Preparation of Compound 10

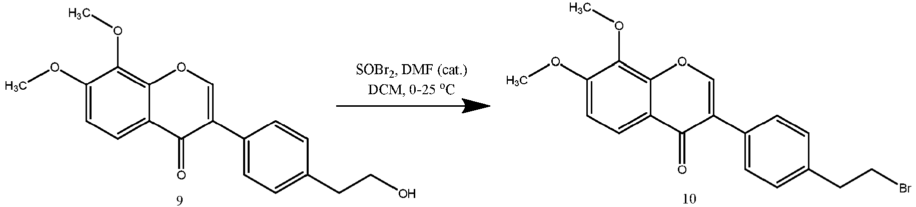

Thionyl bromide (2.38 g, 11.47 mmol, 888.87 µL, 4.80 eq) was added dropwise under nitrogen flow to a solution of Compound 9 (780 mg, 2.39 mmol, 1.00 eq) in dry DCM (9 mL) and DMF (1.75 g, 23.90 mmol, 1.84 mL, 10.0 eq) at 0 °C. The reaction mixture was stirred for 1 min, before the solution was allowed to warm to 25 °C and stirred under nitrogen until it was complete by LC-MS (1.5 h). TLC (SiO₂, PE / EA = 5/1, Rf = 0.4) showed compound 9 was consumed and a new spot was detected. LCMS (EW30065-779-P1B) showed Compound 9 was consumed and desired mass was detected. The reaction mixture was poured into ice water (10 ml), and added aq. NaHCO₃ to pH = 8, and extracted with DCM (10 ml × 3), the organic phase was dried over Na₂SO₄, filtered and concentrated under reduced pressure to give a residue. The residue was purified by column chromatography (SiO2, PE / EA = 1/0 to 3/1). Compound 5 (700 mg, 1.69 mmol, 70.73% yield, 94% purity) was obtained as a white solid, which was confirmed by HNMR.

**¹H NMR** (400 MHz, CHLOROFORM-d) δ = 8.08 - 8.02 (m, 2H), 7.57 - 7.50 (m, 2H), 7.31-7.29 (d, J = 8.0 Hz, 2H), 7.09-7.07 (d, J = 9.2 Hz, 1H), 4.03-4.01 (d, J = 1.2 Hz, 6H), 3.61-3.5J = 7.7 Hz, 2H), 3.23-3.21 (t, J = 7.6 Hz, 2H).

**LCMS: MS (ESI)** Retention time: 0.670 min, (M+1)⁺ = 390.9, EW30065-779-P1B.

### Preparation of Compound SND550

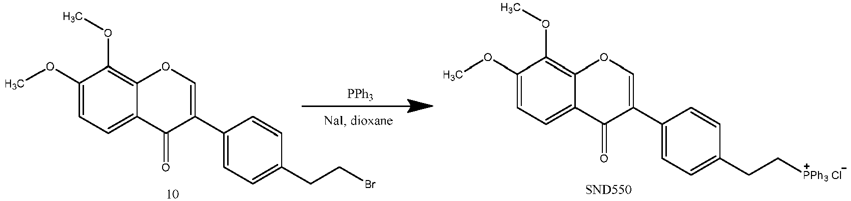

To a solution of Compound 10 (700 mg, 1.80 mmol, 1.00 eq) in dioxane (7 mL) was added PPh₃ (2.36 g, 8.99 mmol, 5.00 eq) and NaI (26.96 mg, 179.84 µmol, 0.10 eq) , and the mixture was stirred at 105 °C for 12 h under N2. LCMS (EW30065-784-P1A) showed Compound 10 was consumed and desired mass was detected. The reaction mixture was concentrated under reduced pressure to remove solvent. The residue was purified by prep-HPLC (HCl). SND550 (188.77 mg, 300.69 µmol, 16.72% yield, 96.86% purity, HCl) was obtained as a yellow solid.

**¹H NMR** (400 MHz, DMSO-d6) δ = 8.50 (s, 1H), 7.96 - 7.85 (m, 10H), 7.84 - 7.77 (m, 6H), 7.55-7.53 (d, J = 8.0 Hz, 2H), 7.40-7.38 (d, J = 8.0 Hz, 2H), 7.33-7.31 (d, J = 9.2 Hz, 1H), 4.04 - 3.94 (m, 5H), 3.89 (s, 3H), 2.99 - 2.89 (m, 2H).

**LCMS: MS (ESI)** Retention time: 1.957 min, (M+1)⁺ = 571.2, EW30065-784-P1F1.

### Synthesis of SND551

Compound SND551 was synthesised using the standard procedures of general synthetic scheme 1 above, in which compound SND551 has the following details.

| | | |
|---|---|---|
| SND551 | X = -F | n = 2 |

**¹H NMR** (400 MHz, DMSO-d6) δ = 8.56 (s, 1H), 7.93 - 7.86 (m, 4H), 7.84 - 7.74 (m, 12H), 7.40-7.38 (d, J = 12.0 Hz, 1H), 7.37 - 7.27 (m, 3H), 3.97 (s, 3H), 3.89 (s, 3H), 3.67-3.65 ( t, J = 14.8 Hz, 2H), 2.73 - 2.63 (m, 2H), 1.84 - 1.72 (m, 2H), 1.61-1.59 (br d, J = 7.6 Hz, 2H).

**LCMS: MS (ESI)** Retention time: 2.077 min, (M+1) + =617.2, EW30065-780-P1F.

### Synthesis of SND552

Compound SND552 was synthesised using the standard procedures of general synthetic scheme 1 above, in which compound SND552 has the following details.

| | | |
|---|---|---|
| SND552 | X= -F | n = 2 |

**¹H NMR** (400 MHz, DMSO-d6) δ = 8.43 (s, 1H), 7.93 - 7.87 (m, 3H), 7.86 - 7.75 (m, 13H), 7.35 - 7.29 (m, 2H), 7.13-7.11 (d, J = 11.4 Hz, 1H), 7.08-7.06 (d, J = 8.0 Hz, 1H), 3.97 (s, 3H), 3.89 (s, 3H), 3.71 - 3.61 (m, 2H), 2.70-2.68 (t, J = 7.2 Hz, 2H), 1.87 - 1.75 (m, 2H), 1.67 - 1.52 (m, 2H).

**LCMS: MS (ESI)** Retention time: 2.017min, (M+1)⁺ =617.1, EW30065-818-P1F.

### Synthesis of SND553

Compound SND553 was synthesised using the standard procedures of general synthetic scheme 1 above, in which compound SND553 has the following details.

| | | |
|---|---|---|
| SND553 | X = -Cl | n = 2 |

**¹H NMR** (400 MHz, DMSO-d6) δ = 8.37 (s, 1H), 7.94 - 7.88 (m, 3H), 7.85 - 7.75 (m, 13H), 7.41-7.39 (d, J = 1.2 Hz, 1H), 7.35 - 7.26 (m, 2H), 7.21-7.19 ( J = 1.2, 7.9 Hz, 1H), 3.98 (s, 3H), 3.90 (s, 3H), 3.72 - 3.60 (m, 2H), 2.73 - 2.64 (m, 2H), 1.85 - 1.73 (m, 2H), 1.66 - 1.54 (m, 2H).

**LCMS: MS (ESI)** Retention time: 2.080 min, (M+1)⁺ =633.1.

### Synthesis of SND554

Compound SND554 was synthesised using the standard procedures of general synthetic scheme 1 above, in which compound SND554 has the following details.

| | | |
|---|---|---|
| SND554 | X = -Cl | n = 2 |

**¹H NMR** (400 MHz, DMSO-d6) δ = 8.57 (s, 1H), 7.93 - 7.86 (m, 4H), 7.84 - 7.76 (m, 12H), 7.66-7.64 (d, J = 1.6 Hz, 1H), 7.47-7.45 (J = 1.6, 7.9 Hz, 1H), 7.35-7.33 (t, J = 9.2 Hz, 2H), 3.97 (s, 3H), 3.89 (s, 3H), 3.72 - 3.62 (m, 2H), 2.77-2.75 ( t, J = 7.2 Hz, 2H), 1.84 -1.73 (m, 2H), 1.64-1.62 ( d, J = 7.2 Hz, 2H).

**LCMS: MS (ESI)** Retention time: 2.127 min, (M+1)⁺ =633.1.

### Synthesis of SND555

Compound SND555 was synthesised using the standard procedures of general synthetic scheme 1 above, in which compound SND555 has the following details.

| | | |
|---|---|---|
| SND555 | X = -CH₃ | n = 2 |

**¹H NMR** (400 MHz, DMSO-d6) δ = 8.30 - 8.24 (m, 1H), 7.93 - 7.88 (m, 3H), 7.86 - 7.75 (m, 13H), 7.32-7.30 (d, J = 9.2 Hz, 1H), 7.08-7.06 (d, J = 7.2 Hz, 2H), 7.03 - 6.99 (m, 1H), 3.97 (s, 3H), 3.89 (s, 3H), 3.65-3.63 ( dd, J = 7.6, 13.4 Hz, 2H), 2.64-2.62 ( t, J = 7.2 Hz, 2H), 2.10 (s, 3H), 1.84 - 1.73 (m, 2H), 1.68 - 1.53 (m, 2H).

**LCMS: MS** (ESI) Retention time: 2.065 min, (M+1)⁺ =613.2.

### Synthesis of SND556

Compound SND556 was synthesised using the standard procedures of general synthetic scheme 1 above, in which compound SND556 has the following details.

| | | |
|---|---|---|
| SND556 | X = -CH₃ | n = 2 |

**¹H NMR** (400 MHz, DMSO-d6) δ = 8.44 (s, 1H), 7.93 - 7.87 (m, 3H), 7.85 - 7.75 (m, 13H), 7.35 - 7.25 (m, 3H), 7.14-7.12 (d, J = 8.0 Hz, 1H), 3.97 (s, 3H), 3.88 (s, 3H), 3.66 (br s, 2H), 2.69 - 2.59 (m, 2H), 2.26 (s, 3H), 1.79 - 1.69 (m, 2H), 1.68 - 1.57 (m, 2H).

**LCMS: MS** (ESI) Retention time: 2.077min, (M+1)⁺ =613.2.

### Synthesis of SND557

Compound SND557 was synthesised using the standard procedures of general synthetic scheme 1 above, in which compound SND557 has the following details.

| | | |
|---|---|---|
| SND557 | X = -OCH₃ | n = 2 |

**¹H NMR** (400 MHz, DMSO-d6) δ = 8.24 (s, 1H), 7.93 - 7.88 (m, 3H), 7.84 - 7.76 (m, 13H), 7.30-7.28 (d, J = 9.2 Hz, 1H), 7.11-7.09 (d, J = 7.6 Hz, 1H), 6.89 (s, 1H), 6.79-6.77 (d, J = 7.6 Hz, 1H), 3.96 (s, 3H), 3.88 (s, 3H), 3.67 (br s, 5H), 2.67-2.65 (t, J = 7.2 Hz, 2H), 1.89 - 1.75 (m, 2H), 1.70 - 1.54 (m, 2H).

**LCMS: MS** (ESI) Retention time: 2.027min, (M+1)⁺ =629.2.

### Synthesis of SND558

Compound SND558 was synthesised using the standard procedures of general synthetic scheme 1 above, in which compound SND558 has the following details.

| | | |
|---|---|---|
| SND558 | X = -OCH₃ | n = 2 |

**¹H NMR** (400 MHz, DMSO-d6) δ = 8.52 (s, 1H), 7.92 - 7.86 (m, 4H), 7.83 - 7.74 (m, 12H), 7.33-7.31 (d, J = 9.2 Hz, 1H), 7.14-7.12 (d, J = 8.4 Hz, 2H), 7.09 - 7.03 (m, 1H), 3.97 (s, 3H), 3.89 (s, 3H), 3.75 (s, 3H), 3.65-3.63 (t, J = 14.4 Hz, 2H), 2.62-2.60 ( t, J = 7.2 Hz, 2H), 1.81 - 1.68 (m, 2H), 1.64 - 1.51 (m, 2H).

**LCMS:** MS (ESI) Retention time: 2.072 min, (M+1)⁺ =627.9.

### EXAMPLES - BIOLOGICAL STUDIES

### Experimental methodology

### Antitumor activity against a panel of cancer cell lines

Antitumor activity of the compounds was assessed by using the CellTiter-Glow^{®} Luminescent Cell Viability assay (Promega # G7572) according to the manufacturer's instructions. The compounds were tested at 5 or 6 concentrations in 1:2 dilution increments (highest concentration 10 µM) in triplicate well conditions.

Tumor cells were grown at 37°C in a humidified atmosphere with 5% CO₂ in RPMI 1640 or DMEM medium, supplemented with 10% (v/v) fetal calf serum and 50 µg/ml gentamicin for up to 20 passages, and were passaged once or twice weekly. Cells were harvested using TrypLE or PBS buffer containing 1 mM EDTA, and the percentage of viable cells is determined using a cell counter.

Cells were harvested from exponential phase cultures, counted and plated in 96 well flat-bottom microtiter plates at a cell density depending on the cell line's growth rate (4,000 - 20,000 cells/well depending on the cell line's growth rate) in RPMI 1640 or DMEM medium supplemented with 10% (v/v) fetal calf serum and 50 µg/ml gentamicin (140 µl/well). Cultures were incubated at 37°C and 5% CO₂ in a humidified atmosphere. After 24 h, 10 µl of test compounds or control medium are added, and left on the cells for another 72 h. Compounds were serially diluted in DMSO, transferred in cell culture medium, and added to the assay plates. The DMSO concentration was kept constant at < 0.2% v/v across the assay plate. Viability of cells was quantified by the CellTiter-Glow^{®} Luminescent Cell Viability assay (Promega # G7572). Luminescence was measured with the microplate luminometer (EnVision Perkin Elmer).

Sigmoidal concentration-response curves were fitted to the data points (test-versus-control, T/C values) obtained for each tumor model using GraphPad prism 5.02 software. IC₅₀ values are reported as absolute IC₅₀ values, being the concentration of test compound at the intersection of the concentration-response curves with T/C = 50%.

### Apoptosis activity against a panel of tumour cell lines

Apoptosis activity was evaluated using Caspase-Glo 3/7 Assay kit (Promega-G8091) according to the manufacturer's instructions.Cells were grown as above and harvested from exponential phase cultures, counted and plated in 96 black well flat-bottom microtiter plates at a cell density depending on the cell line's growth rate (3,000 - 10,000 cells/well depending on the cell line's growth rate). Following an overnight incubation, compounds were added to the cell plates at various concentrations (highest at 10 mM) with the DMSO concentration maintained at 0.1% for a 24 h period treatment. 100 µL of Caspase-Glo 3/7 Reagent was added to each well of 96-well plate containing 100µL culture medium, and after 30 min at room temperature, luminescence was measured with the microplate luminometer (EnVision Perkin Elmer). The induced relative Caspase 3/7 activity of the tested compounds was determined by the following formula: Relative Caspase 3/7 activity (fold increase) = (RLU compound - RLU blank) / (RLU control - RLU blank).

### Signal transduction pathway inhibition

Inhibition of various cellular pathways known to be involved in malignancy was tested using the SelectScreen Cell-based Pathway Profiling GeneBLAzer^{™} cell signaling pathway specific CellSensor^{™} cell lines ((Thermo Fisher). The assay is based on a beta-lactamase reporter activity which is modulated and can be measured quantitatively and selectively with the LiveBLAzer^{™}-FRET B/G Loading Substrate. SelectScreen Cell-Based Pathway Profiling Service uses XLfit from IDBS. The dose response curve is curve fit to model number 205 (sigmoidal dose-response model). Custom logic was built by Thermo Fisher for the data analysis tool to address the different compound characteristics that can be observed with functional assays.

### Specific conditions for the pathways tested:

### JAK/STAT - ISRE-bla HEK 293T - Inhibitor Screen, Activated by IFN-alpha

ISRE-bla HEK 293T cells are thawed and resuspended in Assay Media (OPTI-MEM, 0.5% dialyzed FBS, 0.1 mM NEAA, 1 mM Sodium Pyruvate, 100 U/mL/100 µg/mL Pen/Strep) to a concentration of 625,000 cells/mL. 32 µL of cell suspension is added to each well of a 384-well TC-Treated assay plate. Cells in Assay Media are incubated for 16-24 hours in the plate at 37°C/5% CO2 in a humidified incubator. 4 µL of a 10X serial dilution of JAK Inhibitor I (control inhibitor starting concentration, 1,000 nM) or compounds are added to appropriate wells of the plate and pre-incubated at 37°C/5% CO2 in a humidified incubator with cells for 30 minutes. 4 µL of 10X control activator IFN-alpha at the pre-determined EC80 concentration is added to wells containing the control inhibitor or compounds. The plate is incubated for 5 hours at 37°C/5% CO2 in a humidified incubator. 8 µL of 1 µM Substrate Loading Solution is added to each well and the plate is incubated for 2 hours at room temperature. The plate is read on a fluorescence plate reader.

### MAPK/MEK/B-raf - AP1-bla A375 - Inhibitor Screen, Constitutively Activated

AP1-bla A375 cells are thawed and resuspended in Assay Media (OPTI-MEM, 0.5% dialyzed FBS, 0.1 mM NEAA, 1 mM Sodium Pyruvate, 100U/mL/100 µg/mL Pen/Strep) to a concentration of 312,500 cells/mL. 4 µL of a 10X serial dilution of Raf1 Kinase Inhibitor (control inhibitor starting concentration, 10,000 nM) or compounds are added to appropriate wells of a TC-Treated assay plate. 32 µL of cell suspension (10,000 cells) is added to the wells. 4 µL of Assay Media is added to all wells to bring the final assay volume to 40 µL. The plate is incubated for 16-24 hours at 37°C/5% CO2 in a humidified incubator. 8 µL of 1 µM Substrate Loading Solution is added to each well and the plate is incubated for 2 hours at room temperature. The plate is read on a fluorescence plate reader.

### NFKB - NFKB-bla Jurkat - Inhibitor Screen, Activated by TNF-alpha

NFKB-bla Jurkat cells are thawed and resuspended in Assay Media (DMEM, 10% dialyzed FBS, 25 mM HEPES pH 7.3, 0.1 mM NEAA, 100U/mL/100 µg/mL Pen/Strep) to a concentration of 625,000 cells/mL.. 4 µL of a 10X serial dilution of Withaferin A (control inhibitor starting concentration, 1,000 nM) or compounds are added to appropriate wells of a TC-Treated assay plate. 32 µL of cell suspension is added to the wells and pre-incubated at 37°C/5% CO2 in a humidified incubator with compounds and control inhibitor titration for 30 minutes. 4 µL of 10X control activator TNF-alpha at the pre-determined EC80 concentration is added to wells containing the control inhibitor or compounds.

The plate is incubated for 5 hours at 37°C/5% CO2 in a humidified incubator. 8 µL of 1 µM Substrate Loading Solution is added to each well and the plate is incubated for 2 hours at room temperature. The plate is read on a fluorescence plate reader.

### Oxidative Stress - ARE-bla HepG2 - Inhibitor Screen, Activated by tBHO

ARE-bla HepG2 cells are thawed and resuspended in Assay Media (DMEM, 10% dialyzed FBS, 25 mM HEPES pH 7.3, 0.1 mM NEAA, 100U/mL/100 µg/mL Pen/Strep) to a concentration of 375,000 cells/mL. 32 µL of cell suspension is added to each well of a 384-well Poly-D-Lysine assay plate. Cells in Assay Media are incubated for 16-24 hours in the plate at 37°C/5% CO2 in a humidified incubator. 4 µL of a 10X serial dilution of Ro-31-8220 (control inhibitor starting concentration, 10,000 nM) or compounds are added to appropriate wells of the plate and pre-incubated at 37°C/5% CO2 in a humidified incubator with cells for 30 minutes. 4 µL of 10X control activator tBHQ at the predetermined EC80 concentration is added to wells containing the control inhibitor or compounds. The plate is incubated for 5 hours at 37°C/5% CO2 in a humidified incubator. 8 µL of 1 µM Substrate + Solution D Loading Solution is added to each well and the plate is incubated for 2 hours at room temperature. The plate is read on a fluorescence plate reader.

### Toll-like Receptor (TLR4) - NFkB-bla THP-1 - Inhibitor Screen, Activated by LPS

NFkB-bla THP-1 cells are thawed and resuspended in Assay Media (RPMI, 0.5% dialyzed FBS, 0.1 mM NEAA, 1 mM Sodium Pyruvate, 100 U/mL/100 µg/mL Pen/Strep) to a concentration of 625,000 cells/mL. 32 µL of cell suspension is added to each well of a 384-well TC-Treated assay plate. Cells in Assay Media are incubated for 16-24 hours in the plate at 37°C/5% CO2 in a humidified incubator. 4 µL of a 10X serial dilution of Withaferin A (control inhibitor starting concentration, 10,000 nM) or compounds are added to appropriate wells of the plate and pre-incubated at 37°C/5% CO2 in a humidified incubator with cells for 30 minutes. 4 µL of 10X control activator LPS at the predetermined EC80 concentration is added to wells containing the control inhibitor or compounds. The plate is incubated for 5 hours at 37°C/5% CO2 in a humidified incubator. 8 µL of 1 µM Substrate Loading Solution is added to each well and the plate is incubated for 2 hours at room temperature. The plate is read on a fluorescence plate reader.

### Wnt/Beta-Catenin - LEF-TCF-bla HCT116 - Inhibitor Screen, Constitutively Activated

LEF-TCF-bla HCT116 cells are thawed and resuspended in Assay Media (OPTI-MEM, 0.5% dialyzed FBS, 0.1 mM NEAA, 1 mM Sodium Pyruvate, 100 U/mL/100 µg/mL Pen/Strep) to a concentration of 312,500 cells/mL.32 µL of cell suspension is added to each well of a 384-well Poly-D-Lysine assay plate. Cells in Assay Media are incubated for 16-24 hours in the plate at 37°C/5% CO2 in a humidified incubator. 4 µL of a 10X serial dilution of ICG-001 (control inhibitor starting concentration, 25,000 nM) or compounds are added to appropriate wells of the plate. 4 µL of Assay Media is added to all wells to bring the final assay volume to 40 µL. The plate is incubated for 5 hours at 37°C/5% CO2 in a humidified incubator. 8 µL of 1 µM Substrate Loading Solution is added to each well and the plate is incubated for 2 hours at room temperature. The plate is read on a fluorescence plate reader.

### In vivo tumour inhibition

The A2058 tumor cells were maintained *in vitro* in DMEM medium supplemented with 10% fetal bovine serum and 1% Anti-Anti at 37°C in an atmosphere of 5% CO₂ in air. The tumor cells were routinely subcultured twice weekly.

Each Balb-c/nude mouse was inoculated subcutaneously at the right flank with A2058 melanoma tumor cells (5 x 10⁶ + Matrigel) in 0.2 ml of PBS for tumor development. 10 animals per group were randomized when the average tumor volume reached 74 mm³. Test compounds were prepared as DMSO stocks and diluted in the injection vehicle before administration. The treatment was administered every other day (QOD) as a peritumoral injection. Vehicle composition was 4% DMSO, 5% ethanol, 20% PEG200, 71% saline. Dosing volume for all groups: 5 mL/kg.

Body weight and clinical signs were monitored throughout the study.

Tumor volume was measured twice weekly in two dimensions using a caliper, and the volume was expressed in mm³ using the formula: V = 0.5 *a* x *b*² where *a* and *b* are the long and short diameters of the tumor, respectively. The tumor size was then used for calculations of T/C value. The T/C value (in percent) is an indication of antitumor effectiveness; T and C are the mean volumes of the treated and control groups, respectively, on a given day.

One-way ANOVA was performed to compare tumor volume among treated groups and vehicle group, all data were analyzed using GraphPad Prism 5.0. *p* < 0.05 was considered to be statistically significant.

Cell lines tested are presented in Table 1.

**Table 1. Tumour cell lines type and designation**

| Tumour model | Cell line |
|---|---|
| Brain | Kelly |
| | U87MG |
| | SK-N-SH |
| | CHP-134 |
| | DMS-114 |
| Breast | MCF-7 |
| | MDA-MB-468 |
| Colon | LoVo |
| Leukemia | K562 |
| | MOLT-4 |
| | NALM-6 |
| Lung (NSCLC) | NCI-H1299 |
| | NCI-H1975 |
| | HCC-15 |
| Lung (SCLC) | H69 |
| Lung | SHP-77 |
| Lymphoma | Daudi |
| | Raji |
| Pancreas | MIA-PaCa-2 |
| | BxPC-3 |
| Prostate | 22Rv1 |
| Sarcoma | A-673 |
| Skin (Melanoma) | SK-Mel-5 |
| | A2058 |
| | A375 |
| | MeWo |

### Example 1. Activity of SND derivatives against various carcinomas

SND470 and SND490 inhibited various solid carcinomas and hematological tumour cell growth as presented in Tables 2A and 2B.

**Table 2A. SND470 inhibition IC50 values**

| **Cell Line/IC50 (µM)** | **SND470** |
|---|---|
| Kelly | 0.012 |
| MCF7 | 0.021 |
| LoVo | 0.91 |
| K562 | 0.014 |
| MOLT-4 | 0.4 |
| NALM-6 | 0.25 |
| H-1299 | 0.04 |
| HCC-15 | 0.01 |
| H-1975 | 0.14 |
| SHP-77 | 0.92 |
| Daudi | 0.71 |
| Raji | 0.53 |
| MIA-PaCa-2 | 0.022 |
| A-673 | 0.2 |
| SK-Mel-5 | 0.47 |

**Table 2B. SND490 inhibition IC50 values**

| **Cell Line/IC50 (µM)** | **SND490** |
|---|---|
| MOLT-4 | 0.56 |
| A-673 | 1.95 |

SND530 inhibited various solid carcinomas and hematological tumour cell growth as presented in Table 3.

**Table 3. SND530 inhibition IC50 values**

| **Cell line /IC50 (µM)** | **SND530** |
|---|---|
| U87MG | 1.9 |
| MCF-7 | 1 |
| HCC-15 | 3.7 |
| H1299 | 1.4 |
| Mia-PaCa-2 | 1.2 |
| A673 | 0.5 |
| A375 | 1.5 |
| SK-Mel -5 | 1.1 |
| Raji | 0.14 |

SND477, SND478 and SND479 inhibited various solid carcinomas and hematological tumour cell growth as presented in Table 3B

**Table 3B. SND477-479 inhibition IC50 values**

| **Cell line /IC50 (µM)** | **SND477** | **SND478** | **SND479** |
|---|---|---|---|
| SK-N-SH | 0.55 | 0.49 | 0.21 |
| U87MG | 0.32 | 0.39 | 0.24 |
| MCF-7 | 0.21 | 0.22 | 0.17 |
| HCC-15 | 0.24 | 0.22 | 0.19 |
| H69 | 0.23 | 0.21 | 0.23 |
| H1299 | 0.31 | 0.26 | 0.2 |
| Mia-PaCa-2 | 0.2 | 0.19 | 0.11 |
| BxPC-3 | 0.26 | 0.25 | 0.22 |
| 22Rv1 | 0.11 | 0.17 | 0.17 |
| A673 | 0.6 | 0.65 | 0.44 |
| A375 | 0.21 | 0.24 | 0.21 |
| A2058 | 0.24 | 0.27 | 0.19 |
| SKMel-5 | 0.16 | 0.15 | 0.11 |
| HepG2 | 0.66 | 0.68 | 0.44 |

SND540 to SND544B inhibited various solid carcinomas and hematological tumour cell growth as presented in Tables 4A and 4B.

**Table 4A. SND540 inhibition IC50 values**

| **Cell line /IC50 (µM)** | **SND540** |
|---|---|
| SK-N-SH | 1.38 |
| U87MG | 0.65 |
| MCF-7 | 0.21 |
| HCC-15 | 0.46 |
| H69 | 0.28 |
| H1299 | 0.54 |
| Mia-PaCa-2 | 0.26 |
| BxPC-3 | 0.63 |
| 22Rv1 | 0.17 |
| A673 | 1 |
| A375 | 0.48 |
| A2058 | 0.32 |
| SK-Mel -5 | 0.27 |

**Table 4B. SND541 - SND544B inhibition IC50 values**

| **Cell line /IC50 (µM)** | **SND541** | **SND542** | **SND543** | **SND544** | **SND544B** |
|---|---|---|---|---|---|
| SK-N-SH | 1.05 | 2.75 | 3.87 | 0.38 | 1.13 |
| Kelly | 0.61 | 1.71 | 1.44 | 0.23 | 1.16 |
| MDA-MB-468 | 0.38 | 0.51 | 0.56 | 0.25 | 0.61 |
| H460 | 0.88 | 1.78 | 2 | 0.31 | 0.69 |
| HCC-15 | 0.83 | 1.47 | 1.34 | 0.25 | 0.22 |
| LoVo | 2.98 | 4.83 | 14.7 | 0.59 | 2.83 |
| H69 | 0.2 | 0.21 | 0.97 | 0.49 | 0.96 |
| Mia-PaCa-2 | 0.21 | 0.43 | 0.63 | 0.15 | 0.45 |
| BxPC-3 | 0.73 | 1.46 | 1.81 | 0.51 | 1.72 |
| 22Rv1 | 0.37 | 0.8 | 0.92 | 0.17 | 0.43 |
| LNCaP | 0.37 | 0.69 | 0.68 | 0.54 | 0.67 |
| A375 | 0.58 | 0.76 | 1.51 | 0.25 | 0.71 |
| A2058 | 0.48 | 0.69 | 0.98 | 0.33 | 0.66 |
| SK-Mel -5 | 0.51 | 0.87 | 0.97 | 0.19 | 0.38 |

SND550 to SND558 inhibited various solid carcinomas and hematological tumour cell growth as presented in Tables 5A and 5B.

**Table 5A. SND550 inhibition IC50 values**

| **Cell line /IC50 (µM)** | **SND550** |
|---|---|
| SK-N-SH | 3.69 |
| HCC-15 | 1.3 |
| H69 | 0.68 |
| H1299 | 1.3 |
| Mia-PaCa-2 | 4.3 |
| 22Rv1 | 0.75 |
| A375 | 0.92 |

**Table 5B. SND551 - SND558 inhibition IC50 values**

| **Cell line /IC50 (µM)** | SND551 | SND552 | SND553 | SND554 | SND555 | SND556 | SND557 | SND558 |
|---|---|---|---|---|---|---|---|---|
| SK-N-SH | 0.55 | 0.73 | 0.92 | 0.61 | 0.94 | 0.66 | 2.3 | 0.6 |
| HCC-15 | 0.28 | 0.5 | 0.52 | 0.34 | 0.29 | 0.58 | 0.74 | 0.4 |
| H69 | 0.24 | 0.33 | 0.25 | 0.26 | 0.19 | 0.23 | 0.45 | 0.26 |
| H1299 | 0.35 | 0.53 | 0.42 | 0.32 | 0.39 | 0.45 | 1.2 | 0.42 |
| Mia-PaCa-2 | 0.35 | 0.42 | 0.49 | 0.32 | 0.36 | 0.32 | 0.78 | 0.46 |
| 22Rv1 | 0.18 | 0.26 | 0.14 | 0.17 | 0.16 | 0.28 | 0.139 | 0.21 |
| A375 | 0.28 | 0.31 | 0.33 | 0.28 | 0.25 | 0.39 | 0.7 | 0.36 |

### Example 2. Apoptosis of cancer cells induced by SND derivatives

SND470 was tested for induction of apoptosis in a number of cell lines and the results are presented in Table 5.

**Table 5. SND470 induced apoptosis - Fold activation of Caspase 3/7 at the respective concentration**

| **Cell Line/Fold activation (µM)** | **SND470** |
|---|---|
| CHP-134 | 7.02 (3.16) |
| DMS-114 | 3.322 (10) |
| MDA-MB-468 | 3.15 (10) |
| MeWo | 2.55 (10) |
| SK-Mel-5 | 7.22 (1) |
| A375 | 13.98 (10) |
| A2058 | 3.87 (10) |

### Example 3. Signal transduction inhibition of SND derivatives

SND470 and SND544 were evaluated for the inhibition of various signal transduction pathways most frequently deregulated in many cancers. The results are presented in Table 6.

**Table 6. SND470, SND544 and controls inhibition of signal transduction pathways - IC50 values**

| **Pathway Inh** | **Cell line** | **Stimuli** | **Control** | **SND470 (IC50 µM)** | **SND544 (IC50 µM)** | **Control (IC50 µM)** |
|---|---|---|---|---|---|---|
| JAK/STAT | ISRE-bla Jurkat | IFN-alpha | JAK Inhibitor I | 0.36 | 0.3 | 0.019 |
| MAPK/MEK/B-raf | AP1-bla A375 | None | Raf1 Kinase Inh | 2.7 | >10 | 2.24 |
| NFKB | NFKB-bla Jurkat | TNF-alpha | Withaferin A | 1.53 | 0.64 | 0.24 |
| Oxidative Stress | ARE-bla HepG2 | tBHQ | Ro-31-8220 | 1.35 | 0.92 | 1.67 |
| Toll-like Receptor (TLR4) | NFkB-bla THP-1 | LPS | Ro-31-8220 | 0.56 | 0.61 | 0.46 |
| Wnt/Beta-Catenin | LEF-TCF-bla HCT116 | None | ICG-001 | 0.97 | 0.24 | 1.83 |

### Example 4. In vivo activity of SND derivatives

SND470 and SND540 were evaluated for their activity in vivo, in a nude mouse xenograft model of melanoma. The treatments and control vehicle were administered peritumoral QOD for 6 injections for SND470 and 11 injections for SND540. The dose for both derivatives was 16mg/kg. The treatments were generally well tolerated with some body weight loss observed in all groups due to the tumour model itself. Treatment with SND470 and SND540 caused some slight scabbing at the site of the injection which resolved after the treatment was stopped.

Both SND470 and SND540 showed significant activity in inhibiting A2058 melanoma cell line as showed in Table 7.

**Table 7. SND470 and SND540 in vivo inhibition of melanoma cell line - T/C, TGI and p values at day 20 after treatment start.**

| **Group** | **Treatment** | **Tumor Volume (mm³) ^{a}** | **T/C (%) ^{b}** | **TGI (%) ^{c}** | ***p* value** |
|---|---|---|---|---|---|
| 1 | Vehicle | 2261±252 | -- | -- | -- |
| 2 | SND470,16 mg/kg | 886±127 | 39.2 | 62.8 | *p*<0.0001 |
| 3 | SND540, 12 mg/kg | 706±108 | 31.2 | 71.0 | *p*<0.0001 |

a. Mean ± SEM;
b. Tumor Growth Inhibition is calculated by dividing the group average tumor volume for the treated group by the group average tumor volume for the control group (T/C).
c: TGI (%) = [1-(Ti-To)/ (Vi-Vo)] ×100; Ti is the average tumor volume of a treatment group on a given day, To is the average tumor volume of the treatment group on the day of treatment start, Vi is the average tumor volume of vehicle control group on the same day with Ti, and Vo is the average tumor volume of vehicle group on the day of treatment start.

### Example 5. In vivo activity of SND477 & 479

SND477 and SND479 were evaluated for their activity in vivo, in a nude mouse xenograft model of melanoma.

Both SND477 and SND479 showed significant activity in inhibiting A2058 melanoma cell line as showed in Table 8.

**Table 8. SND477 and SND479 in vivo inhibition of melanoma cell line - T/C, TGI and p values at day 20 after treatment start.**

| **Treatment** | **T/C (%) ^{b}** | **TGI (%) ^{c}** | ***p* value** |
|---|---|---|---|
| SND477, 12 mg/kg | 35.6 | 68.7 | *p*<0.0001 |
| SND479, 12 mg/kg | 33.5 | 70.0 | *p*<0.0001 |

a. Mean ± SEM;
b. Tumor Growth Inhibition is calculated by dividing the group average tumor volume for the treated group by the group average tumor volume for the control group (T/C).
c: TGI (%) = [1-(Ti-To)/ (Vi-Vo)] ×100; Ti is the average tumor volume of a treatment group on a given day, To is the average tumor volume of the treatment group on the day of treatment start, Vi is the average tumor volume of vehicle control group on the same day with Ti, and Vo is the average tumor volume of vehicle group on the day of treatment start.

## Claims

1. A compound of formula (1), or a pharmaceutically acceptable salt, multi-salt or solvate thereof: 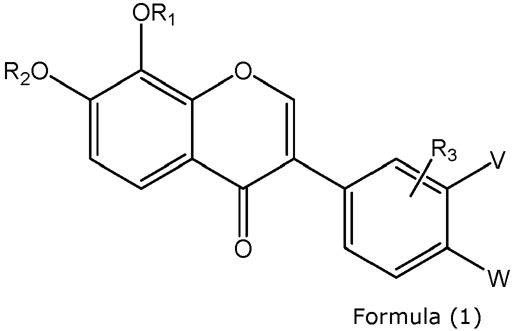 wherein:
either V is -R₃, and W is: 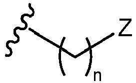
or W is -R₃, and V is: 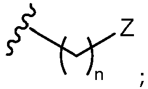
Z is -[P(R⁵)₃]X;
X is a counter anion;
R¹ and R², independently, are selected from -H, -C₁₋₄ alkyl, -C(O)R⁴, -C(O)NHR⁴, and -C(O)N(R⁴)₂; or R¹ and R² together form a C₁₋₄ alkylene group;
R³ is selected from H; halo; -CN; -NO₂; -R^{β}; -OH; -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; -COOR^{β}; and -OCOR^{β};
each -R^{β} is independently selected from a C₁-C₆ alkyl, C₂-C₆ alkenyl, C₂-C₆ alkynyl or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C=CH, -CHO, -CON(CH₃)₂ or oxo (=O) groups;
each -R⁴ is independently selected from C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), - N(C₁₋₆ alkyl)₂, C₁₋₆ alkylsulfinyl, C₁₋₆ alkylsulfonyl, or arylsulfonyl;
R⁵ is independently selected from H, C₁-C₆ alkyl, C₂-C₆ alkenyl, C₃-C₁₄ aryl group, or C₃-C₁₄ aliphatic cyclic group; and wherein any -R⁵ may optionally be substituted with one or more C₁-C₄ alkyl, halo, -CF₃, -OH, -NH₂, -CN, -C=CH or oxo (=O) groups; and
n is an integer from 1 to 10.

2. A compound as claimed in claim 1, wherein the compound is a compound of formula (2), (2A), (2B), (3), (3A) or (3B): 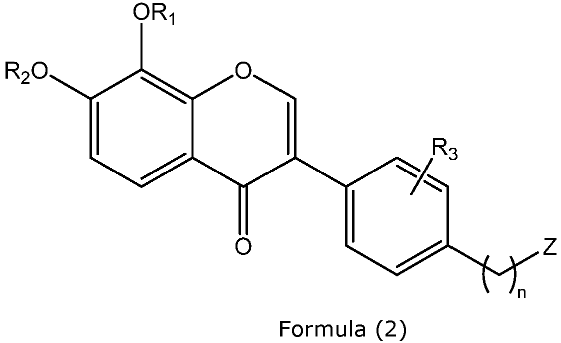 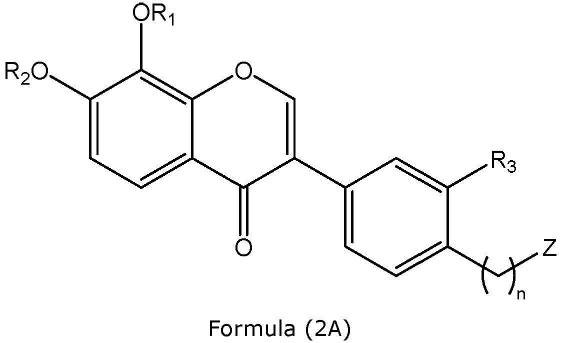 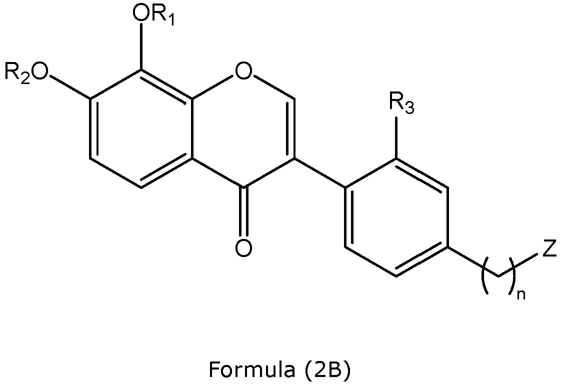 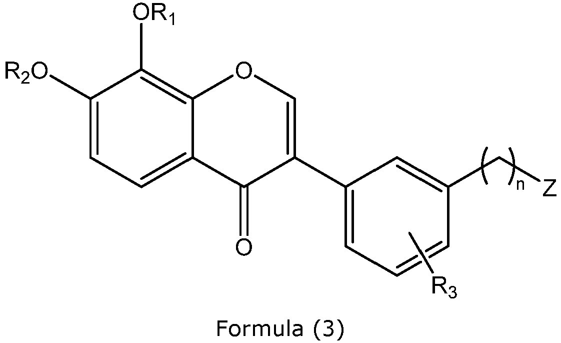 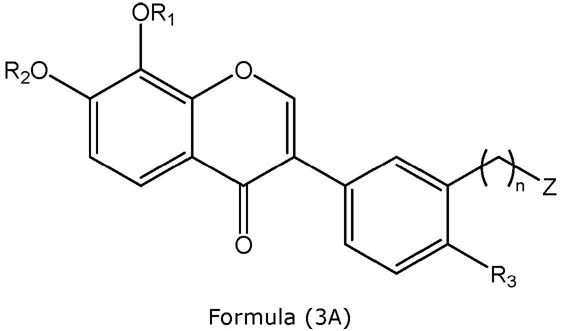 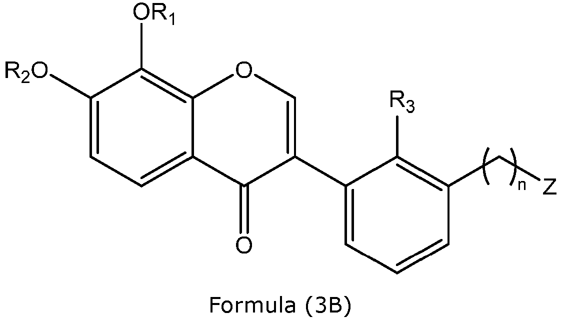 wherein R¹, R², R³, Z and n are as defined in claim 1.

3. A compound as claimed in claim 1 or claim 2, wherein R¹ and R², independently, are selected from -H and -CH₃.

4. A compound as claimed in any one or more of claims 1 to 3, wherein R³ is selected from -CH₃; -Cl; -F; and -OCH₃.

5. A compound as claimed in any one or more of claims 1 to 3, wherein R³ is -H.

6. A compound as claimed in any one or more of the preceding claims, wherein X is fluoride, chloride, bromide or iodide.

7. A compound as claimed in claim 6, wherein X is bromide or chloride, for example X is bromide.

8. A compound as claimed in any one or more of the preceding claims, wherein each - R⁵ is independently a C₃-C₁₄ aryl group; wherein any - R⁵ may optionally be substituted with one or more C₁-C₄ alkyl, halo, -OH, -NH₂, -CN, -C=CH or oxo (=O) groups, or each - R⁵ is a phenyl group; each phenyl group may optionally be substituted with one or more C₁-C₄ alkyl, halo, -OH, -NH₂, -CN, -C=CH or oxo (=O) groups.

9. A compound as claimed in claim 8, wherein each R⁵ is a phenyl group.

10. A compound as claimed in any one or more of claims 1 to 7, wherein each - R⁵ is phenyl optionally substituted with -CF₃.

11. A compound as claimed in any one or more of the preceding claims, wherein n is an integer from 2 to 10, for example, wherein n is 3, 4 or 5, for example n is 4.

12. A compound as claimed in claim 1, wherein the compound of formula (1) is selected from:

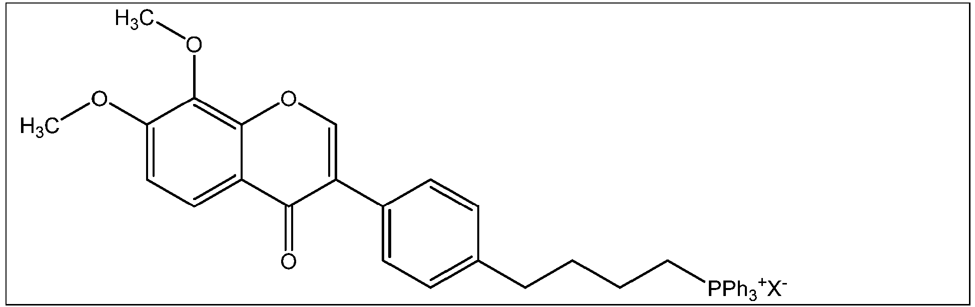

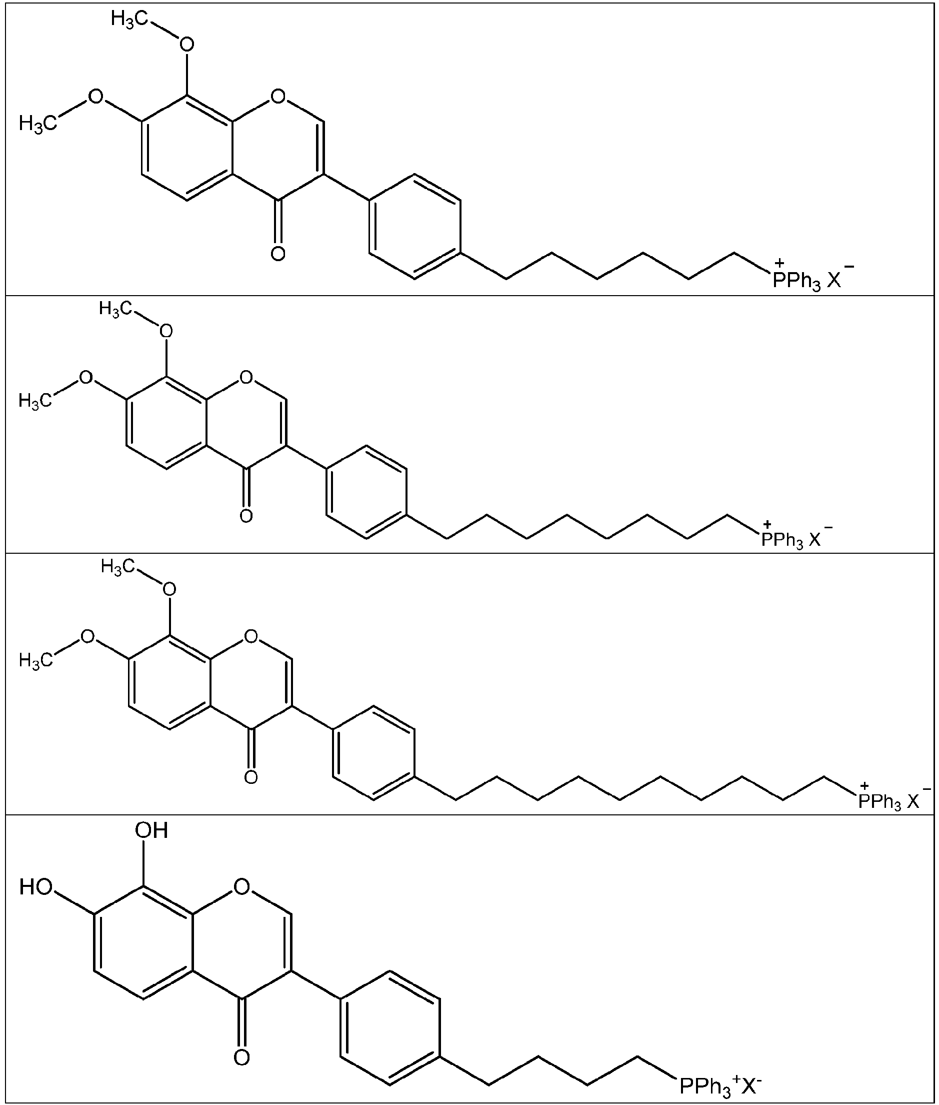

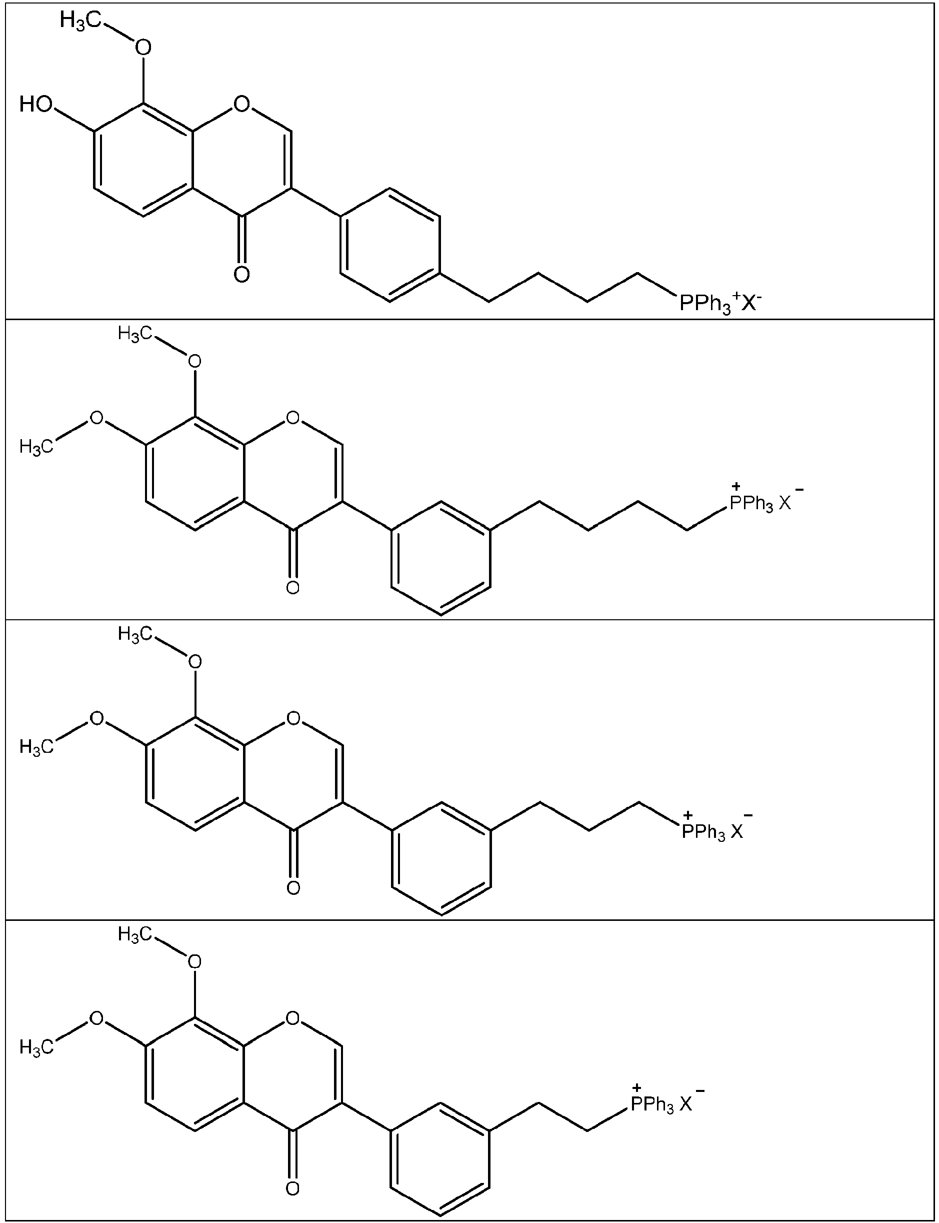

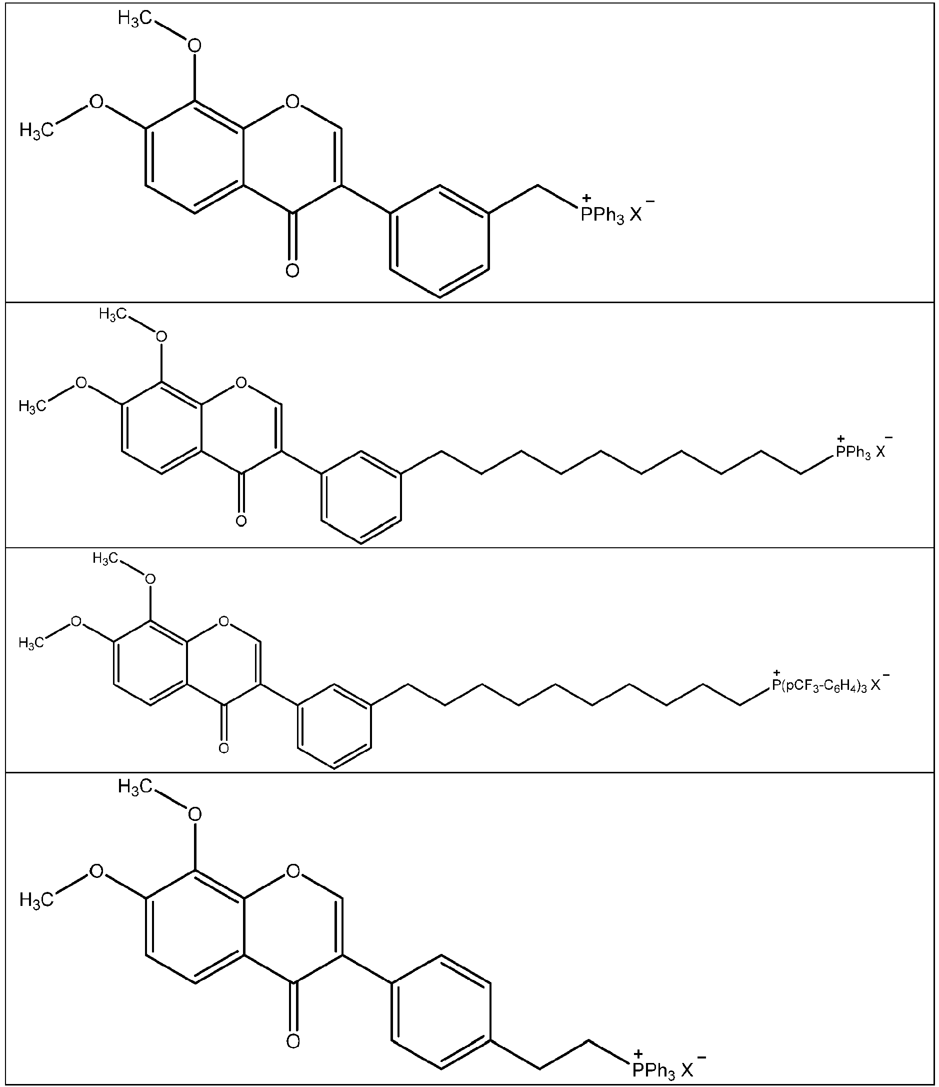

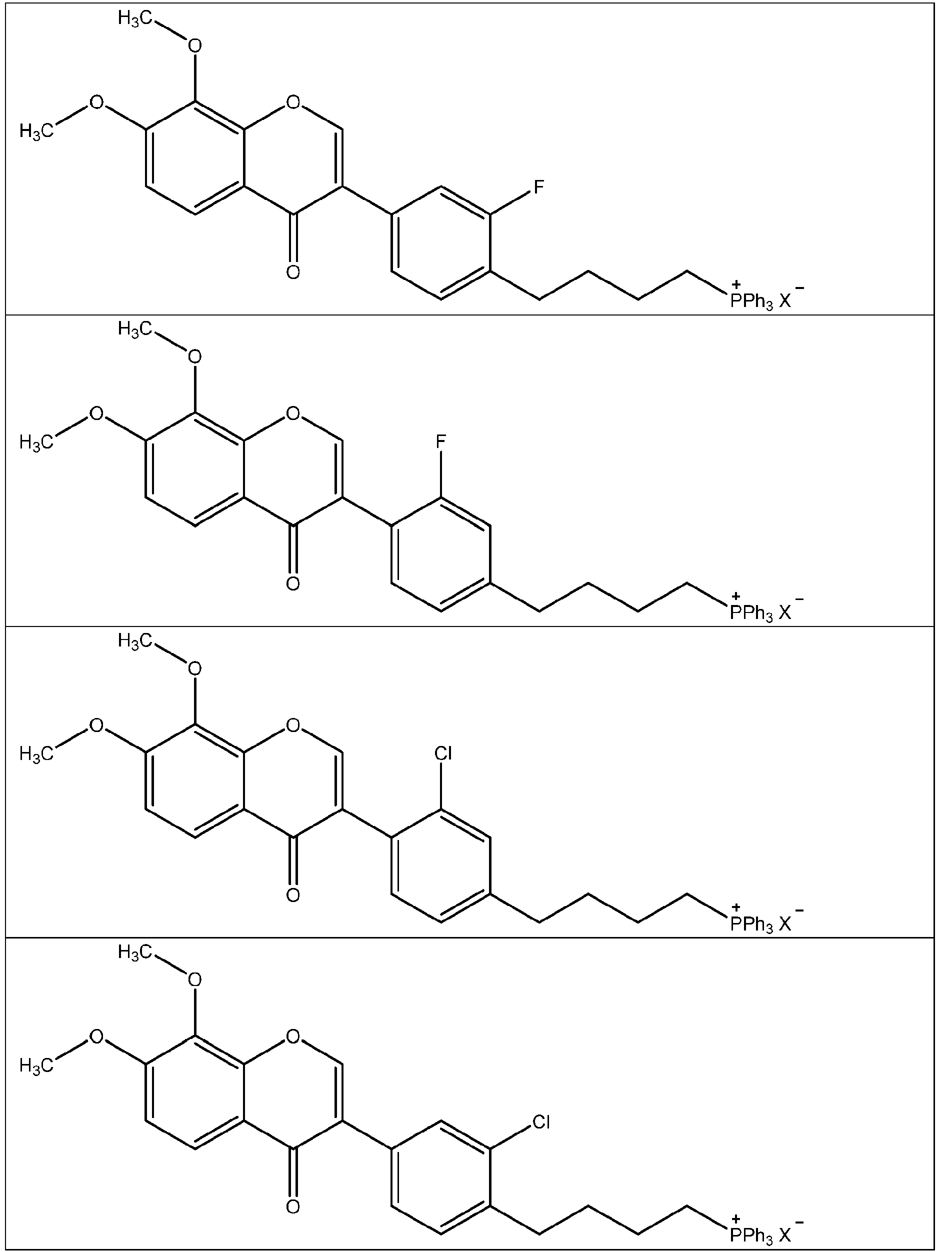

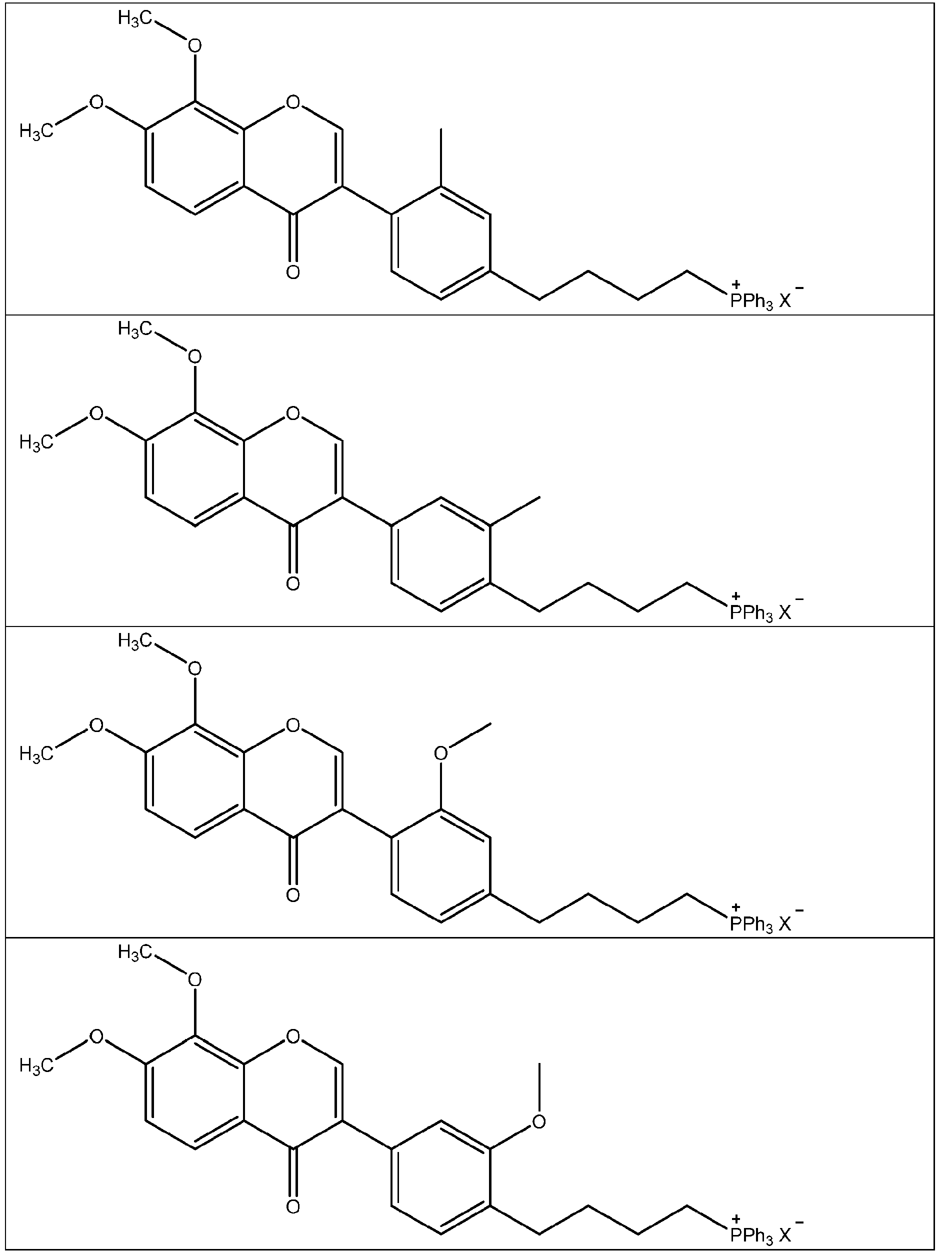

13. A pharmaceutical composition comprising a compound, or a pharmaceutically acceptable salt, multi-salt, or solvate as defined in one or more of claims 1 to 12, and a pharmaceutically acceptable excipient.

14. A compound, or a pharmaceutically acceptable salt, multi-salt, or solvate as defined in one or more of claims 1 to 12, or a pharmaceutical composition as defined in claim 13, for use in medicine.

15. A compound, or a pharmaceutically acceptable salt, multi-salt, or solvate as defined in one or more of claims 1 to 12, or a pharmaceutical composition as defined in claim 13, for use treating or preventing cancer.

## Patentansprüche

1. Verbindung der Formel (1) oder pharmazeutisch unbedenkliches Salz, Mehrfachsalz oder Solvat davon: 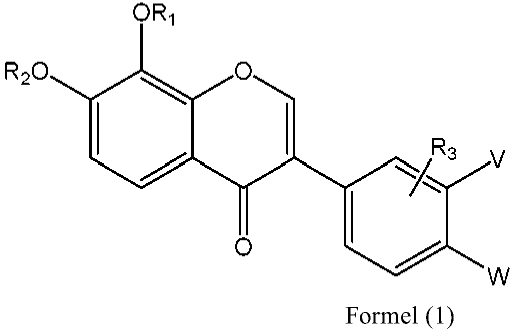 wobei:
entweder V -R₃ ist und W Folgendes ist: 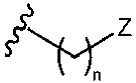
oder W -R₃ ist und V Folgendes ist: 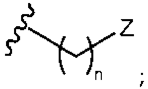
Z -[P(R⁵)₃]X ist;
X ein Gegenanion ist;
R¹ und R² unabhängig ausgewählt sind aus -H, -C₁-₄-Alkyl, -C(O)R⁴, -C(O)NHR⁴ und - C(O)N(R⁴)₂; oder R¹ und R² zusammen eine C₁-₄-Alkylengruppe bilden;
R³ ausgewählt ist aus H; Halogen; -CN; -NO₂; -R^{β}; -OH; -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂, -NHR^{β}; -N(R^{β})₂; -CHO; -COR^{β}; -COOH; -COOR^{β}; und -OCOR^{β};
jedes -R^{β} unabhängig ausgewählt ist aus einer C₁-C₆-Alkyl-, C₂-C₆-Alkenyl-, C₂-C₆-Alkinyl- oder C₃-C₁₄ zyklischen Gruppe, und wobei jedes -R^{β} optional mit einer oder mehreren C₁-C₄-Alkyl-, C₁-C₄-Halogenalkyl-, C₃-C₇-Cycloalkyl-, -O(C₁-C₄-Alkyl), -O(C₁-C₄-Halogenalkyl), -O(C₃-C₇-Cycloalkyl)-, Halogen-, -OH-, -NH₂-, -CN-, -NO₂-, -C=CH-, -CHO-, -CON(CH₃)₂- oder Oxo-(=O)-Gruppen substituiert sein kann;
jedes -R⁴ unabhängig ausgewählt ist aus C₁-C₆-Alkyl, C₂-C₆-Alkenyl, C₂-C₆-Alkinyl, C₃-₁₄-zyklischer Gruppe, Halogen, -NO₂, -CN, -OH, -NH₂, Mercapto, Formyl, Carboxy, Carbamoyl, C₁-₆-Alkoxy, C₁-₆-Alkylthio, -NH(C₁-₆-Alkyl), -N(C₁-₆-Alkyl)₂, C₁-₆-Alkylsulfinyl, C₁-₆-Alkylsulfonyl oder Arylsulfonyl;
R⁵ unabhängig ausgewählt ist aus H, C₁-C₆-Alkyl, C₂-C₆-Alkenyl, C₃-C₁₄-Arylgruppe oder C₃-C₁₄ aliphatischer zyklischer Gruppe; und wobei jedes -R⁵ optional substituiert sein kann mit einer oder mehreren C₁-C₄-Alkyl-, Halogen-, -CF₃-, -OH-, -NH₂-, -CN-, -C≡CH- oder Oxo- (=O)-Gruppen; und
n eine ganze Zahl von 1 bis 10 ist.

2. Verbindung, wie in Anspruch 1 beansprucht, wobei die Verbindung eine Verbindung der Formel (2), (2A), (2B), (3), (3A) oder (3B) ist: 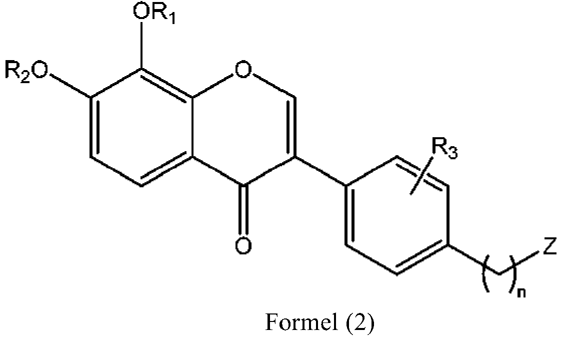 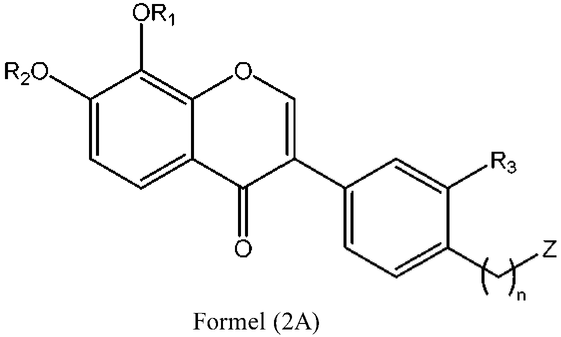 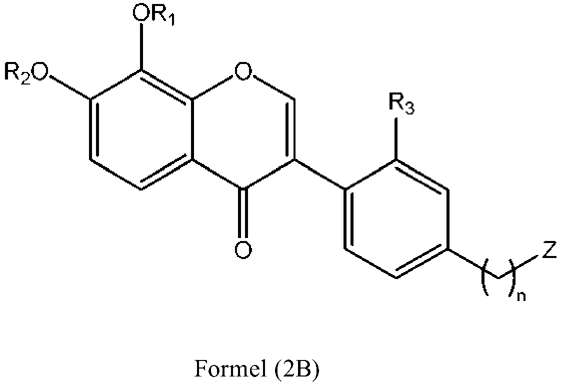 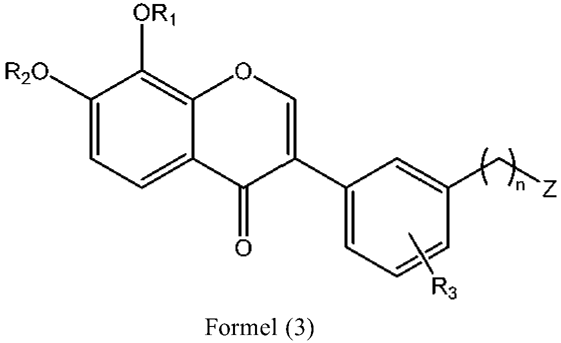 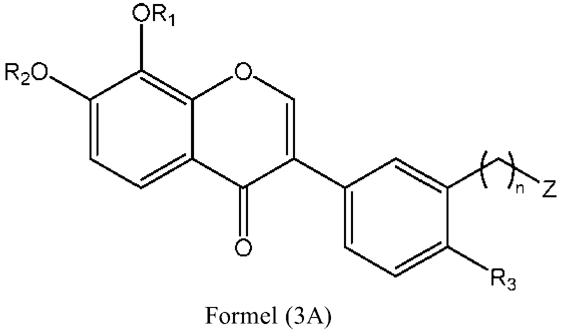 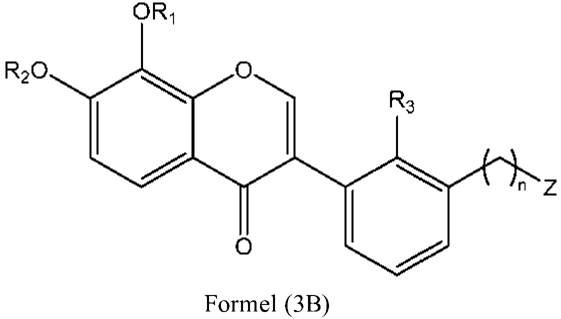 wobei R¹, R², R³, Z und n wie in Anspruch 1 definiert sind.

3. Verbindung, wie in Anspruch 1 oder Anspruch 2 beansprucht, wobei R¹ und R² unabhängig ausgewählt sind aus -H und -CH₃.

4. Verbindung, wie in einem oder mehreren der Ansprüche 1 bis 3 beansprucht, wobei R³ ausgewählt ist aus -CH₃; -Cl; -F; und -OCH₃.

5. Verbindung, wie in einem oder mehreren der Ansprüche 1 bis 3 beansprucht, wobei R³ -H ist.

6. Verbindung, wie in einem oder mehreren der vorhergehenden Ansprüche beansprucht, wobei X Fluorid, Chlorid, Bromid oder Iodid ist.

7. Verbindung, wie in Anspruch 6 beansprucht, wobei X Bromid oder Chlorid ist, zum Beispiel ist X Bromid.

8. Verbindung, wie in einem oder mehreren der vorhergehenden Ansprüche beansprucht, wobei jedes -R⁵ unabhängig eine C₃-C₁₄-Arylgruppe ist; wobei jedes -R⁵ optional mit einer oder mehreren C₁-C₄-Alkyl-, Halogen-, -OH-, -NH₂-, -CN-, -C≡CH- oder Oxo (=O)-Gruppen substituiert sein kann oder jedes -R⁵ eine Phenylgruppe ist; wobei jede Phenylgruppe optional mit einer oder mehreren C₁-C₄-Alkyl-, Halogen-, -OH-, -NH₂-, -CN-, -C=CH- oder Oxo- (=O)-Gruppen substituiert sein kann.

9. Verbindung, wie in Anspruch 8 beansprucht, wobei jedes R⁵ eine Phenylgruppe ist.

10. Verbindung, wie in einem oder mehreren der Ansprüche 1 bis 7 beansprucht, wobei jedes -R⁵ optional mit -CF₃ substituiertes Phenyl ist.

11. Verbindung, wie in einem oder mehreren der vorhergehenden Ansprüche beansprucht, wobei n zum Beispiel eine ganze Zahl von 2 bis 10 ist, wobei n 3, 4 oder 5 ist, zum Beispiel n 4 ist.

12. Verbindung, wie in Anspruch 1 beansprucht, wobei die Verbindung der Formel (1) ausgewählt ist aus:

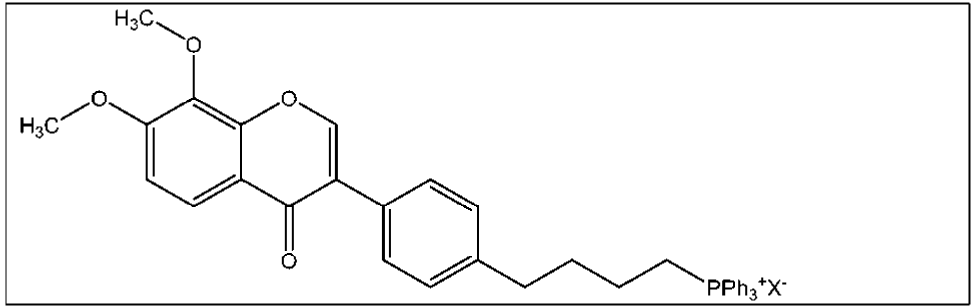

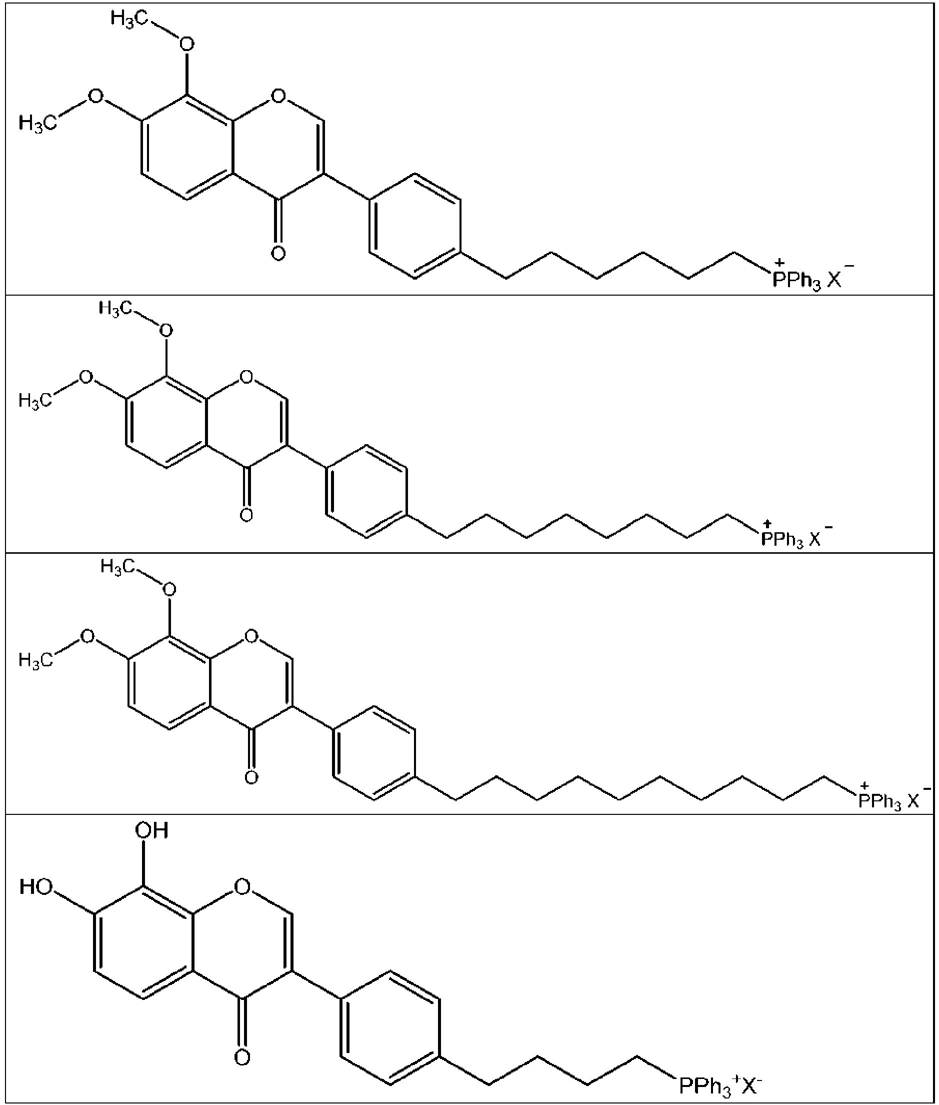

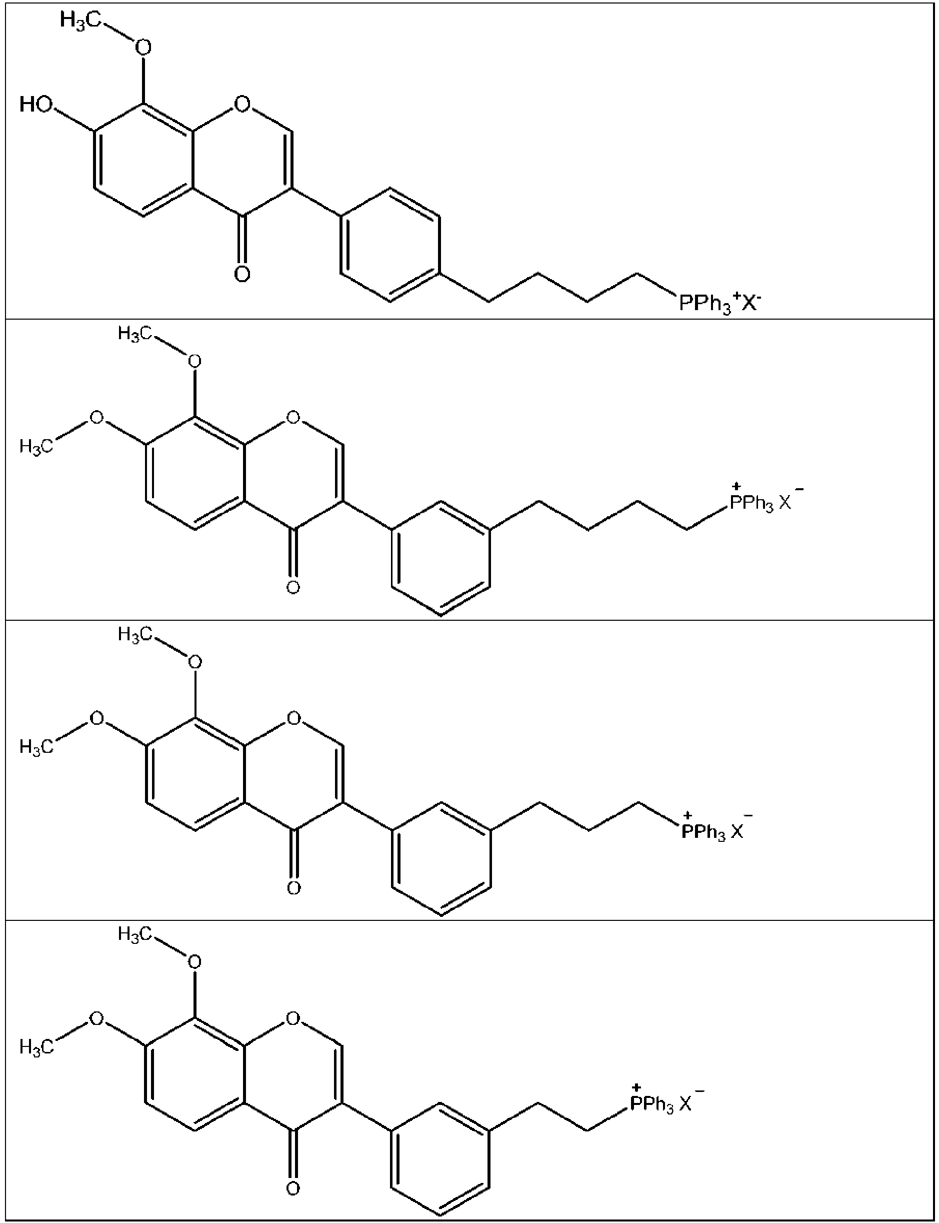

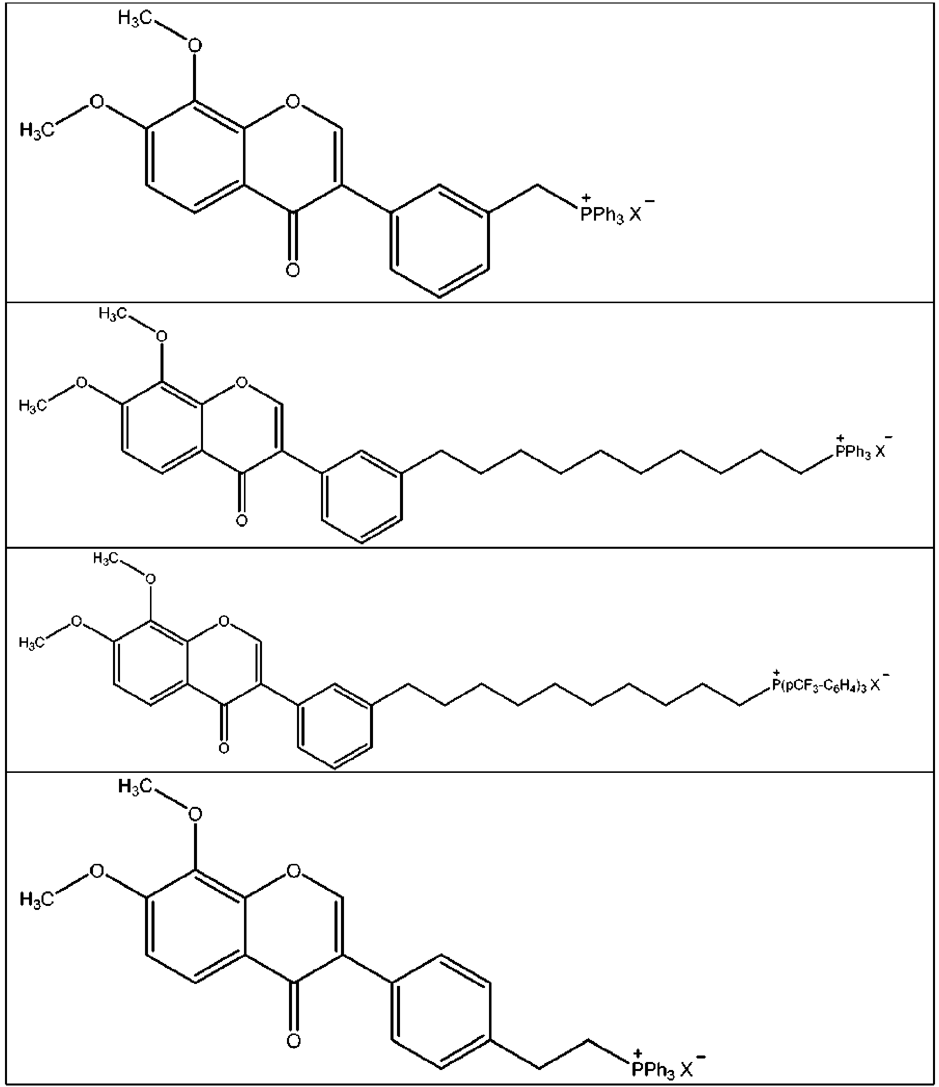

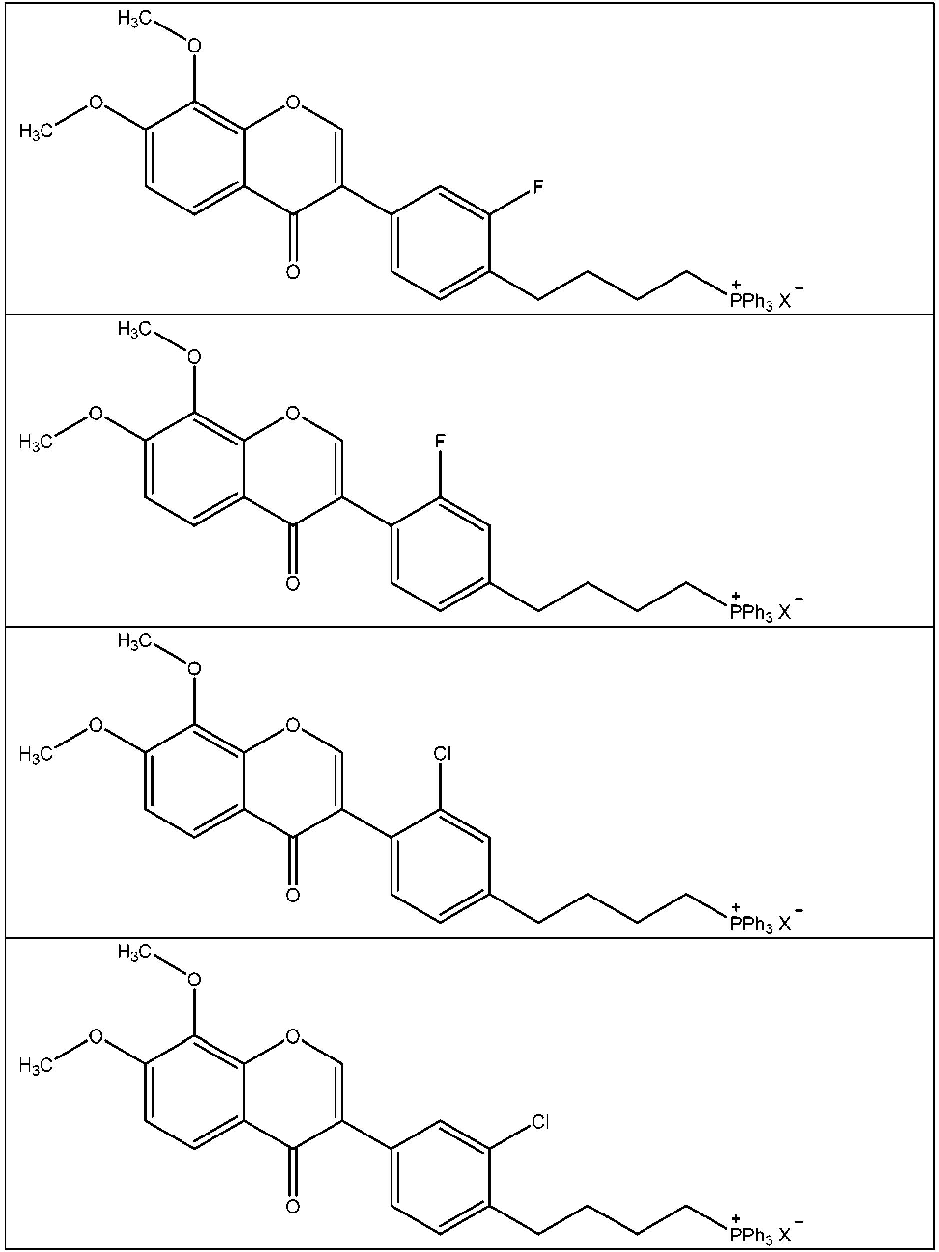

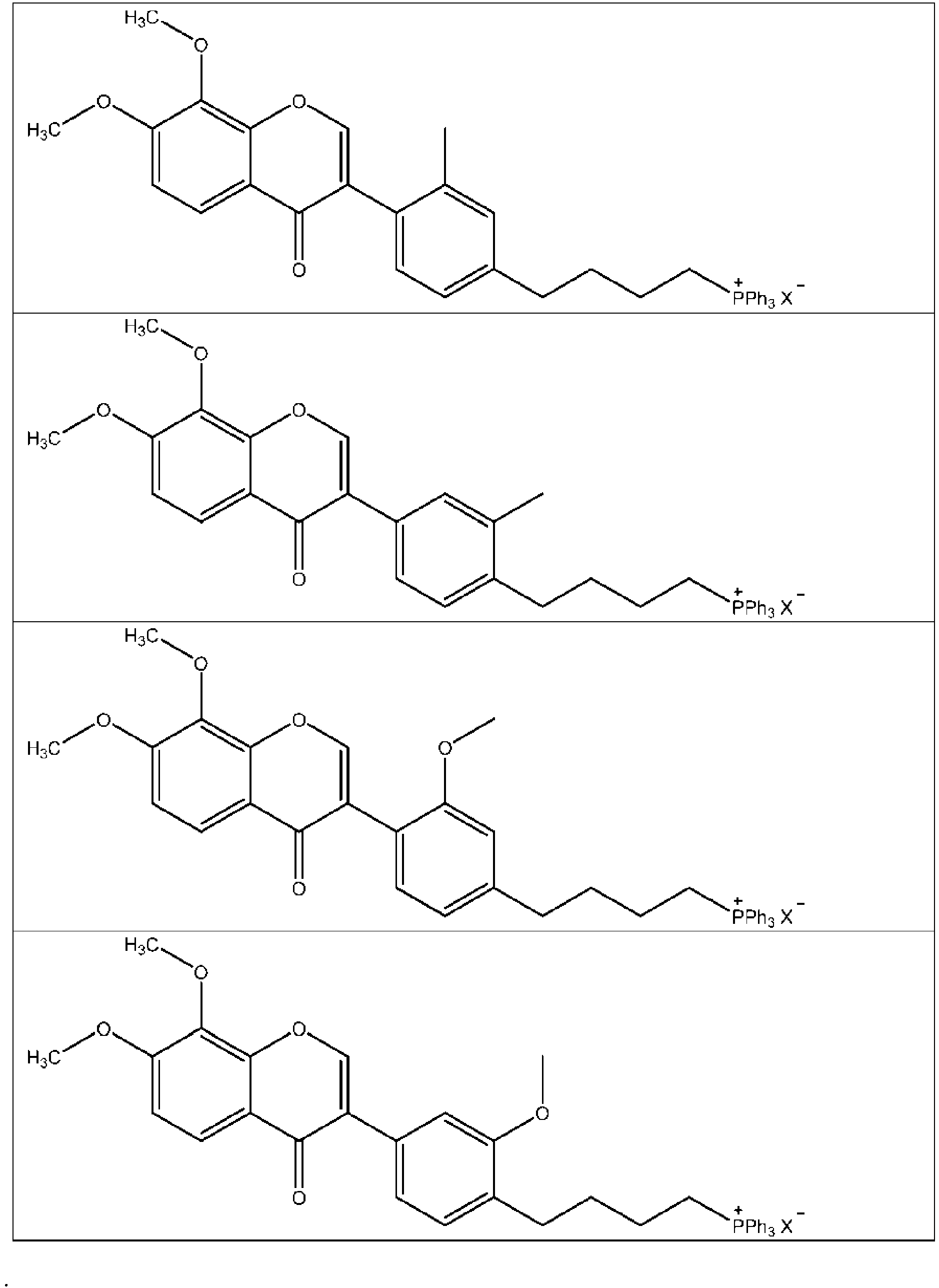

13. Pharmazeutische Zusammensetzung, umfassend eine Verbindung oder ein pharmazeutisch unbedenkliches Salz, Mehrfachsalz oder Solvat, wie in einem oder mehreren der Ansprüche 1 bis 12 definiert, und einen pharmazeutisch unbedenklichen Hilfsstoff.

14. Verbindung oder pharmazeutisch unbedenkliches Salz, Mehrfachsalz oder Solvat, wie in einem oder mehreren der Ansprüche 1 bis 12 definiert, oder pharmazeutische Zusammensetzung, wie in Anspruch 13 definiert, zur Verwendung in der Medizin.

15. Verbindung oder pharmazeutisch unbedenkliches Salz, Mehrfachsalz oder Solvat, wie in einem oder mehreren der Ansprüche 1 bis 12 definiert, oder pharmazeutische Zusammensetzung, wie in Anspruch 13 definiert, zur Verwendung zum Behandeln oder Vorbeugen von Krebs.

## Revendications

1. Composé de formule (1), ou sel, multi-sel ou solvate acceptable pharmaceutiquement de celui-ci : 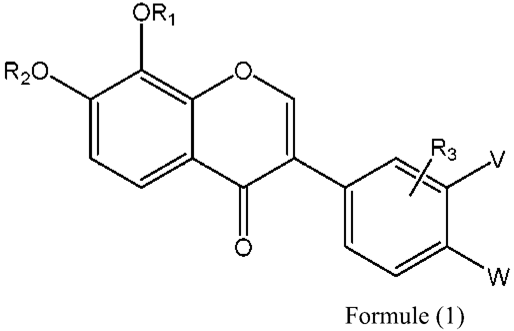 où :
soit V est -R₃ et W est : 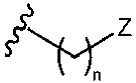
ou W est -R₃ et V est : 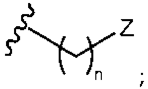
Z est -[P(R⁵)₃]X ;
X est un contre-anion ;
R¹ et R² sont, indépendamment, choisis parmi -H, -alkyle en C₁₋₄, -C(O)R⁴, -C(O)NHR⁴ et -C(O)N(R⁴)₂ ; ou R¹ et R² forment ensemble un groupe alkylène en C₁₋₄ ;
R³ est choisi parmi H ; un halogéno ; -CN ; -NO₂ ; -R^{β} ; -OH ; -OR^{β} ; -SH ; -SR^{β} ; -SOR^{β} ; -SO₂H ; -SO₂R^{β} ; -SO₂NH₂ ; -SO₂NHR^{β} ; -SO₂N(R^{β})₂ ; -NH₂ ; -NHR^{β} ; -N(R^{β})₂ ; -CHO ; -COR^{β} ; -COOH ; -COOR^{β} ; et -OCOR^{β} ;
chaque -R^{β} est indépendamment choisi parmi un alkyle en C₁-C₆, un alcényle en C₂-C₆, un alcynyle en C₂-C₆ ou un groupe cyclique en C₃-C₁₄, et où tout -R^{β} peut éventuellement être substitué par un ou plusieurs groupes alkyle en C₁-C₄, halogénoalkyle en C₁-C₄, cycloalkyle en C₃-C₇, -O(alkyle en C₁-C₄), -O(halogénoalkyle en C₁-C₄), -O(cycloalkyle en C₃-C₇), halogéno, - OH, -NH₂, -CN, -NO₂, -C≡CH, -CHO, -CON(CH₃)₂ ou oxo (=O) ;
chaque -R⁴ est indépendamment choisi parmi un alkyle en C₁-C₆, un alcényle en C₂-C₆, un alcynyle en C₂-C₆, un groupe cyclique en C₃-₁₄, un halogéno, -NO₂, -CN, -OH, -NH₂, un mercapto, un formyle, un carboxy, un carbamoyle, un alcoxy en C₁₋₆, un alkylthio en C₁₋₆, -NH(alkyle en C₁₋₆), -N(alkyle en C₁₋₆)₂, un alkylsulfinyle en C₁₋₆, un alkylsulfonyle en C₁₋₆ ou un arylsulfonyle ;
R⁵ est indépendamment choisi parmi H, un alkyle en C₁-C₆, un alcényle en C₂-C₆, un groupe aryle en C₃-C₁₄ ou un groupe cyclique aliphatique en C₃-C₁₄ ; et où tout -R⁵ peut éventuellement être substitué par un ou plusieurs groupes alkyle en C₁-C₄, halogéno, -CF₃, -OH, - NH₂, -CN, -C=CH ou oxo (=O) ; et
n est un entier de 1 à 10.

2. Composé selon la revendication 1, dans lequel le composé est un composé de formule (2), (2A), (2B), (3), (3A) ou (3B) : 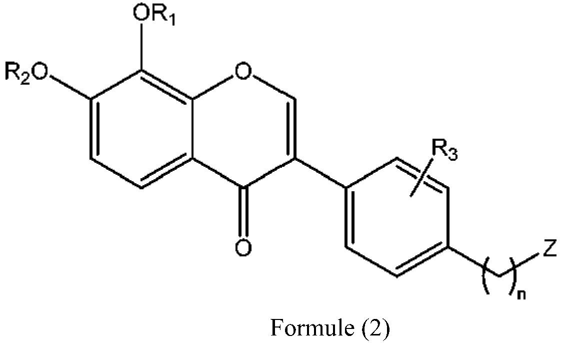 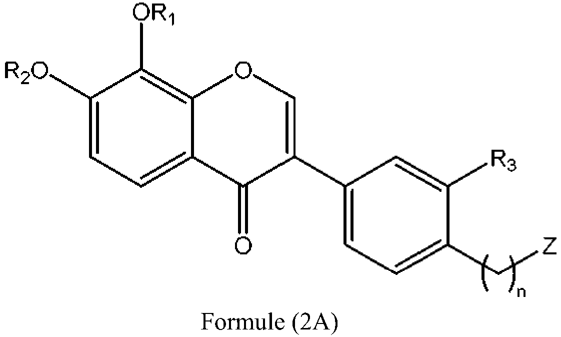 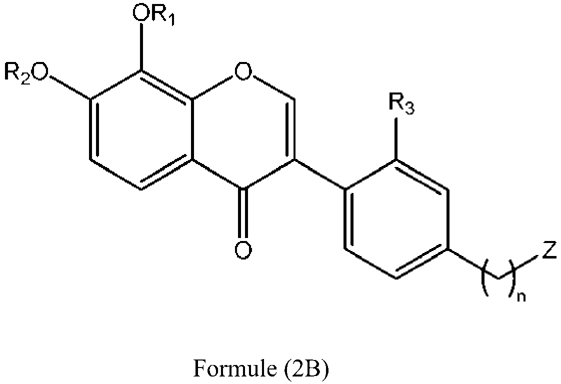 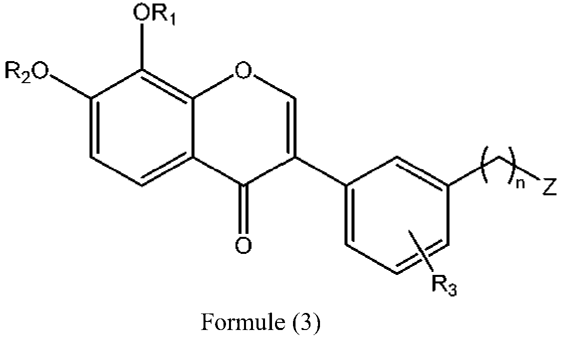 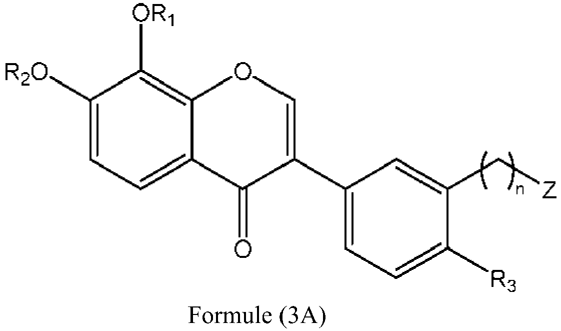 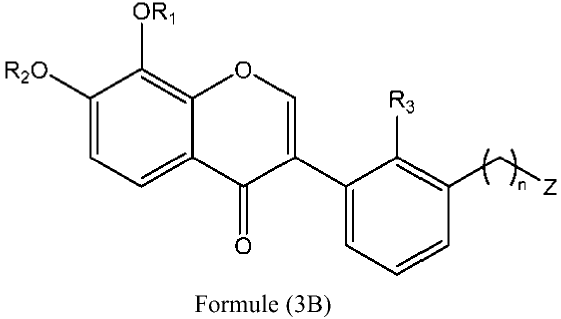 où R¹, R², R³, Z et n sont tels que définis dans la revendication 1.

3. Composé selon la revendication 1 ou la revendication 2, dans lequel R¹ et R² sont, indépendamment, choisis parmi -H et -CH₃.

4. Composé selon l'une quelconque ou plusieurs des revendications 1 à 3, dans lequel R³ est choisi parmi -CH₃ ; -Cl ; -F ; et -OCH₃.

5. Composé selon l'une quelconque ou plusieurs des revendications 1 à 3, dans lequel R³ est -H.

6. Composé selon l'une quelconque ou plusieurs des revendications précédentes, dans lequel X est un fluorure, un chlorure, un bromure ou un iodure.

7. Composé selon la revendication 6, dans lequel X est un bromure ou un chlorure, par exemple X est un bromure.

8. Composé selon l'une quelconque ou plusieurs des revendications précédentes, dans lequel chaque -R⁵ est indépendamment un groupe aryle en C₃-C₁₄ ; où tout -R⁵ peut éventuellement être substitué par un ou plusieurs groupes alkyle en C₁-C₄, halogéno, -OH, -NH₂, -CN, -C=CH ou oxo (=O), ou chaque -R⁵ est un groupe phényle ; chaque groupe phényle peut éventuellement être substitué par un ou plusieurs groupes alkyle en C₁-C₄, halogéno, -OH, -NH₂, -CN, -C=CH ou oxo (=O).

9. Composé selon la revendication 8, dans lequel chaque R⁵ est un groupe phényle.

10. Composé selon l'une quelconque ou plusieurs des revendications 1 à 7, dans lequel chaque -R⁵est un phényle éventuellement substitué par -CF₃.

11. Composé selon l'une quelconque ou plusieurs des revendications précédentes, dans lequel n est un entier de 2 à 10, par exemple, dans lequel n vaut 3, 4 ou 5, par exemple n vaut 4.

12. Composé selon la revendication 1, dans lequel le composé de formule (1) est choisi parmi :

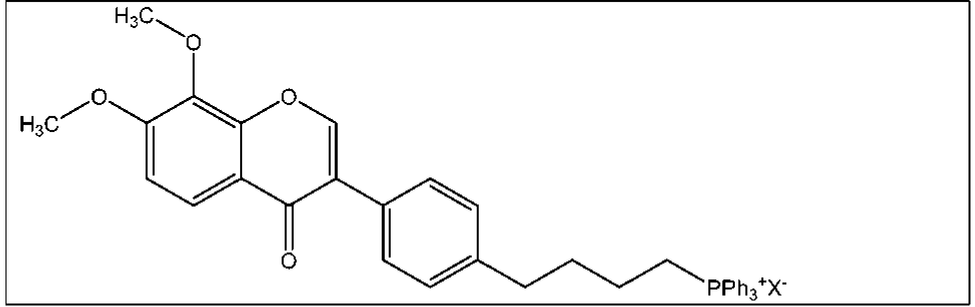

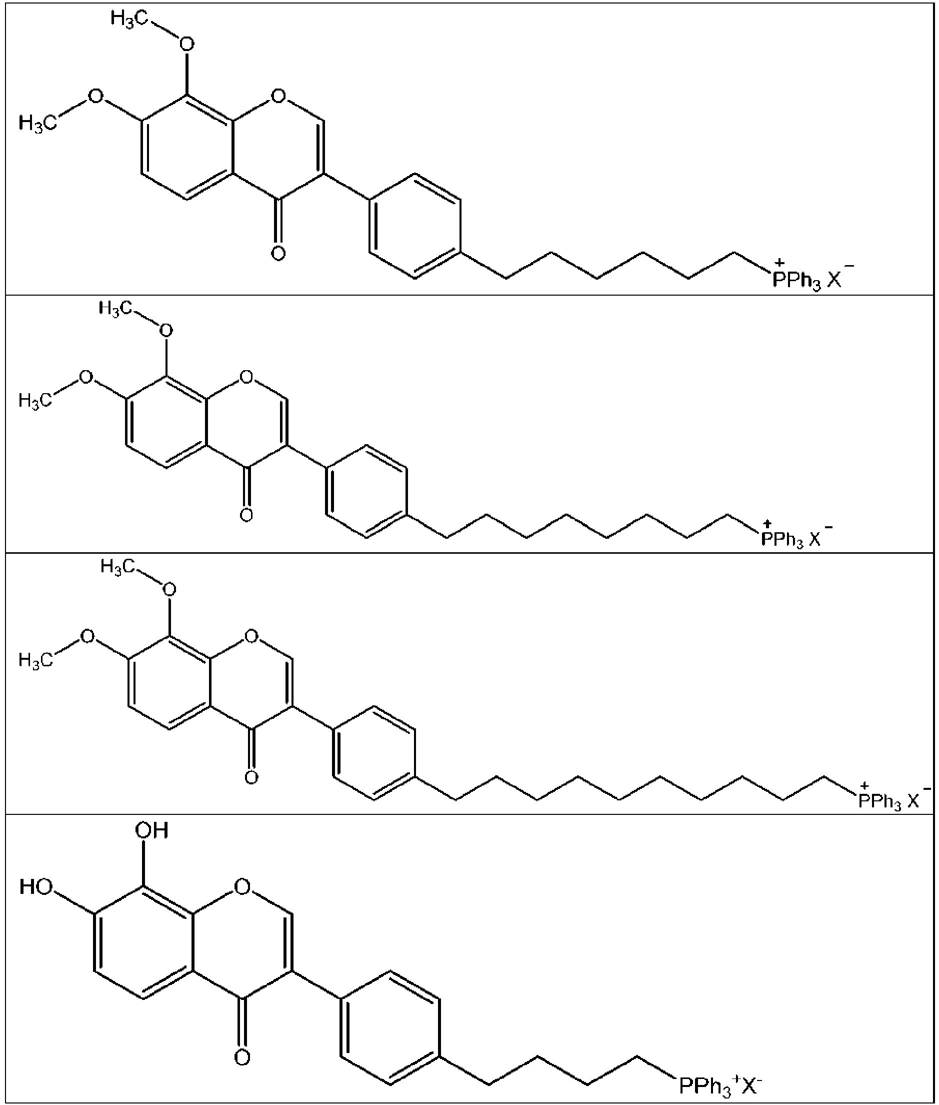

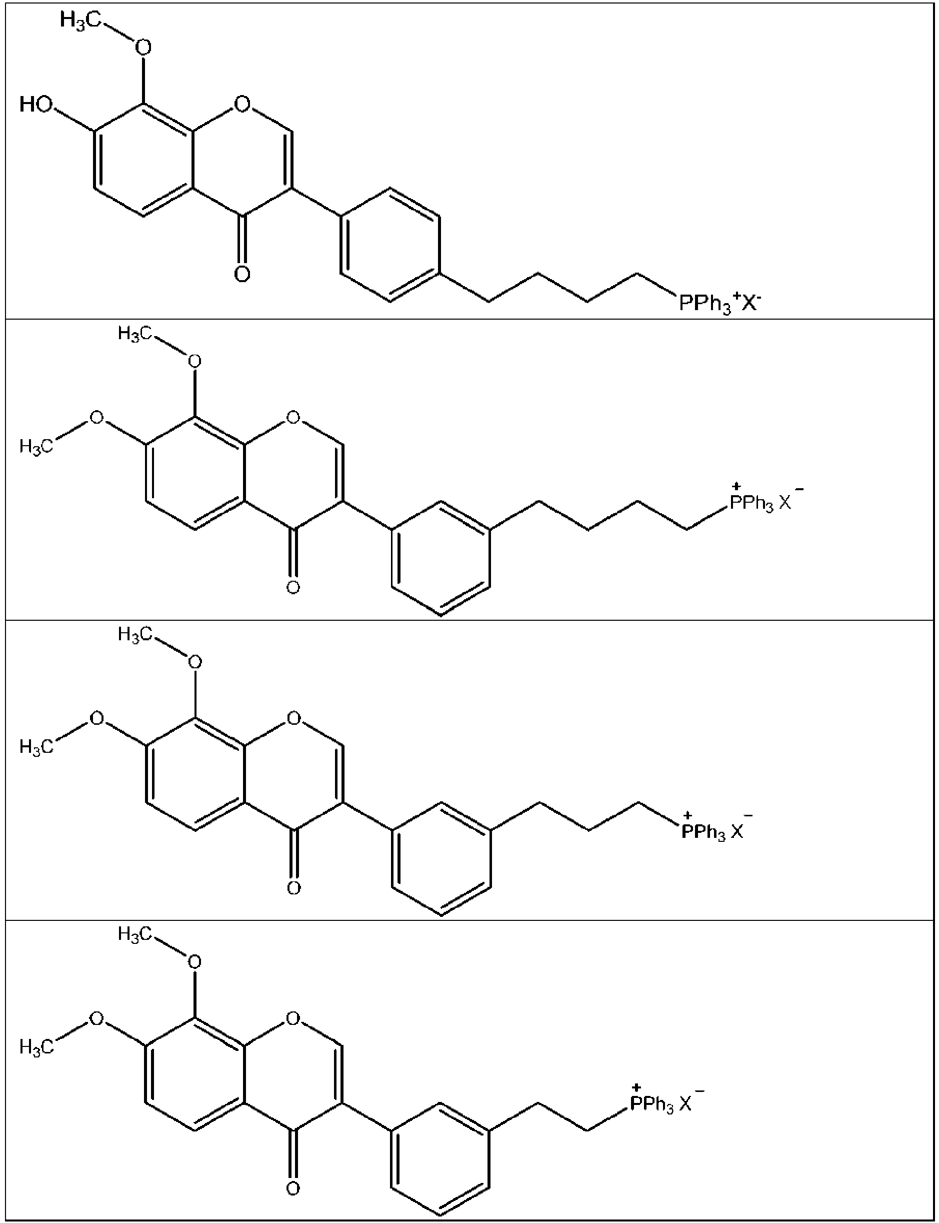

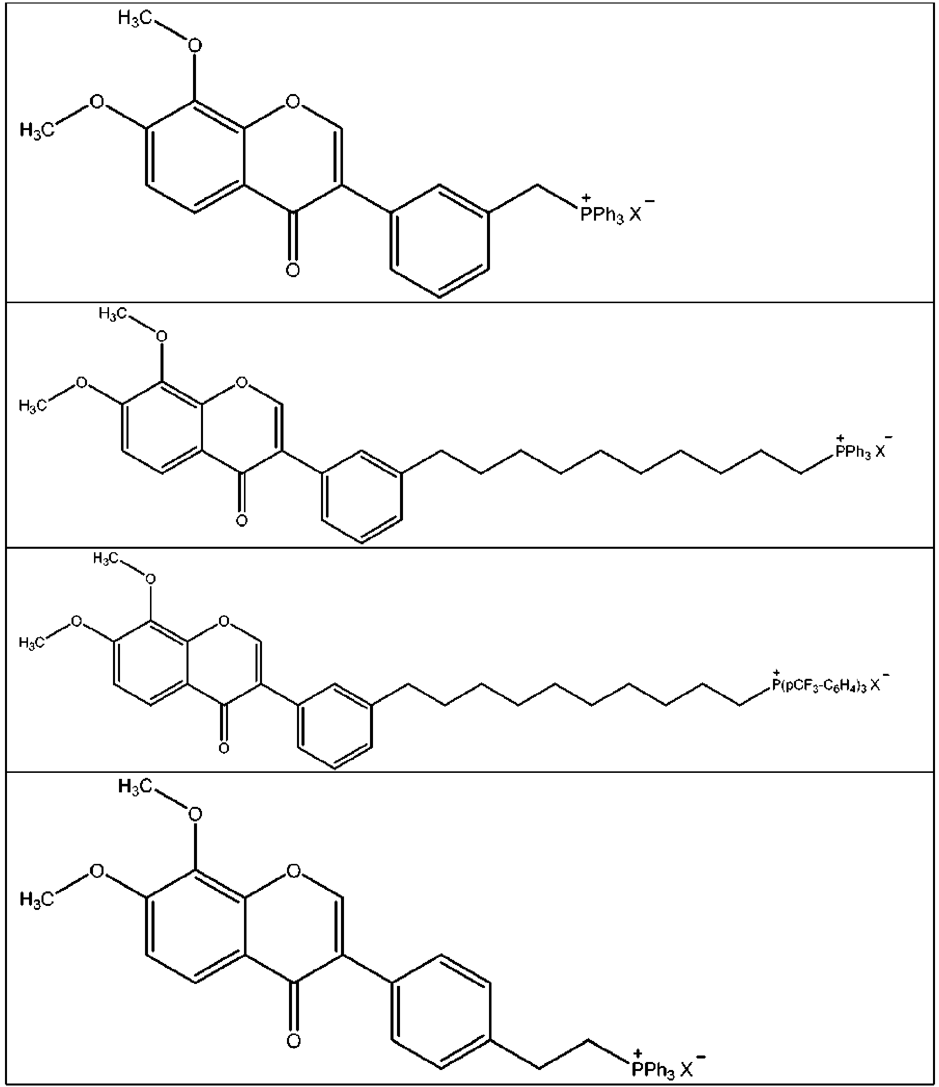

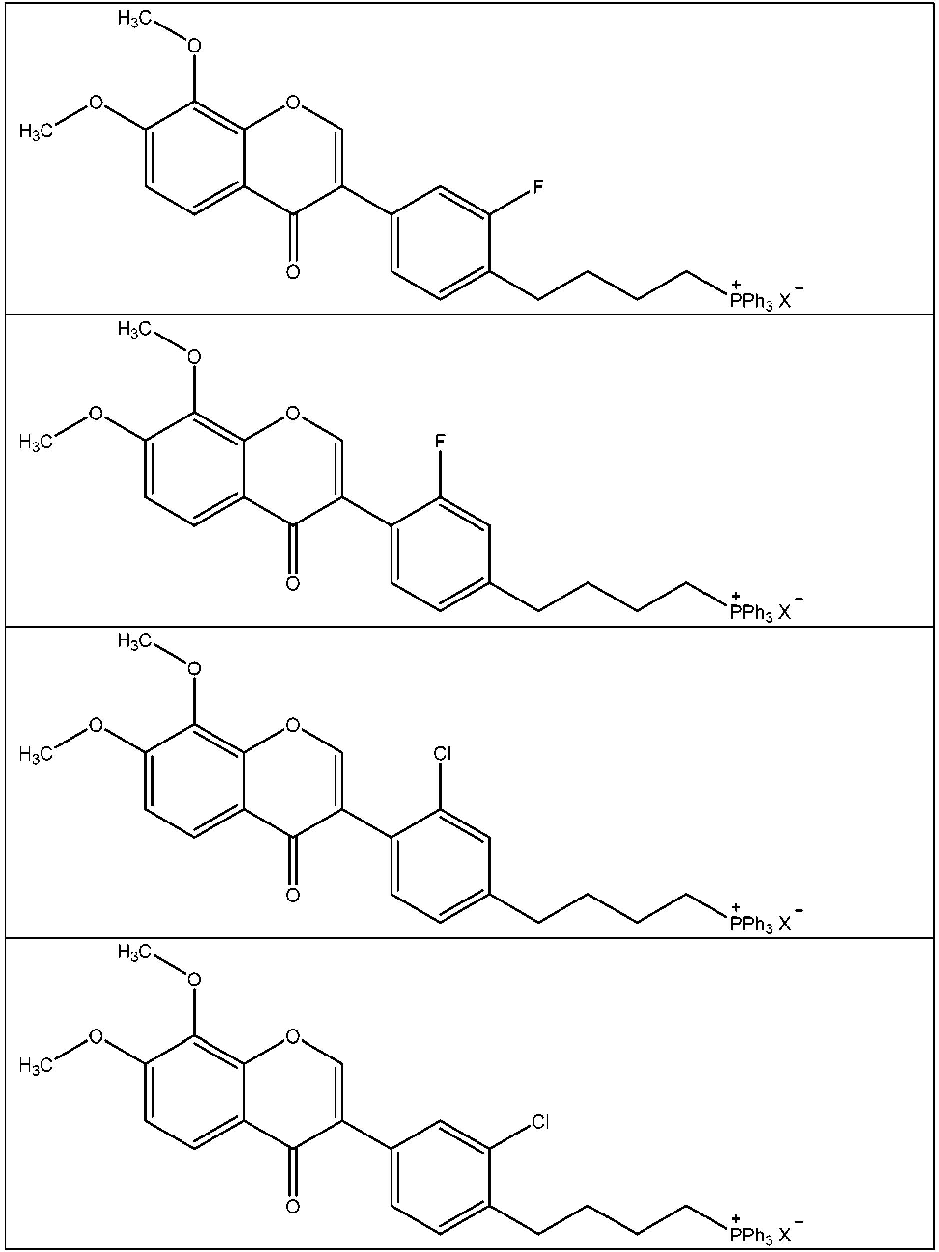

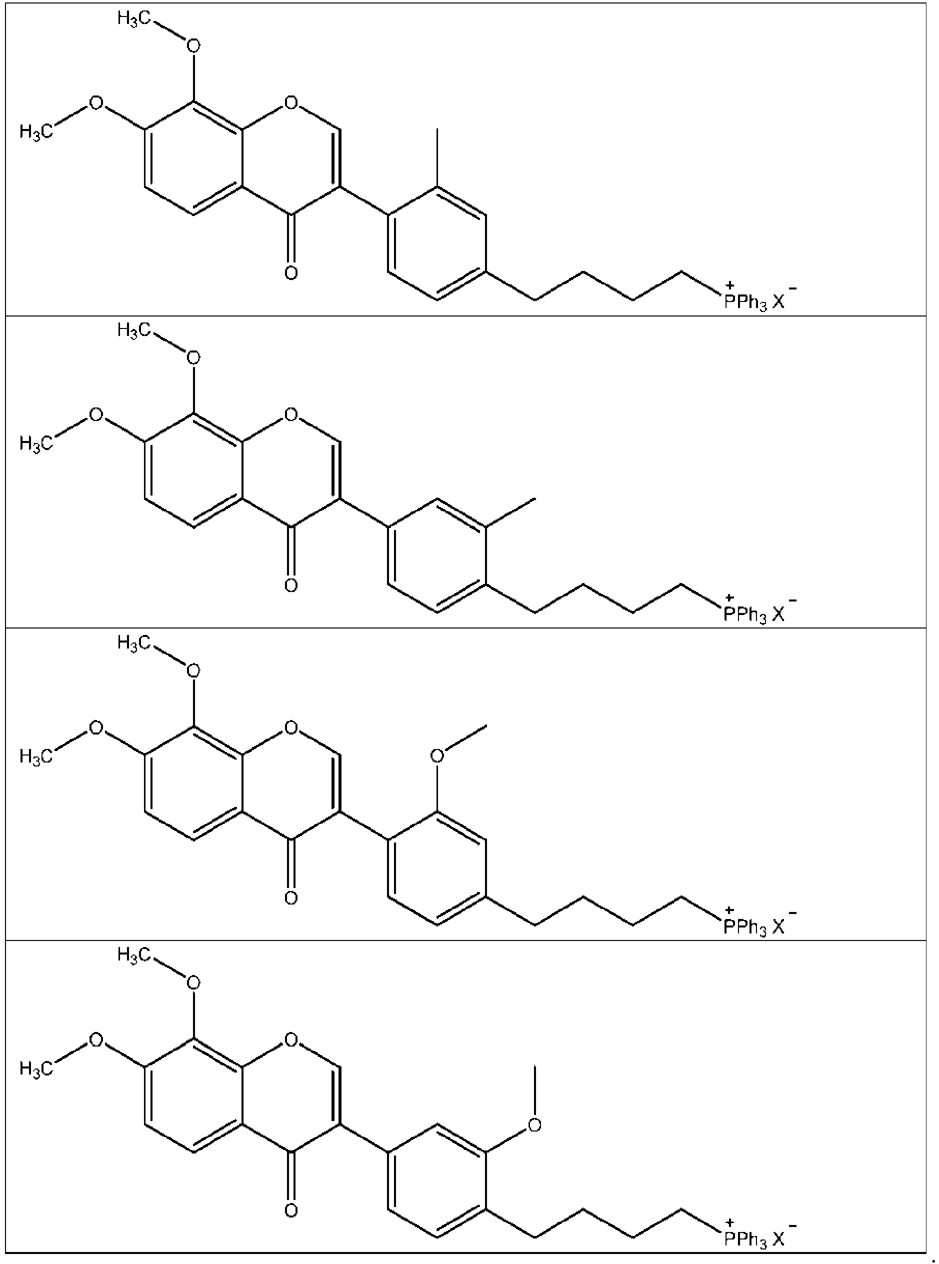

13. Composition pharmaceutique comprenant un composé, ou un sel, un multi-sel ou un solvate acceptable pharmaceutiquement tel que défini dans une ou plusieurs des revendications 1 à 12, et un excipient acceptable pharmaceutiquement.

14. Composé, ou sel, multi-sel ou solvate acceptable pharmaceutiquement tel que défini dans une ou plusieurs des revendications 1 à 12, ou composition pharmaceutique telle que définie dans la revendication 13, destiné(e) à être utilisé(e) en médecine.

15. Composé, ou sel, multi-sel ou solvate acceptable pharmaceutiquement tel que défini dans une ou plusieurs des revendications 1 à 12, ou composition pharmaceutique telle que définie dans la revendication 13, destiné(e) à être utilisé(e) dans le traitement ou la prévention d'un cancer.
